# EUROPEAN PATENT APPLICATION

(11) **EP 2 660 604 A2**
(43) Date of publication of application: **06.11.2013**
(21) Application number: 13170976.8
(22) Date of filing: 05.08.2008
(51) Int. Cl.: G01N 33/569, A61K 39/09

(54) **Immunogenic Streptococcus proteins**

(30) Priority: 06.08.2007 EP 07113844
(62) Divisional of application: 08793829.6
(71) Applicant: Boehringer Ingelheim Vetmedica GmbH, 55216 Ingelheim am Rhein (DE)
(72) Inventor: Smith, Hilda Elizabeth, 8241 AG Lelystad (NL)
(74) Representative: Jansen, Cornelis Marinus

(57) **Abstract**

The invention provides means and methods for identifying a *Streptococcus* protein which is capable of eliciting an immune response against at least two *Streptococcus* strains and/or serotypes. The invention further discloses immunogenic compositions capable of eliciting an immune response against *Streptococcus uberis* comprising at least two recombinant and/or isolated proteins derived from *Streptococcus uberis,* and/or an immunogenic part or analogue or derivative of either or both of said proteins. The invention further discloses nucleic acid molecules encoding said proteins or immunogenic parts thereof, host cells and recombinant carriers comprising such nucleic acid molecule, and vaccines and diagnostic tests based on said proteins and nucleic acids.

## Description

The invention relates to the field of medicine. More specifically, the invention relates to immunogenic *Streptococcus* proteins and immunogenic parts, derivatives and analogues thereof.

The genus *Streptococcus* is comprised of a wide variety of both pathogenic and commensal Gram-positive bacteria which are found to inhabit a wide range of hosts, including humans, horses, pigs and cows. Within the host, streptococci are often found to colonize the mucosal surfaces of the upper respiratory tract. However, in certain circumstances streptococci can also cause diseases that range from subacute to acute or even chronic.

Up to now many commercial vaccines against *Streptococcus* are based on whole cell bacterins. Generally such bacterins do produce significant protection against challenge with homologous serotypes, but do not protect against challenge with heterologous serotypes. Vaccination with whole cell *Streptococcus* often results in an immune response which is directed against the same *Streptococcus* strain, but which is not (sufficiently) directed against other *Streptococcus* strains, let alone other *Streptococcus* serotypes. As a result, many vaccines provide insufficient protection against heterologous strains and/or serotypes because vaccination against one *Streptococcus* strain is generally not efficient in counteracting infection by another *Streptococcus* strain. Moreover, vaccination against one *Streptococcus* serotype is generally not efficient in counteracting infection by another *Streptococcus* serotype. Therefore, immunogenic compositions capable of eliciting an immune response against at least two *Streptococcus* strains, preferably against two *Streptococcus* serotypes, are desired.

It is an object of the present invention to provide *Streptococcus* proteins and immunogenic parts, derivatives and/or analogues thereof, and nucleic acid molecules coding therefore, that are capable of eliciting an immune response against at least two strains of *Streptococcus.*

The invention provides a method for identifying a *Streptococcus* protein which is capable of eliciting an immune response against at least two *Streptococcus* strains, the method comprising:
a) identifying at least part of a secreted protein, a surface-associated protein and/or a protein with at least 50% sequence identity to a bacterial virulence factor;
b) selecting at least one protein identified in step a) which is conserved over at least two *Streptococcus* strains; and
c) determining whether at least one protein selected in step b) or an immunogenic part, derivative and/or analogue thereof is capable of specifically binding an antibody and/or immune cell of an animal infected by a first *Streptococcus* strain and an antibody and/or immune cell of an animal infected by a second *Streptococcus* strain. Said first *Streptococcus* strain and said second *Streptococcus* strain are preferably of the same *Streptococcus* species.

Preferably, said protein with at least 50% sequence identity to a bacterial virulence factor has at least 60%, more preferably at least 70 %, more preferably at least 75%, most preferably at least 80% sequence identity to a bacterial virulence factor.

According to the present invention, at least one *Streptococcus* protein is identified which is capable of eliciting an immune response against at least two *Streptococcus* strains. Said protein is suitable for immunizing an individual and/or non-human animal because it is capable of eliciting a broad immune response. Hence, the present invention obviates the need to provide a vaccine for each and every *Streptococcus* strain and/or serotype. The use of an immunogenic *Streptococcus* protein of the invention therefore saves time and money. More importantly, an immunogenic *Streptococcus* protein of the invention is in principle capable of eliciting an immune response against a *Streptococcus* strain that is not yet known, or against which no specific vaccine is available yet (for instance a strain which has recently evolved in nature).

Preferably, a *Streptococcus* protein of the invention is capable of eliciting an immune response against at least two *Streptococcus* serotypes. A preferred embodiment of the invention therefore provides a method for identifying a *Streptococcus* protein which is capable of eliciting an immune response against at least two *Streptococcus* serotypes, the method comprising:
a) identifying at least part of a secreted protein, a surface-associated protein and/or a protein which has at least 50% sequence identity to a bacterial virulence factor;
b) selecting at least one protein identified in step a) which is conserved over at least two *Streptococcus* serotypes; and
c) determining whether at least one protein selected in step b) or an immunogenic part, derivative and/or analogue thereof is capable of specifically binding an antibody and/or immune cell of an animal infected by a first *Streptococcus* serotype and an antibody and/or immune cell of an animal infected by a second *Streptococcus* serotype.

An immune response against at least two *Streptococcus* strains and/or *Streptococcus* serotypes is defined herein as a humoral and/or a cellular immune response directed against *Streptococcus* of at least two different strains and/or serotypes. Said immune response is for instance elicited in a non-human animal. It is also possible to elicit an immune response against at least two strains and/or serotypes of *Streptococcus* in a human individual in order to prevent and/or counteract a *Streptococcus* related disease. A humoral immune response leads to the production of antibodies, whereas a cellular immune response predominantly enhances the formation of reactive immune cells such as T killer cells. In general, both parts of the immune response are elicited by administration of an immunogenic protein or immunogenic part thereof. An immune response against at least two strains/serotypes of *Streptococcus* preferably comprises antibody production. Said immune response is preferably capable of at least in part decreasing the number of *Streptococcus* organisms in a human individual and/or non-human animal. Said immune response is furthermore preferably capable of at least in part counteracting a *Streptococcus* caused disorder.

A *Streptococcus* strain is identifiable by its morphological, biochemical and serological characteristics, as is well known in the art. A *Streptococcus* serotype is a group of *Streptococcus* whose classification is based on the presence of specific antigenic polysaccharides. Classification of *Streptococcus* serotypes is also well known in the art.

A method of the invention comprises identifying at least part of a secreted protein, a surface-associated protein and/or a protein which has at least 50% sequence identity to a bacterial virulence factor. Said protein is identified in various ways. In one embodiment of the invention a genomic approach is used. A gene encoding a secreted protein and/or a surface-associated protein is identified, for instance by searching for a motif of said secreted protein and/or surface-associated protein. Said motif preferably comprises a lipid attachment site, a signal peptidase cleavage site and/or a sortase attachment site. Of course, it is possible to search for other motifs known in the art. One embodiment of the invention therefore provides a method of the invention wherein said secreted protein and/or surface-associated protein is identified by identifying in at least part of the genomic sequence of a *Streptococcus* a gene comprising a motif of a secreted and/or surface-associated protein.

Additionally, or alternatively, a gene encoding a secreted protein and/or a surface-associated protein is identified by one or more other methods known in the art. For instance, once a gene of a *Streptococcus* species encoding a secreted protein and/or a surface-associated protein is known, it is possible to screen another *Streptococcus* genomic sequence for the presence of a gene with high % sequence identity.

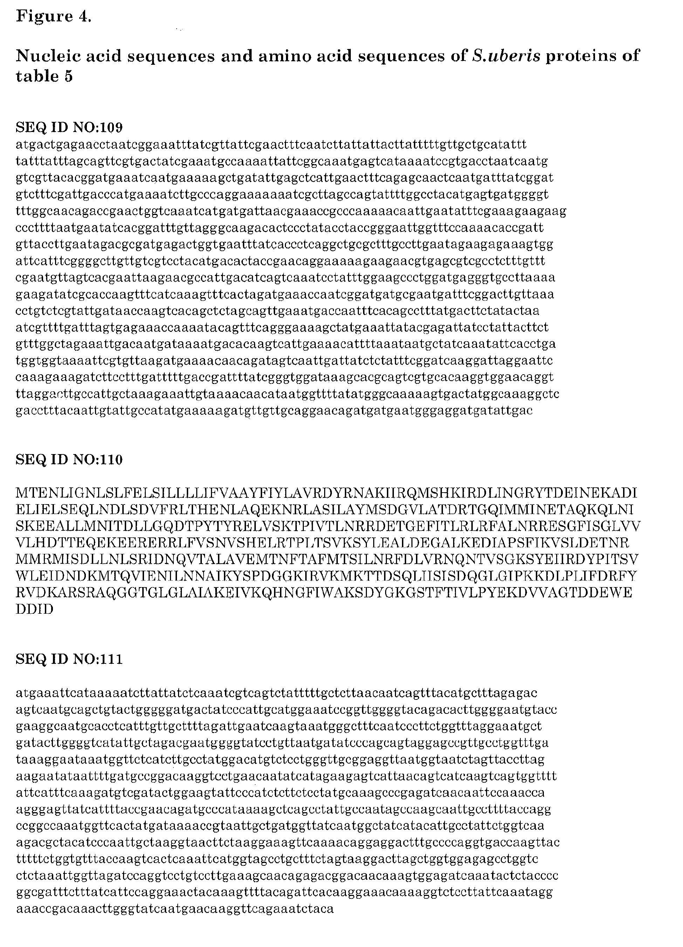

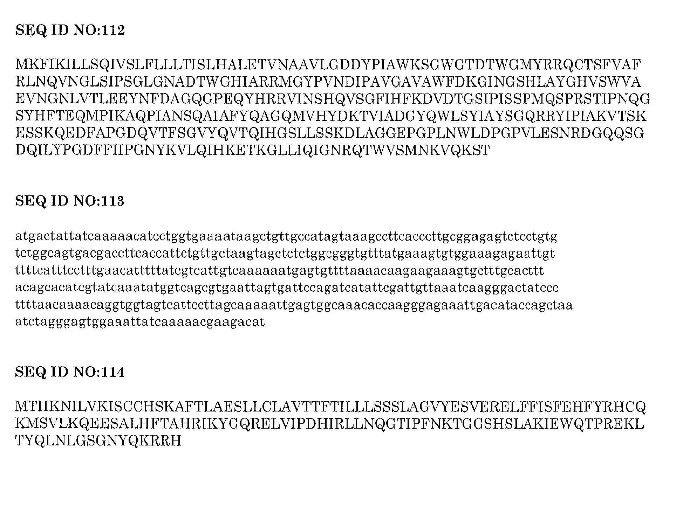

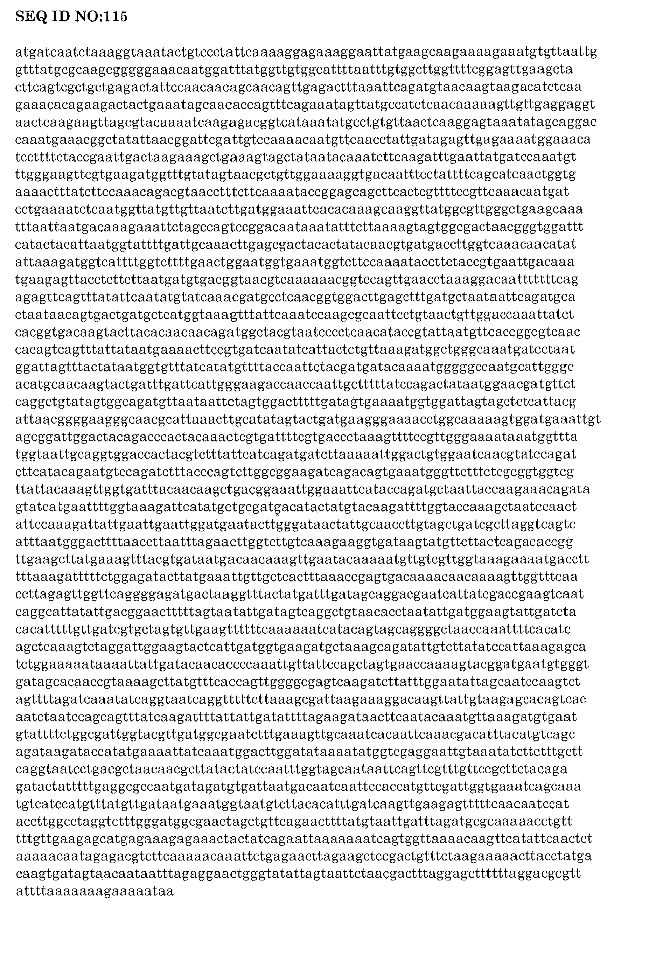

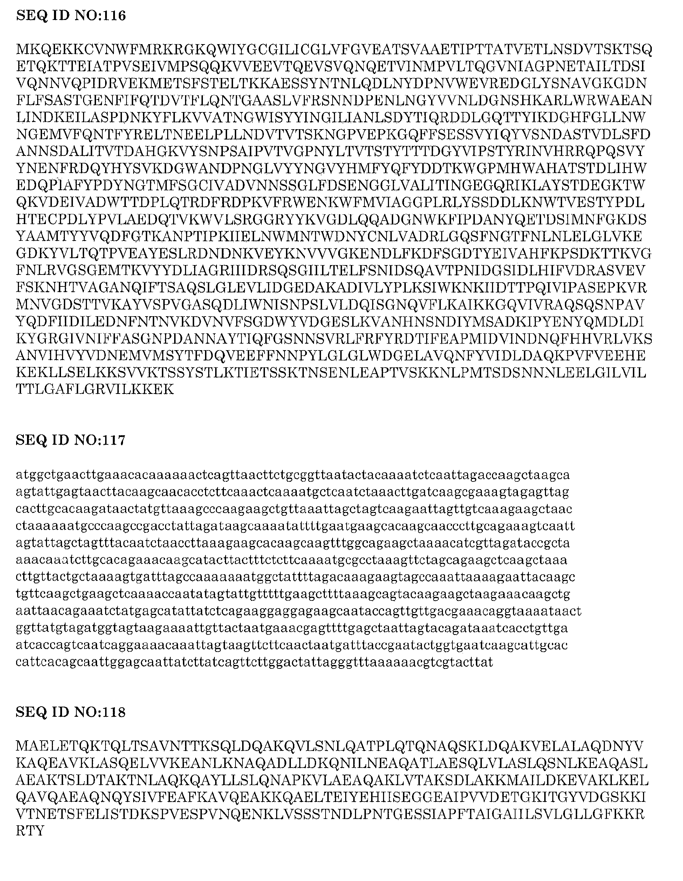

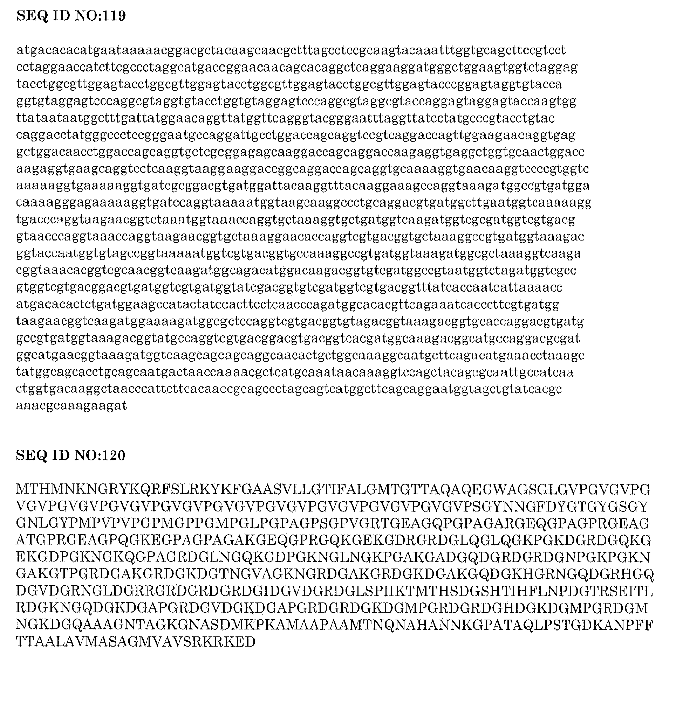

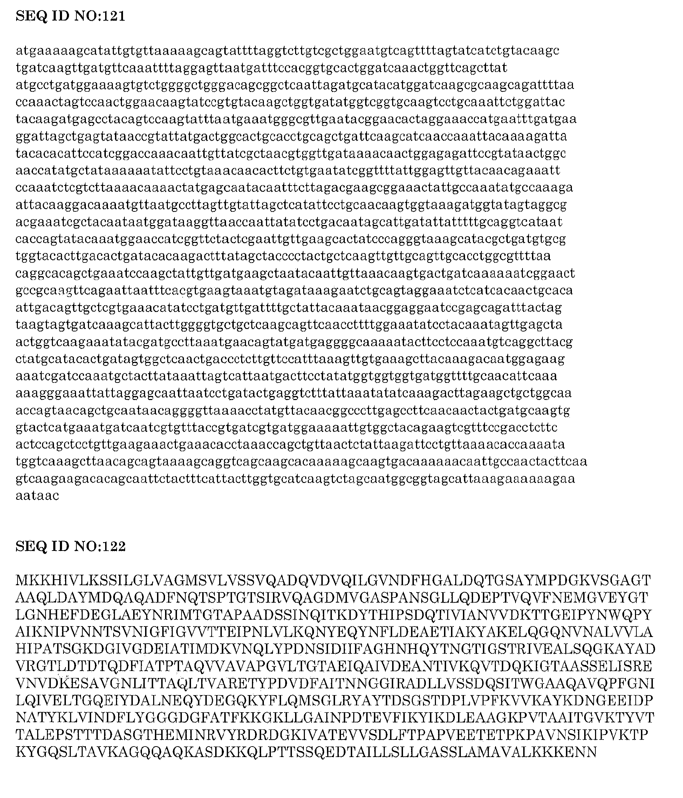

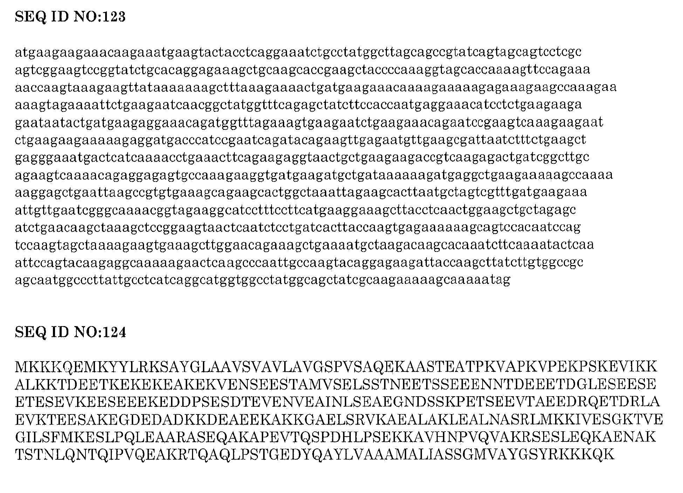

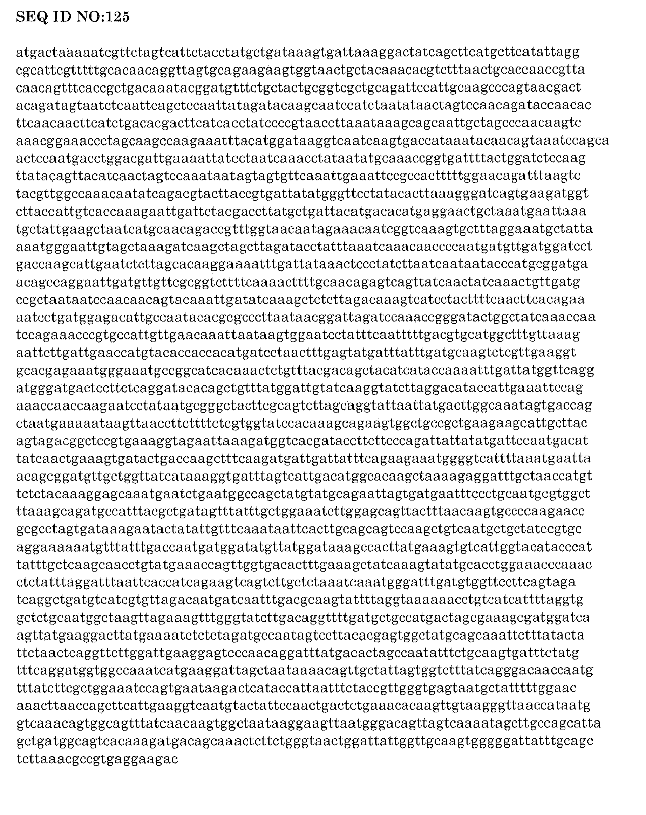

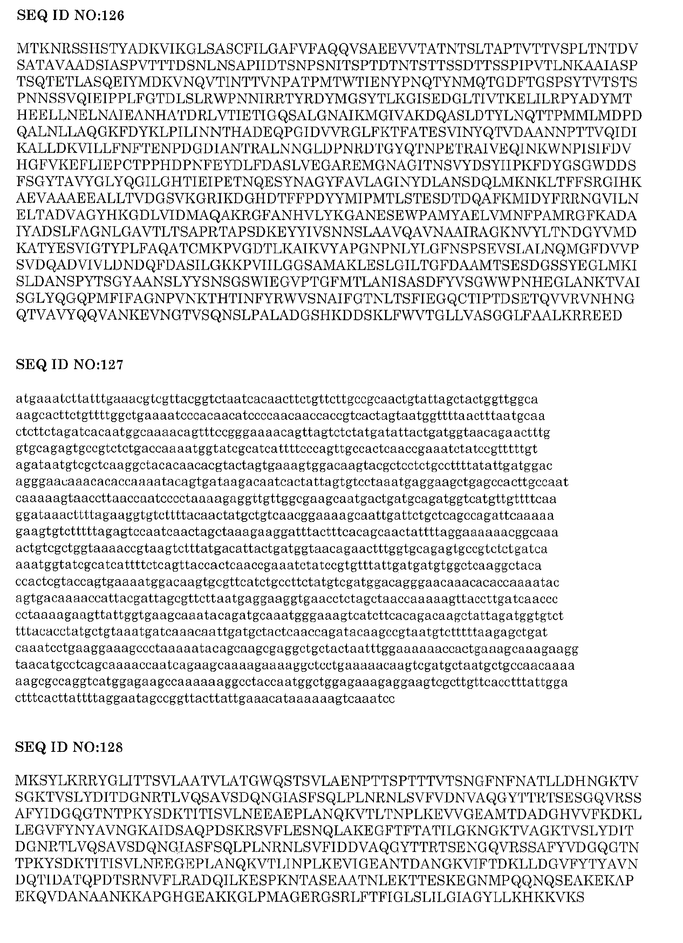

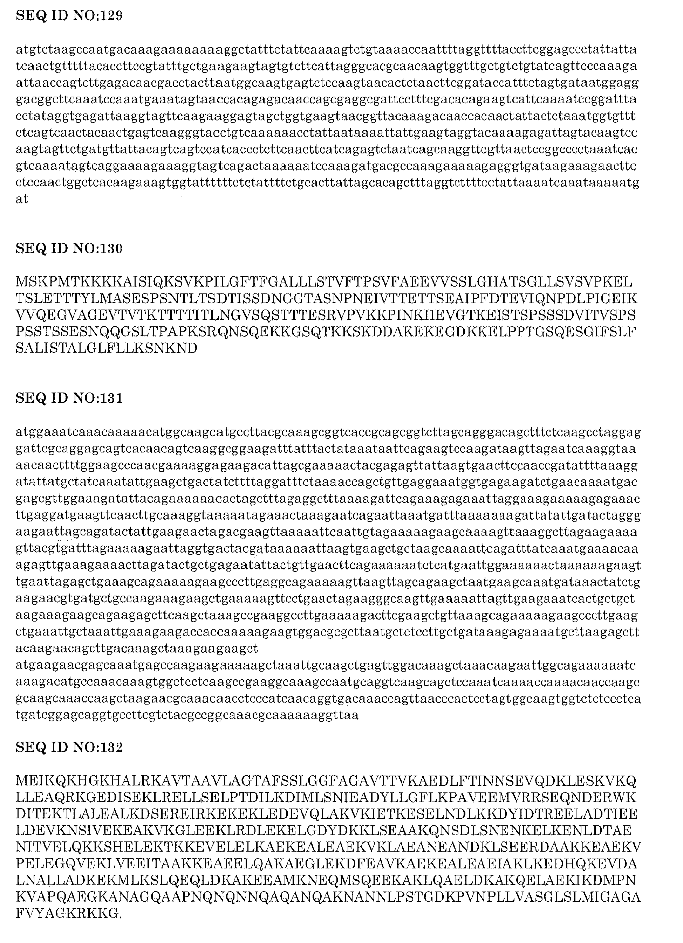

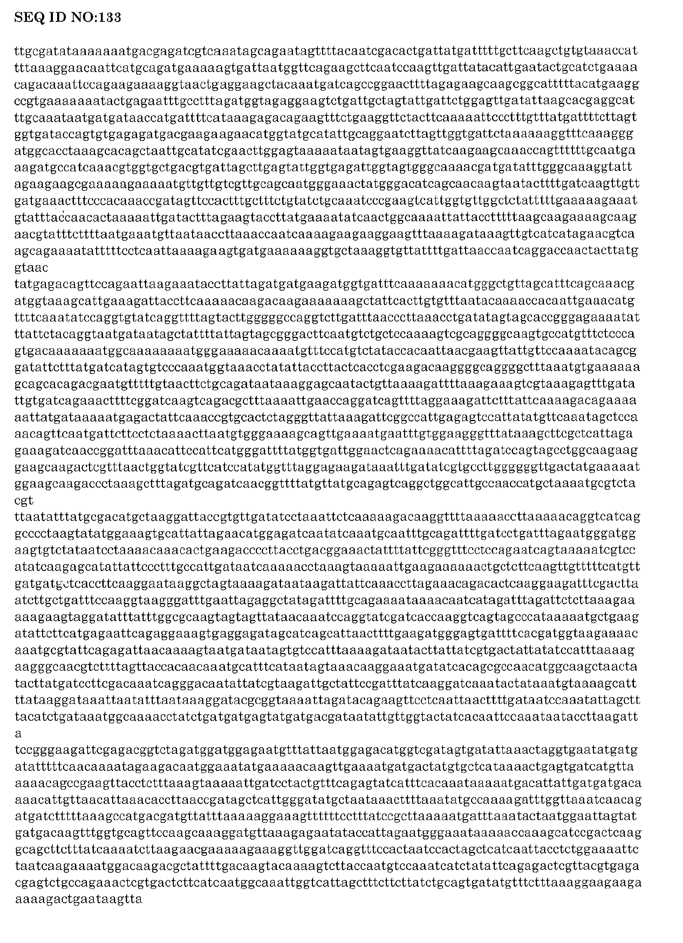

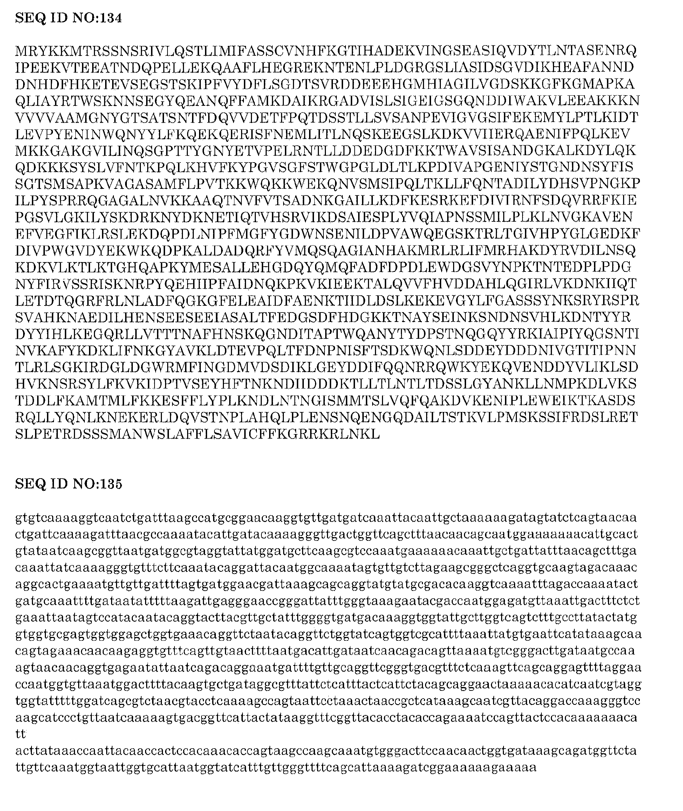

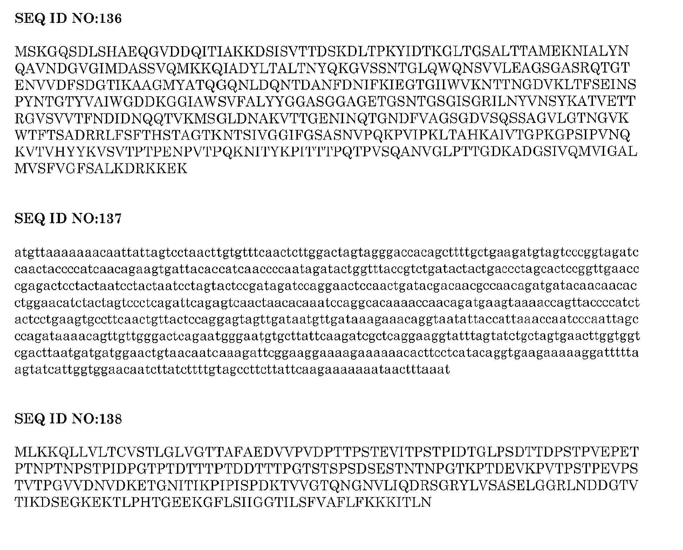

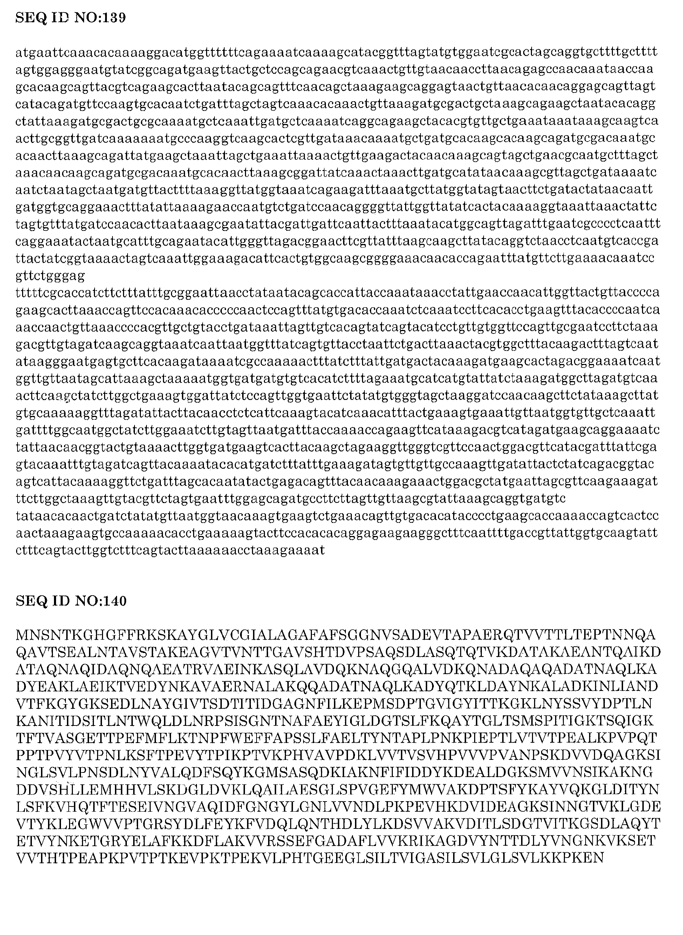

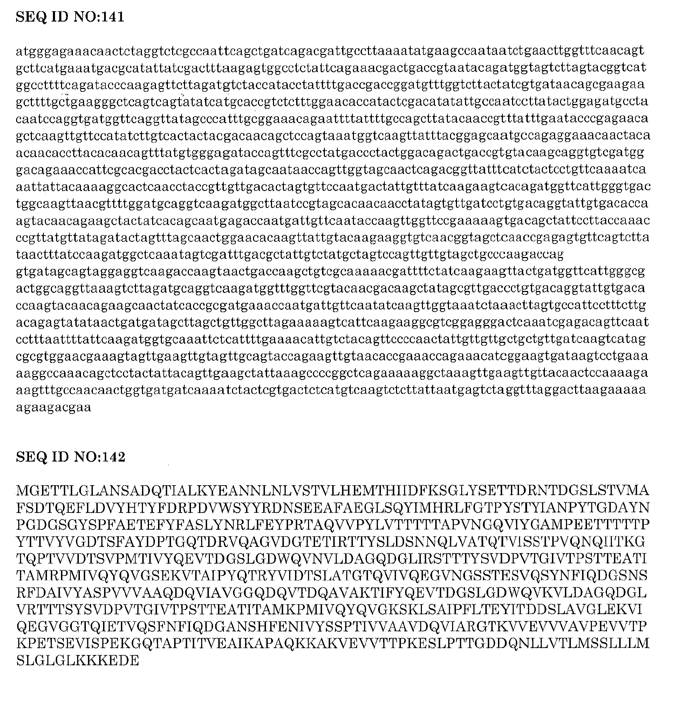

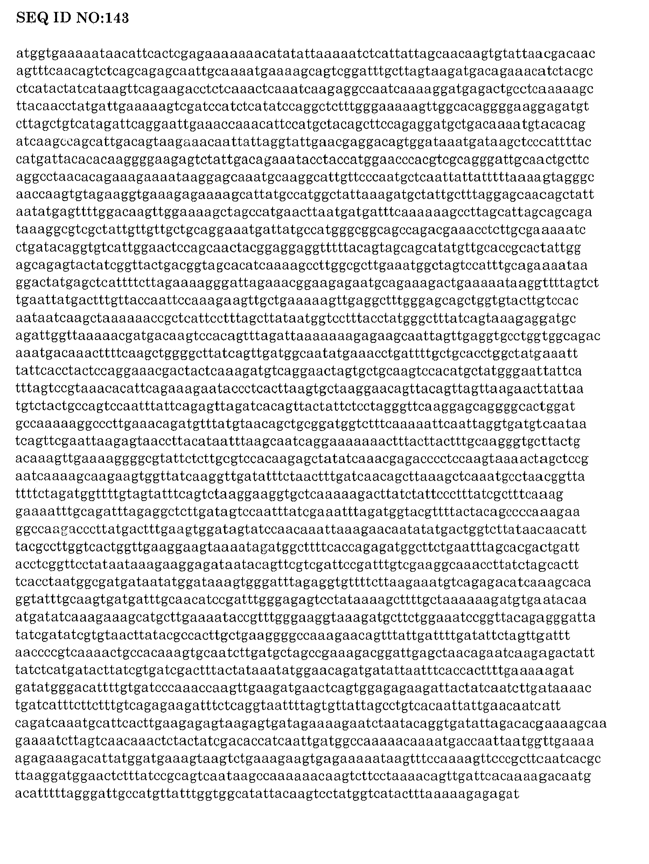

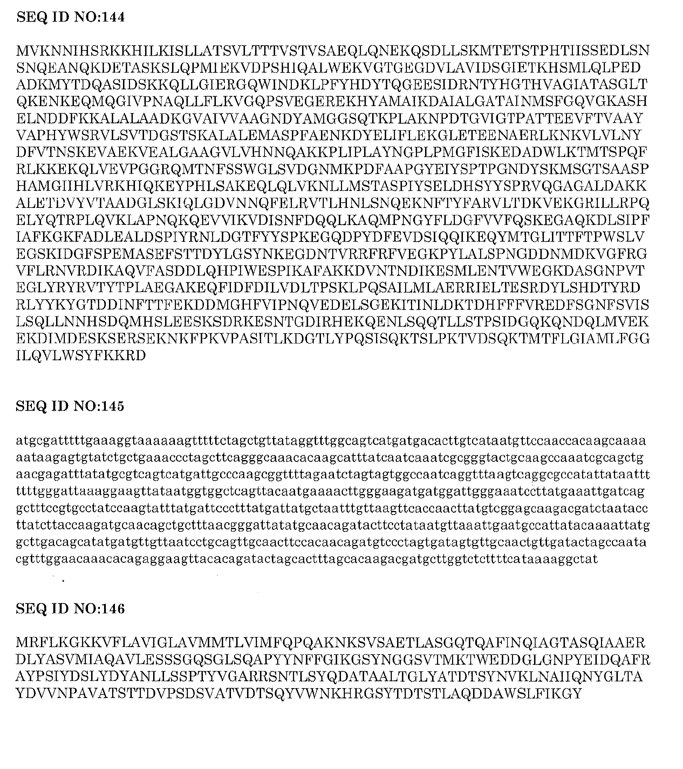

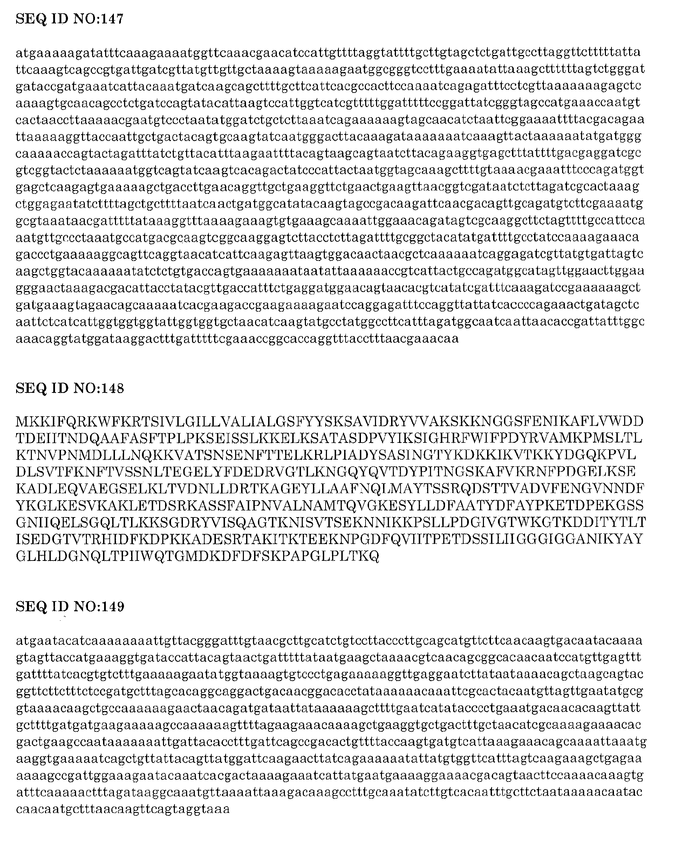

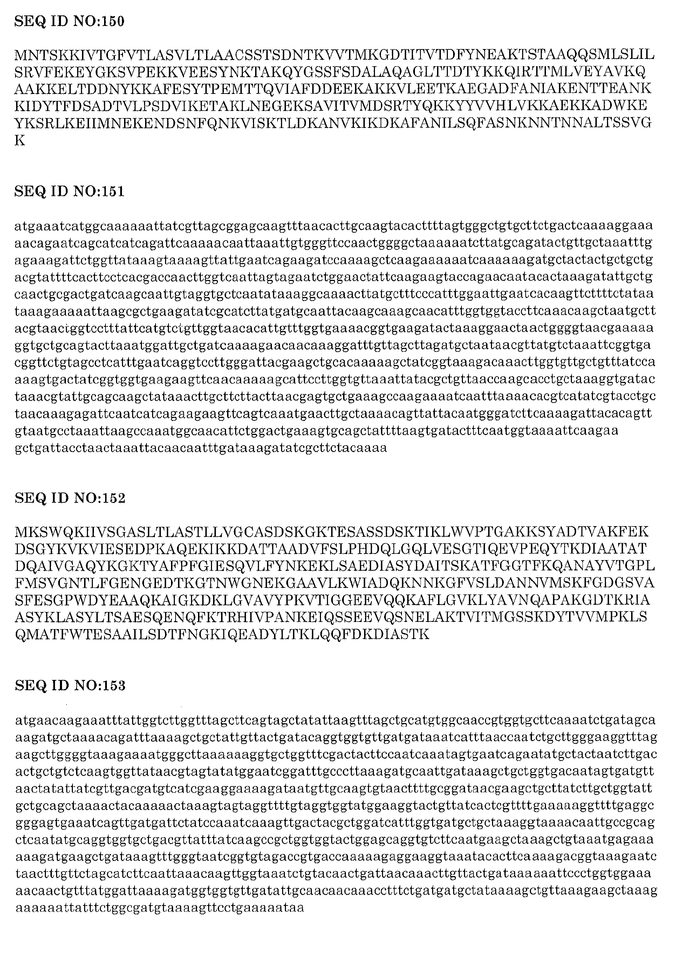

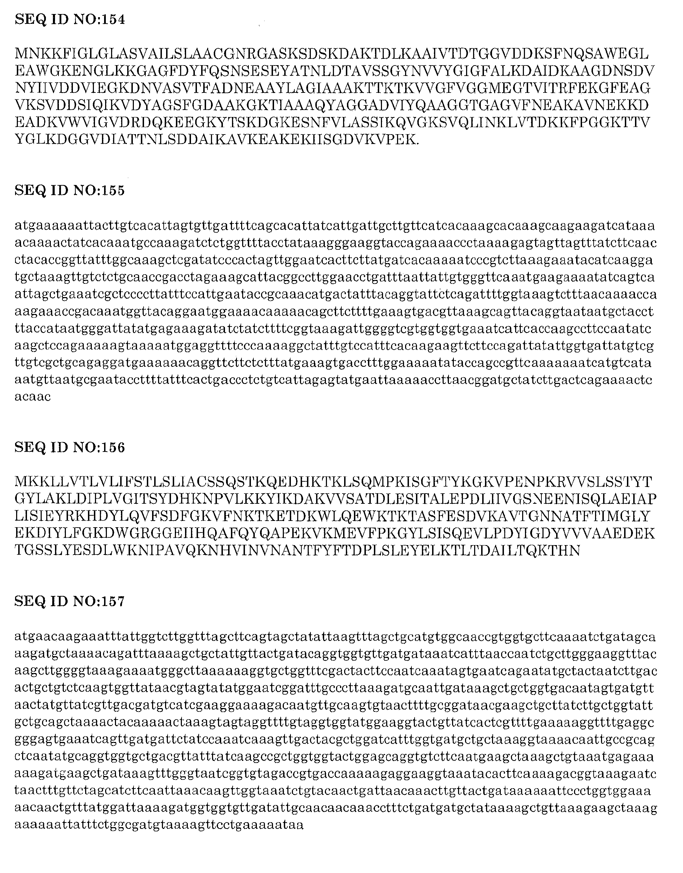

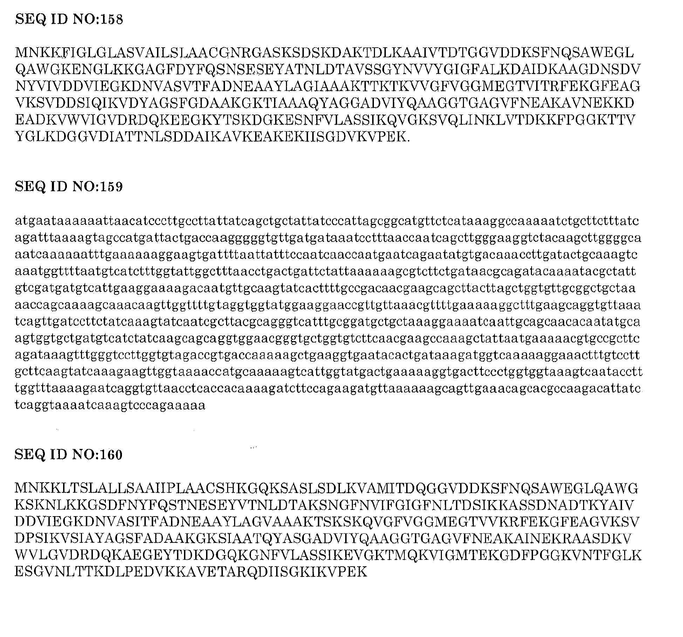

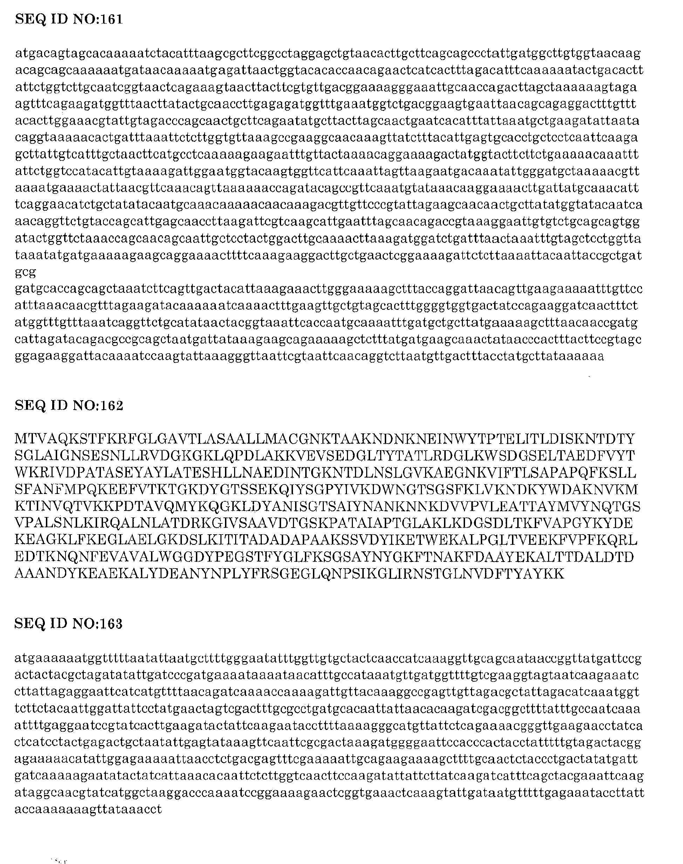

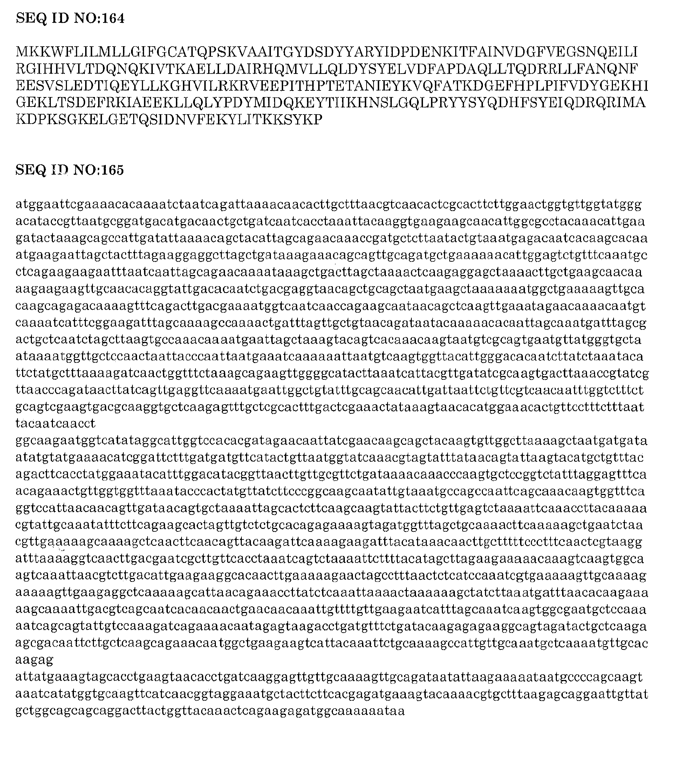

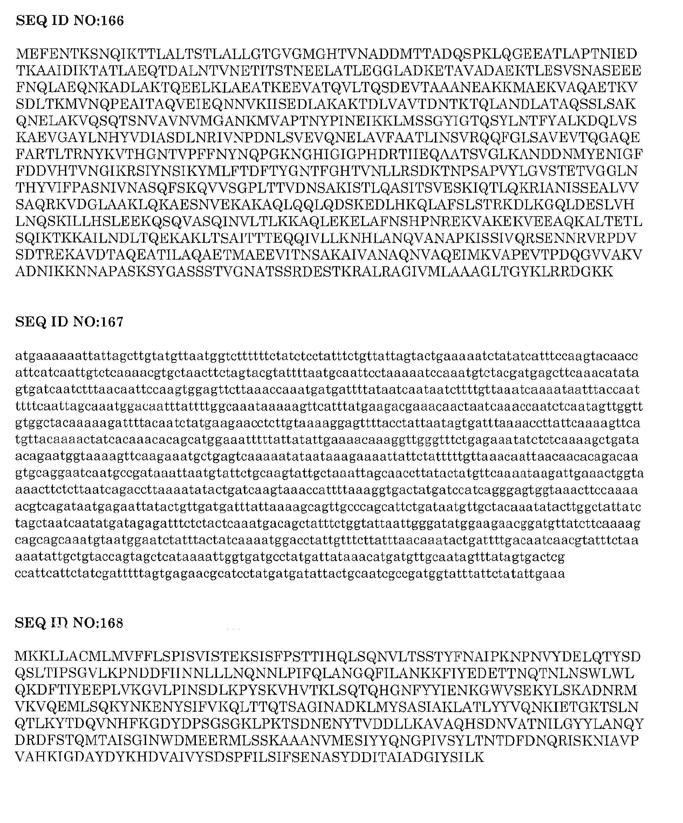

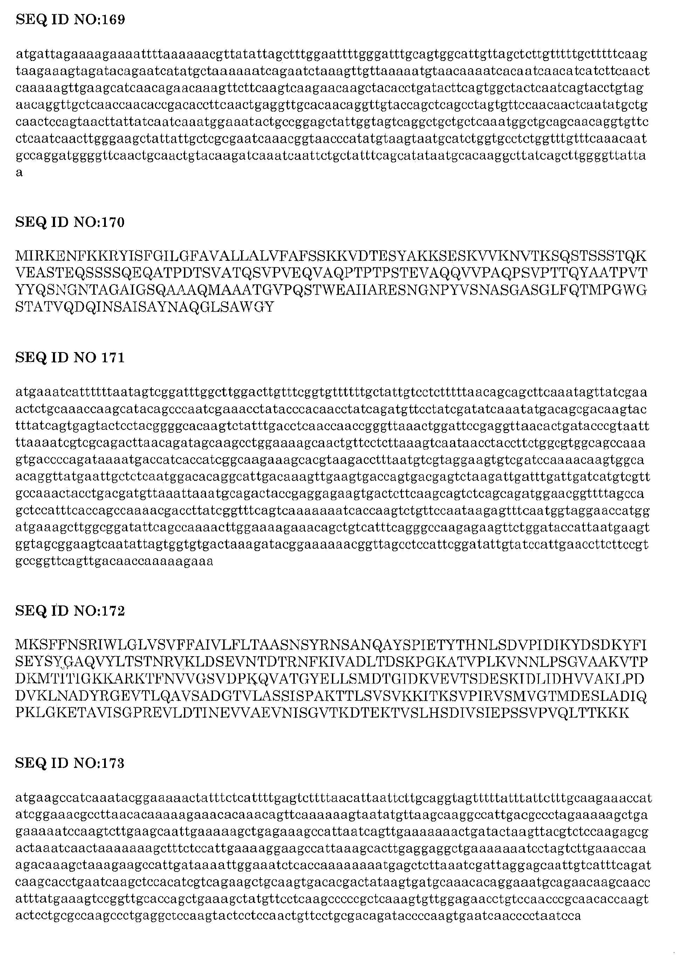

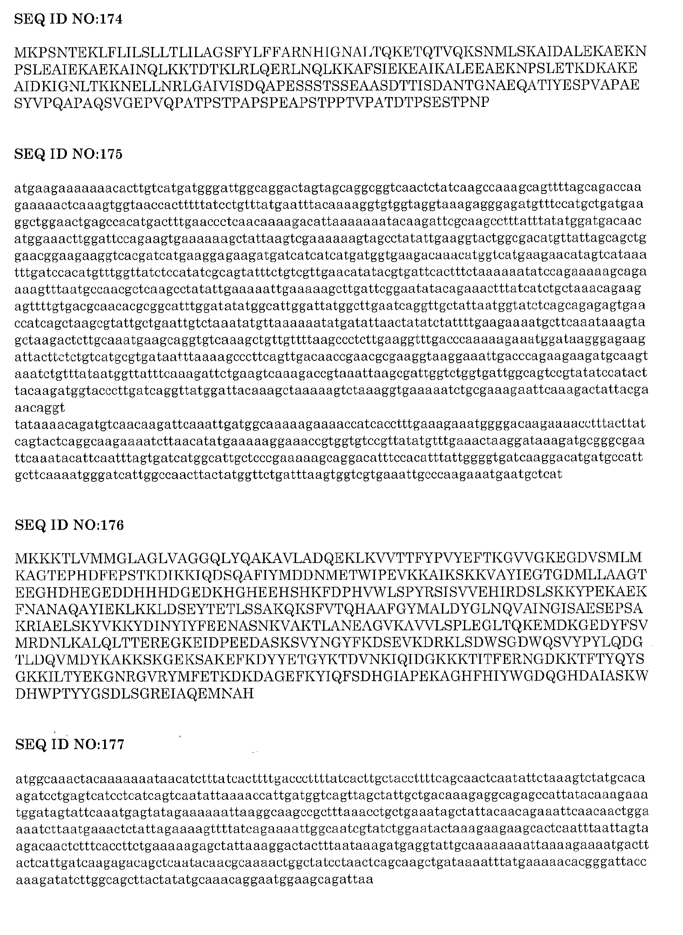

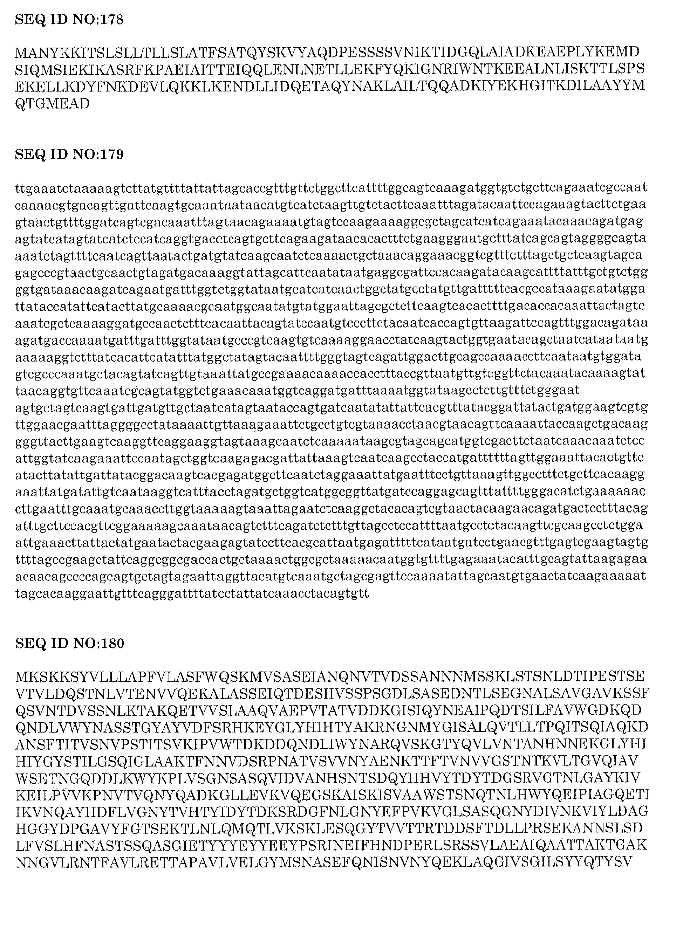

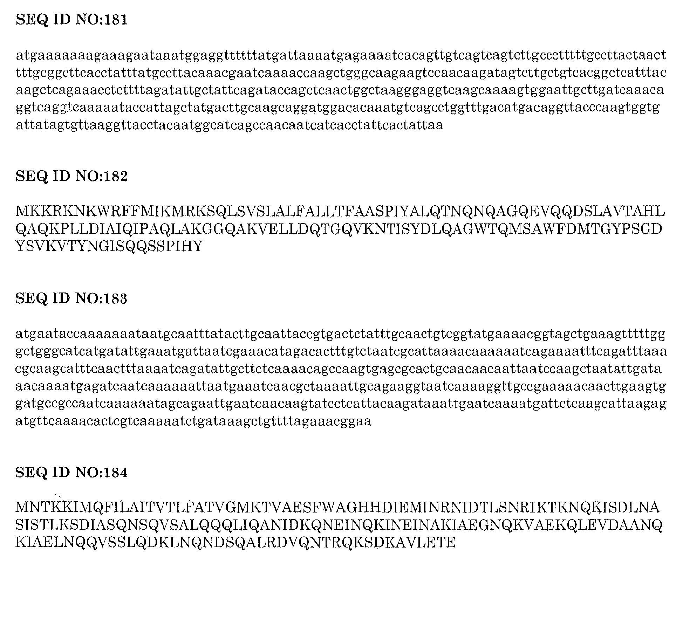

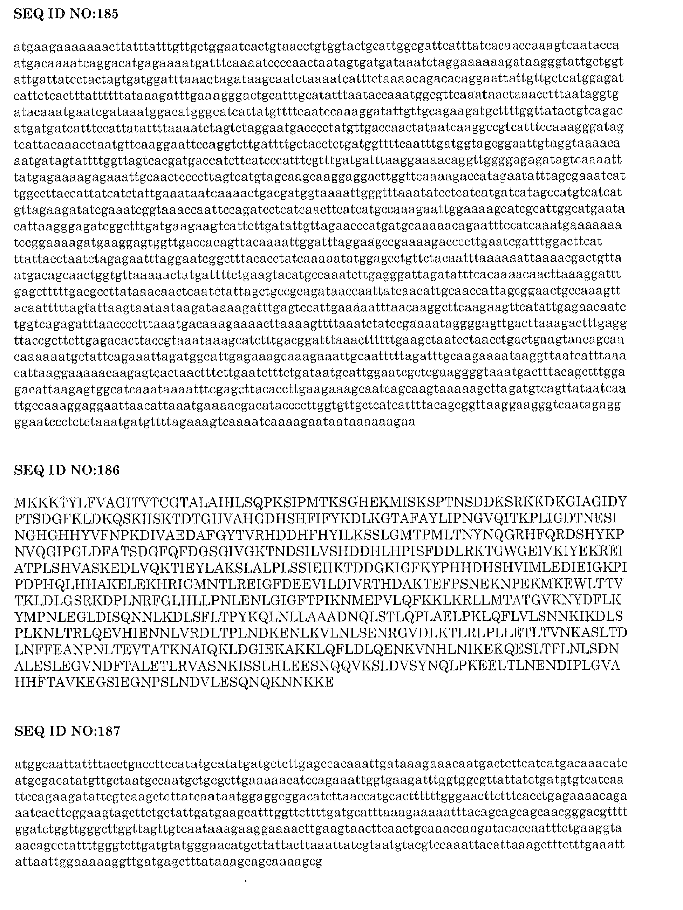

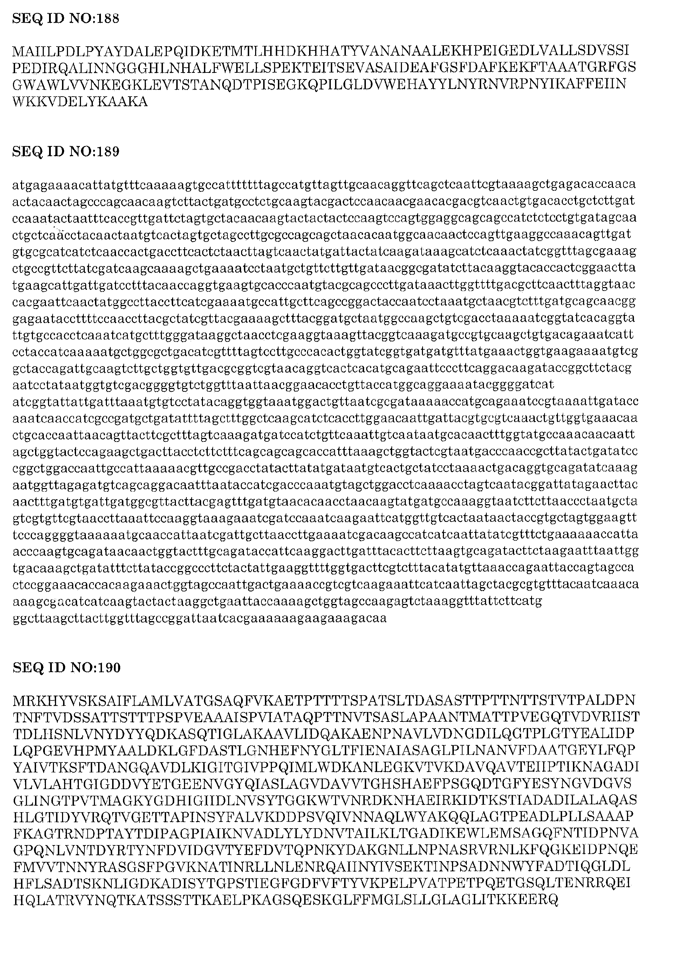

In one embodiment, such a screening method comprises a method in which said another *Streptococcus* genomic sequence is screened for its capability of hybridizing to a nucleotide sequence encoding a secreted and/or surface-associated protein of *Streptococcus.* The invention therefore provides a method according to the invention, wherein said protein which has at least 50% sequence identity to a bacterial virulence factor is identified by identifying in at least part of the genomic sequence of *Streptococcus* a gene which is capable of hybridizing to any of the nucleic acid sequences listed in Figure 4 at 65°C in a buffer having 0.5 M sodium phosphate, 1 mM EDTA, and 7% sodium dodecyl sulphate at a pH of 7.2, wherein the nucleic acid molecule remains hybridized after washing twice with a buffer containing 40 mM sodium phosphate (pH 7.2), 1mM EDTA and 5% sodium dodecyl sulphate for 30 minutes at 65°C and; washing twice with a buffer containing 40 mM sodium phosphate (pH 7.2), 1 mM EDTA and 1% sodium dodecyl sulphate for 30 minutes at 65°C.

Preferably, said protein with at least 50% sequence identity to a bacterial virulence factor has at least 60%, more preferably at least 70 %, more preferably at least 75%, most preferably at least 80% sequence identity to a bacterial virulence factor.

The art furthermore provides various methods for determining whether a *Streptococcus* protein has at least 50% sequence identity to a bacterial virulence factor. For instance, the amino acid sequence of a *Streptococcus* protein is compared with the amino acid sequence of a bacterial virulence factor. It is also possible to apply a genomic approach. A gene encoding a *Streptococcus* protein which has at least 50% sequence identity to a bacterial virulence factor is for instance identified by screening *a Streptococcus* genomic sequence for a nucleotide sequence which has at least 50% sequence identity to a bacterial gene encoding a virulence factor. One embodiment of the invention therefore provides a method of the invention wherein a protein which has at least 50% sequence identity to a bacterial virulence factor is identified by identifying in at least part of the genomic sequence of a *Streptococcus* a gene which has at least 50% sequence identity to a bacterial virulence factor gene. However, many alternative methods for determining whether a *Streptococcus* protein has at least 50% sequence identity to a bacterial virulence factor are known in the art.

Once at least one *Streptococcus* gene encoding a secreted protein, a surface-associated protein and/or a protein which has at least 50% sequence identity to a bacterial virulence factor is identified, it is preferably determined whether at least one of said genes is conserved over at least two *Streptococcus* strains. A gene of a first *Streptococcus* strain is conserved over at least two *Streptococcus* strains if a genome of a second *Streptococcus* strain comprises a nucleic acid sequence which has at least about 60% sequence identity to said gene of said first *Streptococcus* strain. Preferably, said nucleic acid sequence has at least 70%, more preferably at least 75%, more preferably at least 80% more preferably at least 90%, most preferably at least 95% sequence identity to said gene. The term "sequence identity" refers to the percentage identity between two nucleic acid sequences or amino acid sequences. Two nucleic acid sequences have at least 60% sequence identity to each other when said sequences exhibit at least 60% sequence identity after aligning the two sequences and introducing gaps, if necessary, to achieve the maximum percent sequence identity. Methods and computer programs for the alignment are well known in the art. One computer program which may be used or adapted for purposes of determining whether a candidate sequence falls within this definition is "Align 2", authored by Genentech, Inc., which was filed with user documentation in the United States Copyright Office, Washington, D.C. 20559, on Dec. 10, 1991..

According to one embodiment of the invention, if a gene of the invention is conserved over at least two *Streptococcus* strains, the protein encoded by said gene is a good candidate for assessing whether said protein, or an immunogenic part, derivative and/or analogue thereof, is capable of eliciting an immune response against more than one *Streptococcus* strain. Said first *Streptococcus* strain and said second *Streptococcus* strain are preferably of the same *Streptococcus* species.

Preferably, it is determined whether said gene is conserved over at least two *Streptococcus* serotypes, in order to identify a good candidate protein (encoded by said gene) which is tested for its capability of eliciting an immune response against more than one *Streptococcus* serotype. A method of the invention which further comprises selecting a gene which is conserved over at least two *Streptococcus* strains and/or serotypes is therefore preferred.

Once a gene conserved over at least two *Streptococcus* strains/serotypes is identified, a protein encoded by said gene is preferably obtained. Additionally, or alternatively, an immunogenic part, derivative and/or analogue of said protein is obtained. The art provides various methods for obtaining a protein encoded by a gene, or an immunogenic part, derivative and/or analogue thereof. Said gene is for instance expressed by a suitable expression system. Non-limiting examples of expression systems comprise eukaryotic host cells such as yeast and prokaryotic host cells such as *Escherichia coli.* Preferably, a gene of the invention encoding a secreted protein, a surface-associated protein and/or a protein which has at least 50% sequence identity to a bacterial virulence factor, which gene is conserved over at least two *Streptococcus* strains, is expressed in a prokaryotic expression system. A prokaryotic expression system is preferred because a (prokaryotic) *Streptococcus* protein is in principle better expressed in a prokaryotic expression system. Moreover, a prokaryotic expression system is generally more easily set up and used.

A method of the invention comprises determining whether at least one protein of the invention or an immunogenic part, derivative and/or analogue thereof is capable of specifically binding an antibody and/or immune cell of an animal infected by a first *Streptococcus* strain and an antibody and/or immune cell of an animal infected by a second *Streptococcus* strain. Preferably, it is determined whether at least one protein of the invention or an immunogenic part, derivative and/or analogue thereof is capable of specifically binding an antibody and/or immune cell of an animal infected by a first *Streptococcus* serotype and an antibody and/or immune cell of an animal infected by a second *Streptococcus* serotype. Many methods are known in the art for performing said test. Preferably, serum of at least two animals infected by at least two different *Streptococcus* strains is used. Alternatively, serum of only one animal is used, said animal being infected with at least two different *Streptococcus* strains. According to one embodiment, one non-human animal is infected by at least a first *Streptococcus* strain and/or serotype, and a second non-human animal is infected by at least a second *Streptococcus* strain and/or serotype. Said *Streptococcus* strains and/or serotypes are for instance administered intravenously to said animal. Subsequently, according to one embodiment, serum from said animals comprising *Streptococcus-*specific antibodies and/or immune cells is collected. Said serum is optionally processed before use. For instance, antibodies and/or immune cells are at least in part concentrated and/or isolated. A protein of the invention and/or an immunogenic part, derivative and/or analogue thereof is preferably isolated and/or recombinantly produced and subsequently incubated with said serum - or with (partly) isolated antibodies and/or immune cells - derived from said animals. It is possible to administer serum, antibodies and/or immune cells derived from a first animal together with serum, antibodies and/or immune cells derived from a second animal. Alternatively, serum, antibodies and/or immune cells derived from a first animal is administrated firstly, after which serum, antibodies and/or immune cells from a second animal is added. In yet another embodiment serum, antibodies and/or immune cells of a first animal is administrated to one separate batch comprising at least one protein and/or immunogenic part, derivative and/or analogue according to the invention and serum, antibodies and/or immune cells of a second animal is administered to another batch comprising at least one protein and/or immunogenic part, derivative and/or analogue according to the invention. After incubation said serum, antibodies and/or immune cells are washed away and bound antibodies and/or immune cells are visualised, using any method known in the art. Bound antibodies are for instance incubated with a second antibody capable of specifically binding said bound antibodies, which second antibody is conjugated with horse-radish peroxidase. After unbound second antibodies are washed away, hydrogen peroxide is administered. Breakdown of hydrogen peroxide by horse-radish peroxidase is coupled to the oxidation of a chromogenic compound, so that the reaction is made visible.

If a protein of the invention and/or an immunogenic part, derivative and/or analogue thereof appears to be specifically bound by an antibody and/or immune cell elicited by a first *Streptococcus* strain, and by an antibody and/or immune cell elicited by a second *Streptococcus* strain, it indicates that said protein, immunogenic part, derivative and/or analogue is capable of eliciting an immune response against at least two *Streptococcus* strains.

In one preferred embodiment an antibody and/or immune cell derived from a convalescent serum of an animal which was infected with a *Streptococcus* is used. A convalescent serum is derived from an animal which has efficiently counteracted its infection. Hence, a convalescent serum of an animal which was infected with *Streptococcus* comprises antibodies and/or immune cells that are capable of protecting said animal against a challenge with the same *Streptococcus* strain. Therefore, incubation with a convalescent medium is preferred in order to determine whether a protein and/or immunogenic part, derivative and/or analogue according to the invention is capable of eliciting a protective immune response.

One embodiment of the invention thus provides a method for identifying a *Streptococcus* protein which is capable of eliciting an immune response against at least two *Streptococcus* strains, the method comprising:
- obtaining isolated and/or recombinant *Streptococcus* proteins;
- incubating said proteins with an antibody and/or immune cell of an animal infected by a first *Streptococcus* strain and/or serotype, and with an antibody and/or immune cell of an animal infected by a second *Streptococcus* strain and/or serotype, and
- determining whether a protein is capable of binding an antibody and/or immune cell of an animal infected by a first *Streptococcus* strain and/or serotype and an antibody and/or immune cell of an animal infected by a second *Streptococcus* strain and/or serotype.

Proteins of *Streptococcus* are obtained in various ways. Preferably, secreted proteins, surface-associated proteins and/or proteins which have at least 50% sequence identity to a bacterial virulence factor are isolated from a *Streptococcus* culture. In one embodiment surface-associated proteins are stripped from *Streptococcus* using for instance lysozyme.

In one embodiment *Streptococcus* proteins are recombinantly produced using at least one nucleic acid sequence encoding at least one of said proteins. As explained above, a gene encoding a secreted protein, a surface-associated proteins and/or a protein which has at least 50% sequence identity to a bacterial virulence factor is preferably used. More preferably, said gene is conserved over at least two *Streptococcus* strains and/or serotypes.

Alternatively, or additionally, a *Streptococcus* protein or an immunogenic part, derivative and/or analogue thereof is generated using another method known in the art. For instance, an immunogenic *Streptococcus* protein or peptide is generated using a common synthesis technique such as solid phase synthesis. As another example, a *Streptococcus* protein is isolated from a *Streptococcus,* or recombinantly made, after which it is modified in order to produce an immunogenic part, derivative and/or analogue.

In one preferred embodiment *Streptococcus* proteins are separated on a polyacrylamide gel and subsequently incubated with an antibody and/or immune cell of an animal infected by a first *Streptococcus* strain and/or serotype and an antibody and/or immune cell of an animal infected by a second *Streptococcus* strain and/or serotype. Preferably a two-dimensional polyacrylamide gel is used.

In a preferred embodiment a *Streptococcus* protein is identified which is capable of eliciting opsonophagocytosis inducing antibodies. Opsonophagocytosis is a natural process wherein a microorganism is opsonized by opsonins, after which said microorganism is phagocytised by a phagocytic cell and killed. Many microorganisms need to be opsonized by opsonins to enhance their phagocytosis. Opsonization is a process of making a microorganism more susceptible for uptake by a phagocyte. In said process, opsonizing antibodies and/or proteins bind to said microorganism, thereby facilitating the uptake of said microorganism by said phagocyte.

Hence, a *Streptococcus* protein of the invention or an immunogenic part, derivative and/or analogue thereof capable of eliciting opsonophagocytosis inducing antibodies is preferred because administration of such protein and/or immunogenic part, derivative and/or analogue to an animal results in the presence of opsonophagocytosis inducing antibodies in said animal capable of phagocytosing *Streptococcus.*

A *Streptococcus* protein of the invention or an immunogenic part, derivative and/or analogue thereof is capable of eliciting an immune response against at least two strains and/or serotypes of *Streptococcus.* In order to elicit an even broader immune response, it is preferred to identify at least two different *Streptococcus* proteins, and/or an immunogenic part, derivative and/or analogue of at least one of said proteins. More preferably, at least three different *Streptococcus* proteins, and/or an immunogenic part, derivative and/or analogue of at least one of said proteins is identified, et cetera. The higher the number of *Streptococcus* proteins and/or immunogenic parts, derivatives and/or analogues of the present invention that are identified, the broader immune response is elicited.

In another preferred embodiment at least one *Streptococcus* protein and/or an immunogenic part, derivative and/or analogue according to the invention is identified which is capable of eliciting an immune response against at least three strains of *Streptococcus.* Said protein and/or immunogenic part, derivative and/or analogue is particularly suitable for eliciting a broad immune response in a human individual and/or a non-human animal. More preferably at least one *Streptococcus* protein and/or an immunogenic part, derivative and/or analogue according to the invention is identified which is capable of eliciting an immune response against at least three *Streptococcus* serotypes.

An immunogenic part of a protein is defined as a part of a protein which is capable of eliciting an immune response in a human individual and/or a non-human animal. Preferably said immunogenic part is capable of eliciting the same immune response in kind, albeit not necessarily in amount, as said protein. An immunogenic part of a protein preferably comprises one or more epitopes of said protein. An epitope of a protein is defined as a part of said protein, at least about 5 amino acids in length, capable of eliciting a specific antibody and/or immune cell capable of specifically binding said epitope. Two different kinds of epitopes exist: linear epitopes and conformational epitopes. A linear epitope comprises a stretch of consecutive amino acids. A conformational epitope is formed by several stretches of consecutive amino acids that are folded in position and together form an epitope in a properly folded protein. An immunogenic part of the invention is capable of comprising either one, or both, of said kinds of epitopes.

An immunogenic part of a protein comprises at least 5 amino acid residues. Preferably said immunogenic part comprises at least 10, more preferably at least 15, more preferably at least 25 and most preferably at least 30 consecutive amino acids. Said immunogenic part preferably comprises at most about 500 amino acid residues, more preferably at most 250 amino acid residues, depending on the kind of protein from which said immunogenic part is derived.

A derivative of a protein is defined as a molecule which has the same immunogenic properties in kind, not necessarily in amount. A person skilled in the art is capable of altering a protein such that the immunogenic properties of said molecule are essentially the same in kind, not necessarily in amount, as compared to said protein. A derivative of a protein is for instance provided by mutating at least one amino acid residue of said protein and/or by replacing one amino acid residue by another amino acid residue. Preferably, conservative amino acid substitutions are made, like for example replacement of an amino acid comprising an acidic side chain by another amino acid comprising an acidic side chain, replacement of a bulky amino acid by another bulky amino acid, replacement of an amino acid comprising a basic side chain by another amino acid comprising a basic side chain, et cetera.

A person skilled in the art is well able to generate analogous compounds of a protein. This is for instance done through screening of a peptide library or by peptide changing programs. An analogue according to the invention has essentially the same immunogenic properties of said protein in kind, not necessarily in amount. An analogue of a protein of the invention for instance comprises a fusion protein and/or chimaeric protein.

In order to be capable of eliciting an immune response, an immunogenic part, derivative and/or analogue according to the invention is preferably provided with the proper characteristics to enable antibody and/or immune cell production. Said characteristics, which are well known in the art, for instance include suitable flanking sequences and/or proteolytic cleavage sites. Alternatively, or additionally, a protein, immunogenic part, derivative and/or analogue according to the invention is preferably provided with an immunogenic carrier.

Once a protein or an immunogenic part, derivative and/or analogue according to the invention is administered to a human individual or non-human animal, it is usually at risk of degradation caused by a number of different forces, such as for example proteolysis, unfolding, extreme pH values, detergents and high salt concentrations. To prolong the life of a protein or an immunogenic part, derivative and/or analogue thereof, its resistance to degradation is preferably enhanced, for example by synthesizing a peptide with a C-terminal carboxamide and/or acetylating the N-terminal end of a peptide in order to maintain the native charge characteristics. In one embodiment resistance to degradation is further enhanced by mutating a protein or an immunogenic part, derivative and/or analogue according to the invention such that a local unfolding process rendering said protein or immunogenic part, derivative and/or analogue thereof susceptible to autolysis is at least in part inhibited. Stabilizing mutation strategies are known and for instance described by Matthews (1991), Alber (1991), Vriend and Eijsink (1993) and Fersht and Serrano (1993).

A secreted protein is defined as a protein which is naturally produced in a cell and/or organism and at least in part secreted from said cell and/or organism into its environment. Hence, if *Streptococcus* is cultured, a secreted protein is at least in part present in at least part of the culture medium, at least at some time point. A secreted protein needs not be produced and/or secreted continuously. A secreted protein may for instance only be produced and/or secreted during a certain phase of a bacterial life cycle. Furthermore, production and secretion of a secreted protein need not occur at the same time. For instance, some secreted proteins firstly accumulate inside a cell and are secreted at a later time point.

A surface-associated protein is defined as a protein which naturally forms part of a surface of a cell, or which is attached to a surface of a cell. If said surface-associated cell is attached to a surface of a cell, it is either directly or indirectly attached. Indirect attachment for instance involves the presence of at least one linker.

The term "isolated protein" refers to a protein which is at least in part isolated from its natural environment, and/or to a protein which is devoid of at least part of a sequence normally associated with it in nature.

The term "recombinant protein" refers to a protein which is produced by an isolated and/or artificial expression system, preferably using a nucleic acid sequence encoding said protein. Said nucleic acid sequence is preferably operably linked to at least one regulatory sequence such as for instance a promoter, an enhancer and/or a terminater. Preferably said regulatory sequence is inducible, so that it is possible to control the extent of expression of said protein. In one embodiment said nucleic acid sequence comprises an exogenous nucleic acid sequence. An exogenous nucleic acid sequence is a nucleic acid sequence which is present at a site in an organism's genome where said nucleic acid sequence is not naturally present.

After a *Streptococcus* protein capable of eliciting an immune response against at least two *Streptococcus* strains and/or serotypes is identified by a method of the invention, it is preferably produced. Produced protein is for instance suitable for generating an immunogenic composition and/or eliciting an immune response against at least two *Streptococcus* strains and/or serotypes in an animal. As outlined above, various methods for producing a protein are known in the art, such as for instance recombinant production. The invention therefore provides a method for producing at least one protein identified by a method of the invention. A *Streptococcus* protein which is capable of eliciting an immune response against at least two strains and/or serotypes of *Streptococcus* obtainable by a method of the invention is also herewith provided.

A *Streptococcus* protein and/or immunogenic part, derivative and/or analogue according to the invention is particularly suitable for preparing an immunogenic composition. Said immunogenic composition is capable of eliciting a broad humoral and/or cellular immune response against at least two *Streptococcus* strains. Preferably a *Streptococcus* protein and/or immunogenic part, derivative and/or analogue capable of eliciting an immune response against at least two *Streptococcus* serotypes is used for preparing an immunogenic composition, so that a broad immune response against at least two *Streptococcus* serotypes is achieved. A use of a protein obtainable by a method of the invention, or an immunogenic part, derivative and/or analogue thereof, for the preparation of an immunogenic composition capable of eliciting an immune response against at least two *Streptococcus* strains and/or serotypes is therefore also provided, as well as an immunogenic composition capable of eliciting an immune response against at least two *Streptococcus* strains and/or serotypes comprising at least one isolated and/or recombinant protein obtainable by a method of the invention, or an immunogenic part, derivative and/or analogue thereof. In order to provide an even broader protection, at least two or more proteins and/or immunogenic parts, derivatives and/or analogues of the invention are preferably used for the preparation of an immunogenic composition. In one embodiment a combination of at least one protein and at least one immunogenic part, derivative and/or analogue according to the invention is used for the preparation of an immunogenic composition. Besides a broader protection, the use of at least two proteins and/or immunogenic parts, derivatives and/or analogues of the invention decreases the chance of development of escape mutants of *Streptococcus* organisms. Escape mutants of bacterial organisms generally develop under environmental stress, for example in the presence of an antibiotic and/or in the presence of antibodies against an epitope of said organism. By natural variation in the population of an organism some organisms escape from the inhibitory effect of said environmental stress, such as the presence of said antibiotic and/or antibodies, and are capable of multiplying. The chance of development of an escape mutant for several different epitopes at one time is smaller than the chance of development of an escape mutant for only one epitope.

Hence, an immunogenic composition of the invention preferably comprises at least two isolated and/or recombinant proteins, and/or at least one immunogenic part, derivative and/or analogue thereof, obtainable by a method of the invention. In order to even better avoid the formation of escape mutants a protein of the invention preferably comprises an essential protein. This is a protein that is important - preferably essential - for the metabolism, survival and/or multiplication of *Streptococcus.* Hence, a possible escape mutant with an altered essential protein is less - if at all - viable.

Tables 5 and 6 comprise a list of preferred *Streptococcus* uberis proteins that are identified by a method of the invention. These proteins, or at least one immunogenic part, derivative and/or analogue thereof, are suitable for the preparation of an immunogenic composition of the invention. A use of the invention wherein said protein is selected from Table 5 and/or Table 6 is therefore also provided, as well as an immunogenic composition of the invention comprising at least one isolated and/or recombinant protein as depicted in Table 5 and/or 6, or an immunogenic part, derivative and/or analogue thereof. In order to provide an even broader protection, said immunogenic composition preferably comprises at least two proteins as depicted in Table 5 and/or Table 6, and/or immunogenic parts, derivatives and/or analogues thereof. Most preferably, said immunogenic composition comprises at least three proteins as depicted in Table 5 and/or Table 6, and/or immunogenic parts, derivatives and/or analogues thereof.

In a preferred embodiment, the at least one, at least two or at least three proteins as depicted in Table 5 and/or Table 6 are taken from the group consisting of P15, P16, P17, P19, P20, P22, P27, P54, P28, P63, P64, P68, P75, P81, P93, P100, P105, surface exclusion protein, trigger factor (ropA), and nucleoside diphosphate kinase. These proteins are either recognized by antibodies present in sera of *S*. *uberis* infected animals, indicating that these proteins are expressed in vivo and are immunogenic in cows, or are cross-reactive between at least two strains of *S*. *uberis* as depicted in Table 5. The numbering of proteins above, characterized for instance in Table 5, refers to the proteins depicted in for instance Tables 1, 2 and 3 which show non-limiting examples of *S*. *uberis* common surface proteins. Further, Figure 4 shows non-limiting examples of nucleic acid and amino acid sequences of these selected putative surface proteins/virulence factors of *S*. *uberis.*

Proteins that are highly conserved, expressed in vivo and highly immunogenic, such as proteins that are recognized by convalescent sera from cows infected with different strains as shown in Example 11, are especially useful in an immunogenic composition according to the invention. In an even more preferred embodiment therefore, the selection of proteins from Table 5 and/or 6 comprises a protein selected from the group consisting of P15, P16, P20, P27, P54, P28, P63, P68, P93, and P105. Most preferably, the selection of proteins from Table 5 and/or Table 6 comprises a protein selected from the group consisting of P15, P16, P54, P28, P63, and P105. As shown in Example 11, the latter selection was recognized by all convalescent sera used, indicating that these antigens are expressed by all S. uberis strains that cause the respective infection, that these antigens are expressed during infection in the host and that these antigens are highly immunogenic.

Yet another embodiment provides an immunogenic composition capable of eliciting an immune response against at least two strains and/or serotypes of *Streptococcus* comprising at least one nucleic acid molecule encoding at least one protein obtainable by a method of the invention, or an immunogenic part, derivative and/or analogue of said protein. Upon administration of said immunogenic composition to an animal, said nucleic acid molecule is expressed by the animal's machinery, resulting in expression of at least one protein and/or immunogenic part, derivative and/or analogue according to the invention. The production and, optionally, extracellular excretion of said protein and/or immunogenic part, derivative and/or analogue results in an immune response.

In one embodiment a protein of the invention and/or an immunogenic part, derivative and/or analogue thereof is produced recombinantly. The invention provides a method for producing an immunogenic composition capable of eliciting an immune response against at least two strains and/or serotypes of *Streptococcus,* said method comprising providing a cell or another expression system with at least one recombinant vector, said at least one vector comprising a nucleic acid sequence encoding at least one protein obtainable by a method of the invention and/or at least one protein selected from Table 5 and/or 6, and/or an immunogenic part, derivative and/or analogue of said protein. Suitable expression systems are known in the art. In one embodiment at least one nucleic acid sequence encoding one protein of the invention or an immunogenic part thereof is expressed. In another embodiment at least one nucleic acid molecule encoding at least two proteins and/or immunogenic parts is used. It is also possible to use at least two nucleic acid molecules, each nucleic acid molecule encoding one or more proteins and/or immunogenic parts according to the invention, et cetera. For instance, one nucleic acid molecule encoding (at least) one protein and one nucleic acid molecule encoding (at least) one immunogenic part are suitable. Hence, variations of the number of nucleic acid molecules and the number of proteins and/or immunogenic parts encoded by said nucleic acid molecules are possible.

A nucleic acid sequence of the invention is for example inserted into the genome of a cell by homologous recombination. It is also possible to insert a nucleic acid sequence at random, for instance by electroporation. Alternatively, or additionally, said nucleic acid sequence is placed into a vector such as for instance a plasmid vector or a phage vector, which vector is stable in a selected expression system such as a microorganism and/or a cell. Said nucleic acid sequence of the invention is preferably transcribed and translated under the control of a regulatory sequence such as for instance a promoter, enhancer and/or terminator. Preferably said promoter, enhancer and/or terminator is suitable for use in the selected expression system. More preferably, said regulatory sequence is inducible in order to allow for controlled expression. Promoters and terminators suitable for various micro-organisms are disclosed in (Biseibutsugaku Kisokoza (Basic Microbiology), Vol. 8, Genetic Technology, Kyoritsu Shuppan (1990)). For example, suitable plasmid vectors for *Escherichia,* more specifically for *Escherichia coli* are the plasmids of the pBR and pUC series, and suitable promoters for instance comprise *lac* promoter (ß-galactosidase), *trp* operon (tryptofaan operon), and *tac* promoter (*lac-trp* hybride promoter) and promoters derived from λ-faag PL or PR. Preferred terminators comprise *trpA-* or phage derived *rrn*B ribosomal terminator. Plasmid vectors suitable for recombinant production in *Streptococcus* comprise for example pHV1301 (FEMS Microbiol. Lett. 26, 239 (1985)) and pGK1 (Appl. Environ. Microbiol. 50, 94 (1985)).

The invention thus provides a recombinant nucleic acid molecule comprising a nucleic acid sequence encoding at least two *Streptococcus* proteins obtainable by a method of the invention and/or selected from Table 5 and/or 6, and/or an immunogenic part of at least one of said proteins, under the control of a functionally linked regulatory sequence such as for instance a promoter. An isolated host cell comprising a nucleic acid sequence encoding at least two proteins obtainable by a method of the invention and/or selected from Table 5 and/or 6, and/or an immunogenic part thereof, is also herewith provided. Said host cell preferably comprises a prokaryotic host cell.

In a preferred embodiment a nucleic acid molecule of the invention is used for eliciting an immune response against *Streptococcus.* This is preferably performed with a recombinant carrier comprising a nucleic acid encoding at least one protein obtainable by a method of the invention and/or selected from Table 5 and/or 6 and/or an immunogenic part of said at least one protein, or a recombinant nucleic acid molecule of the invention. Said recombinant carrier is therefore also herewith provided. Most preferably a recombinant carrier comprising a nucleic acid encoding at least one protein selected from Table 5 and/or 6 is provided. In one particularly preferred embodiment said recombinant carrier comprises a nucleic acid encoding at least two proteins selected from Table 5 and/or 6. In one embodiment, said recombinant carrier is allowed to produce at least one protein of the invention, after which a combination of the at least one recombinant protein and the carrier itself is used for eliciting an immune response against at least two *Streptococcus* strains and/or serotypes. In one embodiment a killed recombinant carrier of the invention is provided. One preferred embodiment however provides a live recombinant carrier of the invention. In one embodiment said live carrier is an attenuated carrier. A live carrier of the invention is preferably capable of infecting a human individual and/or a non-human animal, after which an immune response against at least two strains and/or serotypes of *Streptococcus* is elicited.

A recombinant carrier of the invention preferably comprises a *Streptococcus* species. This way an immune response directed against *Streptococcus* is both elicited by the protein(s) and/or immunogenic part(s), derivative(s) and/or analogue(s) encoded by said carrier, and by said recombinant carrier itself.

Capsular gene expression products of *Streptococcus* are often highly immunogenic and serotype-specific. Hence, the presence of capsular gene expression products hampers the induction of an immune response directed against various different strains and/or serotypes of *Streptococcus.* In one embodiment, therefore, if a recombinant carrier of the invention comprises a *Streptococcus,* said *Streptococcus* is lacking at least part of a capsular gene expression product. In one embodiment said *Streptococcus* is a non-capsular streptococcus.

As described above, immunization with at least two proteins and/or immunogenic parts, derivatives and/or analogues derived from at least two different *Streptococcus* strains and/or serotypes provides a broad protection and diminishes the chance of the formation of escape mutants. A preferred embodiment of the invention therefore provides a recombinant carrier of the invention comprising a nucleic acid sequence encoding at least one protein and/or immunogenic part thereof derived from a first *Streptococcus* strain and/or serotype, and a nucleic acid sequence encoding at least one protein and/or immunogenic part thereof derived from a second *Streptococcus* strain and/or serotype. Said recombinant carrier preferably comprises a live recombinant carrier.

A recombinant carrier is for instance produced in a suitable host cell. An isolated host cell comprising a recombinant carrier of the invention is therefore also provided.

A recombinant carrier of the invention is suitable for the production of an immunogenic composition capable of eliciting an immune response against at least two strains and/or serotypes of *Streptococcus.* An immunogenic composition capable of eliciting an immune response against *Streptococcus,* said composition comprising a recombinant carrier of the invention is therefore also provided herein.

After administration of an immunogenic composition of the invention to a human individual and/or a non-human animal, an immune response against *Streptococcus* is elicited. Said immune response is preferably capable of at least in part counteracting *a Streptococcus* related disease. An immunogenic composition of the invention for use as a medicament is therefore also herewith provided, as well as a use of an immunogenic composition of the invention for the preparation of a medicament against a *Streptococcus* related disease.

An immunogenic composition of the invention is also suitable for the production of a vaccine. Said vaccine is preferably capable of at least in part providing protection against a *Streptococcus* related disease. Preferably, said vaccine is capable of providing protection against a *Streptococcus* infection. The invention therefore provides a use of an immunogenic composition of the invention for the preparation of a vaccine.

A protein, immunogenic part, derivative, analogue and/or recombinant carrier of the invention is preferably administered to a human individual and/or non-human animal together with a suitable carrier. Said carrier preferably facilitates the acceptance by said human individual and/or animal of said protein, immunogenic part, derivative, analogue and/or recombinant carrier of the invention and preferably increases the immunogenic effect. A suitable carrier of the invention for instance comprises a suitable adjuvant capable of increasing an immunising effect of an immunogenic composition of the invention. Many suitable adjuvants, oil-based and water-based, are known to a person skilled in the art. In one embodiment said adjuvant comprises Diluvac Forte and/or Specol. In another embodiment, said suitable carrier comprises a solution like for example saline, for instance for diluting proteins or immunogenic parts, derivatives and/or analogues thereof. Therefore, the present invention also discloses an immunogenic composition of the invention comprising at least one protein, immunogenic part, derivative, analogue and/or recombinant carrier of the invention and a suitable carrier.

An immunogenic composition of the invention is capable of eliciting an immune response against *Streptococcus* in a human individual and/or non-human animal and thereby decreasing and/or controlling the number of *Streptococcus* organisms in said individual and/or animal. The invention therefore provides a method for decreasing and/or controlling the number of *Streptococcus* organisms in a human individual and/or non-human animal comprising providing said individual and/or non-human animal with an immunogenic composition of the invention.

An immunogenic composition of the invention is preferably capable of at least in part counteracting and/or preventing a *Streptococcus* related disease. Once a *Streptococcus* related disease is already present, an immunogenic composition of the invention is preferably capable of at least in part counteracting said disease. A pharmaceutical composition comprising an immunogenic composition of the invention and, preferably, a suitable carrier such as for instance Diluvac Forte and/or Specol is therefore also herewith provided.

A further embodiment of the invention provides a method for measuring the immunity of a human individual and/or non-human animal against *Streptococcus,* said method comprising determining in at least one sample from said individual and/or animal the presence of antibodies and/or immune cells directed against a protein obtainable by a method of the invention and/or selected from Table 5 and/or 6, or an immunogenic part thereof. A diagnostic kit comprising at least one protein obtainable by a method of the invention and/or selected from Table 5 and/or 6, or an immunogenic part thereof, and a means for detecting antibody binding and/or immune cell binding to said protein or immunogenic part thereof is also herewith provided. In a particularly preferred embodiment said diagnostic kit comprises at least two proteins selected from Table 5 and/or 6.

### Detailed description

A method according to the invention is in a preferred embodiment applied for identifying a *Streptococcus uberis* protein which is capable of eliciting an immune response against at least two strains and/or seroypes of *Streptococcus uberis.* Such *Streptococcus uberis* protein is preferably used for the preparation of an immunogenic composition capable of eliciting an immune response against at least two strains and/or serotypes of *Streptococcus uberis.* An immunogenic composition capable of eliciting an immune response against at least two strains and/or serotypes of *Streptococcus uberis* comprising at least one, preferably at least two, isolated and/or recombinant protein(s) obtainable by a method according to the present invention, or at least one immunogenic part, derivative and/or analogue thereof, is therefore also herewith provided, as well as uses thereof for the preparation of a medicament against *Streptococcus uberis* mastitis. The invention furthermore provides an isolated or recombinant nucleic acid molecule comprising a nucleic acid sequence encoding at least two *Streptococcus uberis* proteins obtainable by a method according to the present invention, and/or selected from Table 5 and/or 6. Further provided are recombinant carriers, host cells and immunogenic compositions comprising said nucleic acid, as well as uses thereof.

*Streptococcus uberis* is associated with bovine mastitis. Bovine mastitis is an infection of the mammary gland of a cow, usually caused by bacteria. The inflammatory response following infection results in decreased yield and quality of the milk, and causes major annual economic losses to the dairy industry. The economic damage in the Netherlands is estimated to be around 100 Euro per cow per year.

Among the bacterial species most commonly associated with mastitis are various species of the genus *Streptococcus,* including *Streptococcus uberis* (untypeable), *Streptococcus agalactiae* (Lancefield group B), *Streptococcus dysgalactiae* (Lancefield group C), *Streptococcus zooepidemicus,* and the Lancefield groups D, G, L and N streptococci. Some of those species are contagious (e.g. *S. agalactiae),* while others are considered environmental pathogens (e.g. *S*. *dysgalactiae* and *S.uberis*).

Mastitis resulting from infection with *S.uberis* is commonly sub-clinical, characterized by apparently normal milk with an increase in somatic cell counts due to the influx of leukocytes.

Mastitis varies in severity according to the clinical effects caused by the infection. A mild form of mastitis may cause some rise in body temperature, and/or increase in temperature of the udder. In more severe cases, *S.uberis* mastitis may also take the form of an acute clinical condition, with obvious signs of disease such as clots or discoloration of the milk and swelling or hardness of the mammary gland. Some cases of the clinical disease can be severe and pyrexia may be present. For a review of the clinical manifestations of *S.uberis* mastitis, see Bramley (1991); and Schalm et al. (1971).

Conventional antibacterial control methods such as teat dipping and antibiotic therapy are effective in the control of many types of contagious mastitis, but the environmental organisms typically found in all dairy barns are often resistant to such measures. These measures have therefore not influenced the incidence of mastitis caused by environmental pathogens such as *Streptococcus uberis* and *Escherichia coli* that are now responsible for over 95% of cases of mastitis. From these two species, *S.uberis* is the most important environmental pathogen, as shown by surveys executed in the United Kingdom (Hillerton et al., 1993), in New Zealand (McDougall, 1998), in the US (Hogan et al., 1989), and in the Netherlands (Animal Health Service, 2000). There is also evidence that *S.uberis,* once infection is established from the environment, can directly spread from an infected cow to a susceptible animal (Neave et al., 1969, Oliver et al., 1999, Zadoks et al., 2001). There are several strains of *S.uberis* that differ in virulence and antigenicity.

The failure of current methods aiming at *S.uberis* mastitis control has led to the search for alternative control measures such as more effective vaccines. Several types of vaccines have been developed up to now and have been tested in cows.

Repeated immunization of dairy cattle with killed whole bacteria resulted in reduction of the number of bacteria present in the milk following experimental challenge with the same strain (Leigh, 1999; Leigh, 2000). The killed vaccine did however not prevent the infection nor the inflammatory response in the mammary gland, and had no effect on the incidence of *S.uberis* mastitis in the field (Leigh, 1999). Therefore, it was concluded that immunization with killed bacteria was not a solution to the problem of *S. uberis* mastitis.

Immunization with live *S.uberis* induced partial protection against experimental challenge with the same (or homologous) strain (Finch et al., 1997). Protection was achieved in the absence of opsonising activity and without a large influx of neutrophils. However, the vaccine did not seem to protect against other *S.uberis* strains. The relative low success with these whole cell vaccine approaches indicates that it is difficult to protect an animal against S.uberis using conventional whole bacteria vaccines.

More recently, a subunit vaccine was produced, based on one protein of *S.uberis.* (Fontaine *et al.* 2002). The publication of said subunit vaccine has up to now not led to a follow up, which has led to the conclusion that the chances on finding a single protein that will protect an animal against several types of *S. uberis* are small and subunit vaccines of this kind generally are not the answer to the problem of controlling *S.uberis* mastitis.

In summary, mastitis caused by *S. uberis* is not effectively prevented or cured by vaccination with either whole, life or killed bacteria or with a subunit vaccine comprising one protein.

Despite the above-described discouraging results of vaccination against *S.uberis* mastitis, we here disclose that mastitis caused by a variety of *S.uberis* strains is successfully prevented and/or diminished by using an antigenic composition capable of eliciting an immune response against *S. uberis* according to the invention.

The present invention provides a method for identifying a *Streptococcus uberis* protein which is capable of eliciting an immune response against at least two *Streptococcus uberis* strains and/or types, the method comprising:
a) identifying at least part of a secreted protein, a surface-associated protein and/or a protein which has at least 50% sequence identity to a bacterial virulence factor;
b) selecting at least one protein identified in step a) which is conserved over at least two *Streptococcus uberis* strains and/or types; and
c) determining whether at least one protein selected in step b) or an immunogenic part, derivative and/or analogue thereof is capable of specifically binding an antibody and/or immune cell of an animal infected by a first *Streptococcus uberis* strain and/or type, and an antibody and/or immune cell of an animal infected by a second *Streptococcus uberis* strain and/or type. Preferably, said protein which has at least 50% sequence identity to a bacterial virulence factor has at least 60%, more preferably at least 70 %, more preferably at least 75%, most preferably at least 80% sequence identity to a bacterial virulence factor.

The present invention furthermore discloses that a combination of at least two isolated or recombinant *S.uberis* surface proteins or an immunogenic part thereof in an antigenic composition enhances the immune response against *S.uberis* strains considerably. Whereas whole bacterial cell vaccines, comprising many bacterial immunogenic proteins do not elicit a broad protection against various *S.uberis* strains, two or more proteins or an immunogenic part thereof in an immunogenic composition of the invention have the desired effect of enhancing the immune response against *S.uberis.* We disclose in the present invention that selecting at least two immunogenic proteins or an immunogenic part thereof of a *S.uberis* organism, and preferably of at least two strains or types of *S.uberis* organisms and combining said at least two immunogenic proteins or an immunogenic part thereof in a immunogenic composition enhances the immunity against different strains of *S.uberis* because the immune response is directed against a broader range of different *S.uberis*organisms.

For eliciting an immune response in a subject or an animal, preferably an immunogenic part of a protein is presented to said subject or animal. In this invention, the term "immunogenic site" is used interchangeably with the term "immunogenic part". By "immunogenic part or site" is meant a part of a protein, which is capable of eliciting an immunological response in a subject. Preferably said immunogenic part of a protein comprises one or more epitopes and thus elicits an immunological response. An immunogenic part comprises at least 5 amino acids, preferably at least 10-15, and most preferably 25 or more consecutive amino acids. Therefore, the invention in another embodiment provides a protein or an immunogenic part thereof comprising at least a stretch of 30 consecutive amino acids of a proteinaceous molecule encoded by a nucleic acid according to the invention. A conformational epitope is generally formed by several stretches of consecutive amino acids that are folded in position and together form an epitope when the protein takes on its three dimensional structure. The present invention also discloses the use of conformational epitopes as immunogenic parts.

A derivative of a protein is defined as a protein, which has the same kind of immunogenic properties in kind, not necessarily in amount. A person skilled in the art is capable of altering a protein such that the immunogenic properties of said molecule are essentially the same in kind, not necessarily in amount. A derivative of a protein can be provided in many ways, for instance through conservative amino acid substitution, for example by replacement of one amino acid in a protein by another amino acid. In conventional replacement mapping, preferably conservative changes are made, like for example replacement of an amino acid comprising an acidic side chain by another amino acid comprising an acidic side chain, bulky amino acids by bulky amino acids, amino acids comprising a basic side chain by amino acids comprising a basic side chain, amino acids comprising an uncharged polar side chain by amino acids comprising an uncharged polar side chain, and amino acids comprising an nonpolar side chain by amino acids comprising an nonpolar side chain. A person skilled in the art is well able to generate analogous compounds of a protein. This is for instance done through screening of a peptide library or by peptide changing programs. For use as an immunogen, a peptide is synthesized with the proper characteristics to insure high probability of success in antibody production. These include a C-terminal free carboxyl group if the peptide is the actual C-terminal sequence of the native protein and a free N-terminal amino group if the peptide is the actual N-terminal sequence of the native protein. Such an analogue has essentially the same immunogenic properties of said protein in kind, not necessarily in amount.

A protein or peptide is subject to degradation by a number of different forces, such as for example proteolysis, unfolding, extreme pH values, detergents and high salt concentrations. To prolongue the life of a recombinant protein or peptide, said protein or peptide is made more stable to withstand degradation, for example by synthesizing said peptide with a C-terminal carboxamide and/or acetylating the N-terminal end in order to maintain the native charge characteristics. This is further done by mutations using a stabilizing mutation strategy to inhibit the local unfolding processes that generally render the protein susceptible to autolysis. Said stabilizing mutation strategy is based on generally accepted principles of protein structure and stability as described by for example Matthews (1991), Alber (1989), Vriend and Eijsink (1993) and Fersht and Serrano (1993).

In one embodiment an immunogenic composition of the invention comprises a composition comprising at least two recombinant or isolated surface proteins or a derivative or an analogue, and/or immunogenic parts thereof, wherein administration of the composition to a subject or an animal, preferably a cow, results in the development of a humoral and/or a cellular immune response to said surface proteins or immunogenic parts thereof.

An immunological response comprises the development of a humoral and/or a cellular immune response directed against said protein or immunogenic part thereof in a subject or an animal, preferably a cow. A humoral immune response leads to the production of antibodies in a subject or an animal, whereas the cellular immune response predominantly enhances the formation of reactive immune cells. In general, both parts of the immune response are elicited by administration of an immunogenic protein or part thereof. A preferred immune response against *S. uberis* is antibody production. Preferably, said immune response prevents and/or decreases mastitis, and/or decreases the number of *S.uberis* organisms in the udder. The present invention discloses methods to select and produce proteins and epitopes for eliciting said antibody response. Another preferred immune response against *S.uberis* is the cellular immune response. The present invention also discloses methods to select T-cell epitopes of surface proteins, and to produce T-cell epitopes causing an enhanced T-cell reactivity, for example by coupling multiple pre-selected T-cell epitopes in a string-of bead fashion as for example described by Van der Burg et al (WO 97/41440). In one embodiment of the invention, said immunogenic composition is capable of decreasing the duration and/or severity of the infection and/or increasing the resistance of the animal to *S.uberis*infection.

The present invention discloses that an immune response directed against the outside of *S.uberis* is preferred. Therefore, the present invention discloses an immunogenic composition or an immunogenic part thereof that is capable of eliciting an immune response to antigens that are preferably located in or near the cell surface of *S.uberis.* A surface protein of the invention comprises proteins that are in nature preferably near or on the surface of a *S.uberis* bacterium, and/or proteins that are in nature preferably produced and/or excreted extracellular by an *S.uberis* bacterium. Said surface proteins preferably have homologous proteins in other strains of *S.uberis.* Therefore, the immune response elicited with immunogenic proteins or parts thereof, derived from one strain of *S.uberis,* is also effective against other strains of *S.uberis.* Thus the present invention discloses an immunogenic composition capable of eliciting an immune response against *S.uberis,* said composition comprising at least two recombinant and/or isolated surface proteins derived from *Streptococcus uberis,* and/or an immunogenic part of either or both of said proteins.

The term: "recombinant protein" refers to a protein produced by recombinant DNA techniques; i.e., produced by a cell transformed by a nucleic acid construct encoding the desired protein. Said nucleic acid construct is for example a recombinant DNA construct with a regulatory sequence such as a promoter and/or a terminator sequence, and/or an enhancer sequence, which controls the expression sequence.

The term: "isolated protein" refers to a protein separate and discrete from the whole organism, with which the molecule is found in nature; and/or a protein devoid, in whole or in part, of substances normally associated with it in nature.

Said immunogenic composition comprises either at least two proteins or an immunogenic part thereof derived from the same *S. uberis* organism or it comprises at least one protein or an immunogenic part thereof from one type of *S. uberis* and at least one protein or an immunogenic part thereof from another type of *S.uberis.*The invention also discloses the combination of at least 3 or 4 or more proteins or an immunogenic part thereof, of which one or two or more are derived from other types of *S. uberis.*

Preferably, the immunogenic composition or an immunogenic part thereof of the invention comprises proteins of at least two different *S. uberis* organisms, because the resulting broad immune response is cross-protecting i.e. is directed against different types of *S.uberis.* Furthermore, the use of immunogenic proteins or an immunogenic part thereof of at least two types of *S.uberis* strains decreases the chances of development of escape mutants of *S. uberis* organisms. Escape mutants of bacterial organisms generally develop under environmental stress, for example in the presence of an antibiotic or in the presence of antibodies against an epitope of said organism. By natural variation such as for example caused by a low mutation frequency in the population of an organism, some organisms of said population are more inhibited in their replication by said antibodies, than others, which escape from the inhibitory effect of the presence of said antibodies and keep multiplicating, thereby obtaining a predominant role in the new population. The chance of development of an escape mutant for several different epitopes at one time is smaller than the chance of development of an escape mutant for only one epitope. An immunogenic composition and/or an immunogenic part thereof preferably elicits an immune response against at least two proteins preferably causing a broad protection against infection and decrease of clinical signs of mastitis. Therefore, the present application provides an immunogenic composition capable of eliciting an immune response against *Streptococcus uberis* comprising at least two recombinant and/or isolated surface proteins derived from at least one *Streptococcus uberis* strain, and/or an immunogenic part or analogue or derivative of either or both of said proteins.

Proteins that are important for the metabolism or survival or multiplication of a bacterial organism are generally known as essential proteins of an organism. The sequence and function of said essential proteins is generally rather conserved between different types of *S.uberis.* In a preferred embodiment of the invention, said immunogenic proteins are essential proteins of an *S.uberis.*In this way, the immune response is directed against an essential protein or an immunogenic part thereof, thus forming an defence against an homologous *S.uberis* organism, but also a cross-reactive defence against different types of *S. uberis,* because said conserved protein or essential protein is also present on the surface of other types of *S.uberis.* Therefore, the use of an essential surface protein of an *S.uberis* organism as an immunogenic protein of the invention increases the protective efficacy of the immune response against infection with different types of *S.uberis* organisms and decreases the possibility of said organisms to escape the immune response.

Capsular antigens of *S.uberis* are generally good immunogenic epitopes, because capsular antigens are readily detected by convalescent sera of cows, (which have endured an *S.uberis* mastitis). Said immunogenic properties are capable of enhancing the immune response against related *S.uberis* immunogenic epitopes. Therefore, in another embodiment, the immunogenic composition comprises at least one capsular antigen in addition to the immunogenic proteins, because said capsular antigen increases the immune response against said immunogenic composition.

The present patent application discloses in Table 5 and Table 6 preferred recombinant and or isolated surface proteins derived from *S. uberis* and selected for their capability of eliciting an immune response against different strains of *S.uberis.* Therefore, the present application provides an immunogenic composition of the invention, and/or an immunogenic part or analogue or derivative of either or both of said proteins wherein at least two proteins are selected from Table 5 and/or Table 6.

In a preferred embodiment of the invention, a selection is made from the proteins of Table 5 and/or Table 6 and a combination is made of two or more proteins like for example protein no 63 and/or an immunogenic part thereof from *S. uberis* strain 0140J, together with protein no 15 or 22 and/or both and/or an immunogenic part thereof from *S. uberis* strain 41-241. Such a selection provides proteins or immunogenic parts thereof from two different strains of *S. uberis,* thereby providing broad protection for several strains of *S. uberis.*

In a preferred embodiment the selection of proteins from Table 5 and/or Table 6 comprises a protein selected from the group consisting of P15, P16, P17, P19, P20, P22, P27, P54, P28, P63, P64, P68, P75, P81, P93, P100, and P105. As said before,these proteins are either recognized by antibodies present in sera of *S. uberis* infected animals, indicating that these proteins are expressed in vivo and are immunogenic in cows, or are cross-reactive between at least two strains of *S. uberis* as depicted in Table 5.

The proteins as identified in Example 11, are especially useful for eliciting an immune response. In an even more preferred embodiment therefore, the selection of proteins from Table 5 and/or 6 comprise a protein selected from the group consisting of P15, P16, P20, P27, P54, P28, P63, P68, P93, and P105. Most preferably, the selection of proteins from Table 5 and/or Table 6 comprises a protein selected from the group consisting of P15, P16, P54, P28, P63, and P105. As said before, the latter selection of proteins is expressed by all *S. uberis* strains that cause the respective infection of Example 11, are expressed during infection in the host and are highly immunogenic. The numbering of proteins above, characterized for instance in Table 5, refers to the proteins depicted in for instance Tables 1, 2 and 3 which show non-limiting examples of *S. uberis* common surface proteins. Further, Figure 4 shows non-limiting examples of nucleic acid and amino acid sequences of these selected putative surface proteins/virulence factors of *S. uberis.*

A low number of bacteria in the milk or on or in an udder is often found under field conditions and does not need to be harmful to an animal. Mastitis may develop when the number of bacteria, for example *S. uberis* organisms, increases in the milk or in the udder. An immune response, elicited by proteins or immunogenic parts thereof according to the invention, is preferably effective in inhibiting at least in part the bacterial growth of *Streptococcus uberis* organisms in an udder. Decreasing the numbers of *S. uberis* organisms in the direct environment also helps preventing mastitis. The present invention discloses how to prevent and/or decrease *S. uberis* mastitis by immunising the cows, thereby keeping the number of S.uberis organisms low. Said low level of S.uberis organisms is further kept low by applying a hygienic regime at milking for example by cleaning the udder, the teats, and all apparatuses that come into contact with the udder and/or the teats.

Recombinant and/or isolated surface proteins derived from *S. uberis,* as provided by the invention, are in one embodiment produced by a production system using a prokaryotic cell or a eukaryotic cell. Examples of cells with a well developed host/vector systems for production of recombinant protein are for example for the bacteria: *Escherichia, Bacillus, Pseudomonas, Serratia, Brevibacterium, Corynebacterium, Streptococcus* and *Lactobacillus;* and for the yeasts: *Saccharomyces, Kluyveromyces, Schizosaccharomyces, Zygosaccharomyces, Yarrowia, Trichosporon, Rhodosporidium, Hansenula, Pichia* and *Candida;* and for the fungi: *Neurospora, Aspergillus, Cephalosporium* en *Trichoderma.*

For production of a recombinant protein of interest, the gene, encoding said protein or part thereof is either integrated in the genome for example by homologous recombination or at random, or said gene is placed in a plasmid vector or in a phage vector, which is stably maintained and expressed in the selected microorganism or cell. For the expression of the selected DNA construct in the microorganism or cell, the gene is transcribed and translated under the control of a promoter and a terminator. Preferably said promoter and terminator are suitable for the selected microorganism. Promoters and terminators suitable for various micro-organisms are disclosed in "Biseibutsugaku Kisokoza (Basic Microbiology), Vol. 8, Genetic Technology, Kyoritsu Shuppan (1990)", and those preferred for yeasts in "Adv. Biochem. Eng. 43, 75-102 (1990)" and in "Yeast 8, 423-488 (1992)". For example, suitable plasmid vectors for *Escherichia,* more specifically for *Escherichia coli* are the plasmids of the pBR and pUC series, and suitable promoters comprise *lac* promoter (ß-galactosidase), *trp* operon (tryptofaan operon), and *tac* promoter (*lac-trp* hybride promoter) and promoters derived from λ-faag PL or PR. Preferred terminators comprise *trp*A- or phage derived *rrn*B ribosomal terminator. Plasmid vectors suitable for recombinant production in *Streptococcus* comprise for example pHV1301 (FEMS Microbiol. Lett. 26, 239 (1985)) and pGK1 (Appl. Environ. Microbiol. 50, 94 (1985)). Plasmid vectors suitable for recombinant production in *Lactobacillus* comprise for example those disclosed for *Streptococcus,* like for example pAM61 (J. Bacteriol. 137, 614 (1979)). Plasmid vectors suitable for recombinant production in *Saccharomyces,* preferably *Saccharomyces cerevisiae,* comprise for example vectors of the series YRp, YEp, YCp en YIp. An integration vector (EP5327456) constructed by applying homologous recombination of ribosomal DNA with multicopy in the chromosome is suitable for the insertion of multicopy and for stable gene control. Plasmid vectors suitable for recombinant production in *Kluyveromyces,* preferably *Kluyveromyces lactis,* comprise for example the 2 µm plasmid series derived from *Saccharomyces cerevisiae,* plasmids of the pKD1 series (J. Bacteriol. 145, 382-390 (1981)), and pGK11-derived plasmid involved in killer activity, plasmid of the KARS series with the autonomous replication gene of *Kluyveromyces* and an integration vector (EP 537456). Plasmid vectors suitable for recombinant production in *Pichia* comprise for example the host vector system developed in *Pichia pastoris* using a gene, which is involved in autonomous replication in *Pichia* (Mol. Cell. Biol. 5, 3376 (1985). Plasmid vectors suitable for recombinant production in *Candida* comprise for example the host vector system developed in *Candida maltosa, Candida albicans* and *Candida tropicalis.* (Agri. Biol. Chem. 51, 51, 1587 (1987). Plasmid vectors suitable for recombinant production in *Aspergillus,* comprise for example a vector constructed by integration of the gene in the plasmid or chromosome and the promoter for extracellular protease or amylase (Trends in Biotechnology 7, 283-287 (1989)). Plasmid vectors suitable for recombinant production in *Trichoderma* comprise for example the host vector system developed in *Trichoderma reesei,* and the promoter for extracellular cellulase, which is suitable for construction of the vector (Biotechnology 7, 596-603 (1989)).

Preferably, said production system is provided with a nucleic acid construct, preferably a DNA construct, encoding for a protein of Table 5 and/or Table 6 or an immunogenic part thereof.

In another embodiment, said production system is provided with a DNA construct encoding for two or three or four or even more proteins of Table 5 and/or Table 6, or an immunogenic part thereof.

In yet another embodiment, said production system is provided with at least two DNA constructs, each encoding for at least one protein of Table 5 and/or Table 6, or an immunogenic part thereof.

In a preferred embodiment, said protein of Table 5 and/or 6 is selected from the group consisting of P15, P16, P17, P19, P20, P22, P27, P54, P28, P63, P64, P68, P75, P81, P93, P100, and P105. Even more preferred said protein of Table 5 and/or 6 is selected from the group consisting of P15, P16, P20, P27, P54, P28, P63, P68, P93, and P105. Most preferred, said protein of Table 5 and/or 6 is selected from the group consisting of P15, P16, P54, P28, P63, and P105.

In a more preferred embodiment, said nucleic acid construct encodes for a fusion protein, comprising immunogenic epitopes derived from more than one protein of *S.uberis.* More preferably, said fusion protein comprises epitopes derived from proteins derived from more than one *S.uberis* strain.

In another embodiment of the invention said nucleic acid construct encodes for epitopes, which have been modified to enhance the humoral and/or cellular immune response. Therefore, the present application provides a method for producing an immunogenic composition comprising at least two proteins of *S.uberis* capable of eliciting an immune response against *Streptococcus uberis,* said method comprising providing a cell with a recombinant vector, said vector comprising a nucleic acid encoding at least two proteins as listed in Table 5 and/or Table 6, and/or an immunogenic part or analogue or derivative of either or both of said proteins. For recombinant production of a protein from a nucleic acid, said nucleic acid is preferably placed under the control of an inducible regulatory sequence capable of enhancing the expression of said protein, preferably resulting in a higher protein yield. Preferably the accumulation of said recombinant protein or immunogenic part thereof is either cytoplasmic, for example in those cases wherein the produced recombinant protein is harvested from the cells, or the protein is excreted, for example when the produced protein is harvested from the culture fluid. Therefore, the recombinant construct encoding said immunogenic protein is provided with the correct regulatory sequences and/or a functionally linked promoter for intracellular or extracellular accumulation. Therefore, the present invention provides a recombinant molecule comprising a nucleic acid sequence encoding at least two proteins as listed in Table 5 and/or Table 6, and/or an immunogenic part or analogue or derivative of either or both of said proteins under the control of a functionally linked promoter.

In a preferred embodiment, said protein as listed in Table 5 and/or 6 is selected from the group consisting of P15, P16, P17, P19, P20, P22, P27, P54, P28, P63, P64, P68, P75, P81, P93, P100, and P105. Even more preferred said protein as listed in Table 5 and/or 6 is selected from the group consisting of P15, P16, P20, P27, P54, P28, P63, P68, P93, and P105. Most preferred, said protein as listed in Table 5 and/or 6 is selected from the group consisting of P15, P16, P54, P28, P63, and P105.

The application also provides a live recombinant carrier comprising a nucleic acid sequence of the invention or a recombinant DNA molecule of the invention. In a preferred embodiment, the carrier is a first *S. uberis* strain and the recombinant nucleic acid encodes immunogenic epitopes of another *S.uberis* strain. Therefore, the present invention provides a live recombinant carrier comprising a nucleic acid sequence encoding one or more proteins as listed in Table 5 and/or Table 6, and/or an immunogenic part or analogue or derivative of either or both of said proteins under the control of a functionally linked promoter. Of course, said live recombinant carrier is also immunogenic when killed. Therefore, the present invention also discloses a killed recombinant carrier.

The application further provides an isolated host cell comprising a nucleic acid sequence encoding one or more proteins as listed in Table 5 and/or Table 6, and/or an immunogenic part or analogue or derivative of either or both of said proteins under the control of a functionally linked promoter. An isolated host cell for example comprises a bacterial cell such as for example: *Escherichia, Bacillus, Pseudomonas, Serratia, Brevibacterium, Corynebacterium, Streptococcus uberis, Streptococcus suis, Lactobacillus,* or a yeast such as for example: *Saccharomyces, Kluyveromyces, Schizosaccharomyces, Zygosaccharomyces, Yarrowia, Trichosporon, Rhodosporidium, Hansenula, Pichia, Candida,* or a fungus such as for example: *Neurospora, Aspergillus, Cephalosporaum,andlor Trichoderma.*

With the abovementioned isolated host cell comprising a recombinant molecule comprising a nucleic acid sequence encoding one or more proteins as listed in Table 5 and/or table 6, and/or an immunogenic part or analogue or derivative of either or both of said proteins under the control of a functionally linked promoter the present invention discloses to a skilled person how to produce a recombinant proteinaceous molecule. Because of variation between different *S.uberis* strains, proteins and peptides from various strains may show a slight variation in amino acid sequence and yet have the same function. Therefore, a proteinaceous molecule derived from one *S.uberis*strain has sequence identity to a functionally identical proteinaceous molecule of another *S.uberis*strain. "Sequence identity" refers to the percent sequence identity between two amino acid sequences or nucleotide sequences after aligning the two sequences and introducing gaps, if necessary, to achieve the maximum percent sequence identity. Methods and computer programs for the alignment are well known in the art. One computer program which may be used or adapted for purposes of determining whether a candidate sequence falls within this definition is "Align 2", authored by Genentech, Inc., which was filed with user documentation in the United States Copyright Office, Washington, D.C. 20559, on Dec. 10, 1991. Two amino acid sequences, have a high degree of "sequence identity" to each other when the sequences exhibit at least about 80%, preferably at least about 90%, and most preferably at least about 95% sequence identity of the molecules after alignment. Therefore, the present application discloses an isolated and/or recombinant proteinaceous molecule that has at least 80% sequence identity to a protein encoded by a nucleic acid according the invention. In a preferred embodiment, the invention discloses an isolated and/or recombinant proteinaceous molecule that has at least 95% sequence identity to a proteinaceous molecule encoded by a nucleic acid according to the invention.

To induce or elicit an immune response in an animal against a protein or an immunogenic part of the invention, said animal is provided with a protein and/or an immunogenic part that comprises at least one immunogenic site. An immunogenic part or site of a protein is formed by one or more epitopes and thus is capable of eliciting an immunological response. An immunogenic site comprises preferably at least 5 amino acids, more preferably at least 10-15, and most preferably 25 or more consecutive amino acids. The invention in another preferred embodiment provides a protein or an immunogenic part thereof comprising at least a stretch of 25 consecutive amino acids of a proteinaceous molecule encoded by a nucleic acid according to the invention. Preferably said stretch of at least 25 consecutive amino acids comprises an immunogenic site. A recombinant nucleic acid molecule in a preferred embodiment encodes for at least one protein or a fusion protein encoding for at least two proteins or immunogenic parts thereof. In a preferred embodiment, said fusion protein comprises immunogenic parts of proteins of at least two strains of *S.uberis.* Therefore, the invention discloses a nucleic acid encoding a proteinaceous molecule according to the invention.

Expression of said nucleic acid in a host cell provides an immunogenic protein of the invention. Said immunogenic protein is preferably incorporated into an immunogenic composition of the invention. Therefore, the present invention provides an immunogenic composition capable of eliciting an immune response against *Streptococcus uberis,* said composition comprising an isolated and/or recombinant proteinaceous molecule according to the invention.

Said recombinant protein of the invention is produced by a host cell. As a host cell, bacterial species such as for example an *E.coli* and/or yeast or fungi or eukaryotic cells are used for the production.

An immunogenic composition incorporating an isolated or recombinant protein, and/or a cell exposing said protein is capable of eliciting an immune response against *Streptococcus uberis,* when administered to an animal, preferably a cow. Preferably, said cell comprising a nucleic acid of the invention under a suitable regulatory sequence expresses said protein on the surface. Such a cell is called a carrier cell. Said carrier cell preferably is incorporated in the immunogenic composition of the invention. Preferably, said cell carrying said immunogenic protein is a live recombinant carrier, but in another embodiment said carrier cell is capable of eliciting an immune response when the cell is killed by for example formalin treatment. Therefore, the present invention provides an immunogenic composition capable of eliciting an immune response against *Streptococcus uberis,* said composition comprising a live or killed recombinant carrier of the invention. Because a cause of mastitis in cows is *Streptococcus uberis,* in a preferred embodiment of the invention, said live or killed recombinant carrier is a *Streptococcus* species. In one embodiment of the invention, said host cell is a *streptococcus* species. The proteinaceous molecule is then preferably presented in the context of other streptococcal proteins, which enhances eliciting an immune response. Furthermore, the immunogenicity can be enhanced by over-expression of the recombinant proteins on the surface of the host cell, preferable a streptococcal cell. In addition, the use of different strains of streptococcal host cells enhances the immunogenicity of the immunogenic proteins or parts thereof. Therefore, the present invention also provides an immunogenic composition according to the invention, wherein said host cell is a *streptococcus* species. There are several streptococcus species that are also suitable as a host cell, for example, *S*. *suis* or *S*. *agalactiae* or *S*. *dysgalactiae.*

Because of differences between various *streptococcus* species, and because some proteins, naturally occurring on *Streptococcus uberis,* are capable of assisting in eliciting an immune response against *Streptococcus uberis,* in a preferred embodiment, attenuated *Streptococcus uberis* is used as a live recombinant carrier in an immunogenic composition of the invention. Preferably, said *S.uberis* organism expresses proteins of another strain of *S.uberis,* thereby disclosing a differentiating vaccine, because the serum of an animal vaccinated with said vaccine is discernible from the serum of an animal infected with a field infection, by detecting antibodies against proteins of both strains. In another embodiment, said *S.uberis* organism is replaced as a host cell or as a live or killed carrier by an *S.suis,* or a *Staphylococcus* species or an *E.coli,* either live or killed.

Administering an immunogenic protein of the invention or a part thereof, or administering a nucleic acid encoding said protein of the invention elicits an immune response. Said nucleic acid, when administered to a cow, is expressed in cells of said cow and recognized by the immune system of said cow. The nucleic acid is thereby acting as an immunogenic composition, like for example a DNA vaccine against mastitis. Therefore, the present invention provides an immunogenic composition capable of eliciting an immune response against *Streptococcus uberis,* said composition comprising a nucleic acid of the invention.

Because the immunogenic composition of the invention elicits an immune response in an animal, preferably a cow, the protein of the invention reduces illnesses related to mastitis and improves the health of said cows, thereby rendering cows much more resistant to other (secondary) infections. Therefore, the present invention provides an immunogenic composition according the invention for use as a medicament. Preferably, the immunogenic composition of the invention is used to manufacture a medicament against *Streptococcus uberis* mastitis that reduces specific illness as a result of mastitis caused by *Streptococcus uberis.* Therefore, the present invention provides the use of an immunogenic composition according to the invention for the preparation of a medicament against *Streptococcus uberis* mastitis.

An immunogenic composition of the invention is also used to produce or formulate a vaccine against mastitis. A vaccine generally prevents animals or humans from contracting a disease. Preferably, the immunogenic composition of the present invention is capable of preventing mastitis. Therefore, the present invention discloses in a preferred embodiment the use of an immunogenic composition according to the invention for the preparation of a vaccine.

In another embodiment, the immunogenic composition of the invention is preferably used for decreasing and/or controlling the numbers of *S. uberis* organisms in the milk and/or in the udder of the cow. The milking process on a dairy farm comprises a potential danger of transferring *S.uberis*organisms from a diseased cow to another cow. The decrease in numbers of *S.uberis* organisms is therefore most suitable to suppress the spread of the infection from udder teat to udder teat and/or from animal to animal.

For administration of an immunogenic composition of the invention to a subject, admixing the proteins or immunogenic parts thereof or host cells with a suitable carrier facilitates the acceptance by a subject of the immunogenic composition and increases the immunogenic effect of the composition. A suitable carrier of the invention comprises for example a suitable adjuvant to increase the immunising effect of said immunogenic composition. Many suitable adjuvants, both based on oils and water-based, are known to a person skilled in the art, for example Diluvacforte® adjuvant or Specol® adjuvant. In one embodiment, said suitable carrier comprises for example a solution like for example saline for diluting bacteria or proteins or immunogenic parts thereof. Therefore, the present invention discloses a pharmaceutical composition comprising an immunogenic composition of the invention and a suitable carrier.

In an animal, preferably a cow, which is immunised with an immunogenic composition of the invention, antibodies are produced that are directed against *S.uberis.* The presence and the level of said antibodies are indicative for the immunity after immunisation with an immunogenic composition or a vaccine of the invention. Said antibodies are preferably not directed against epitopes that were present as wild type *S. uberis* strains in the field. For discerning a vaccinated animal from an animal that had a field type infection, the immunity of said vaccinated animal is in one embodiment preferably measured by measuring antibodies directed against said immunogenic composition of the invention. The antiserum of said vaccinated cow is also tested for the presence of antibodies against *S.uberis* antigens, which are not present in the immunogenic composition. Detecting antibodies against an *S.uberis* antigen not present in the immunogenic composition or vaccine of the invention is an indication of a wild type infection. Therefore, the present invention discloses a method for measuring the immunity of an animal against *S.uberis,* said method comprising determining in at least one sample from said animal the presence of antibodies directed against a protein selected from Table 5 and/or Table 6, or an immunogenic part thereof. In a preferred embodiment, said protein selected from Table 5 and/or 6 is selected from the group consisting of P15, P16, P17, P19, P20, P22, P27, P54, P28, P63, P64, P68, P75, P81, P93, P100, and P105. Even more preferred said protein selected from Table 5 and/or 6 is selected from the group consisting of P15, P16, P20, P27, P54, P28, P63, P68, P93, and P105. Most preferred, said protein selected from Table 5 and/or 6 is selected from the group consisting of P15, P16, P54, P28, P63, and P105.

For the detection of antibodies, which bind to the immunogenic composition of the invention, a diagnostic kit is suitable which comprises at least one of the proteins of Table 5 and/or Table 6 or an immunogenic part thereof. Binding of an antibody to said protein or immunogenic part thereof is detected by means of for example immunefluorescent antibody detection or enzyme-linked antibody detection or any other means of detection of antibody bound to said protein or immunogenic part thereof.

In a preferred embodiment, said at least one of the proteins of Table 5 and/or 6 is selected from the group consisting of P15, P16, P17, P19, P20, P22, P27, P54, P28, P63, P64, P68, P75, P81, P93, P100, and P105. Even more preferred said at least one of the proteins of Table 5 and/or 6 is selected from the group consisting of P15, P16, P20, P27, P54, P28, P63, P68, P93, and P105. Most preferred, said at least one of the proteins of Table 5 and/or 6 is selected from the group consisting of P15, P16, P54, P28, P63, and P105.

Therefore, the present invention discloses a diagnostic kit comprising at least one protein selected from Table 5 and/or table 6, or immunogenic part thereof and a means of detecting antibody binding to said protein or immunogenic part thereof. Such a diagnostic kit is for example an ELISA test, or any other test suitable for screening sera. Preferably said test kit is suitable for screening large numbers of sera. In another embodiment, a nucleic acid of the invention is used for the detection of animals infected with wild type *S.uberis* strains in a population of animals vaccinated with an immunogenic composition or a vaccine of the invention. This detection is for example achieved by using a PCR.

The present patent application discloses that successful immunogenic proteins of the invention are proteinaceous molecules and/or proteins accessible to antibodies at the bacterial surface and common to a number of *S. uberis* strains. As an example, surface proteins were identified from the genome sequences of strains 41-241 and 0140J by selecting for genes containing one or more sequences commonly found in surface proteins of gram-positive bacteria, like for example a LPXTG sortase motif required for anchoring of the protein to the cell wall, or a lipid attachment motif required for lipoproteins, or a signal sequence or a transmembrane region predicting a surface localization of the encoded protein.

The present application discloses the presence of selected proteins in strains of *S. uberis* as examined by probing chromosomal DNA of a number of *S. uberis* strains with PCR products obtained from the genes as selected above.

The invention provides the following items:
1. A method for identifying a *Streptococcus* protein which is capable of eliciting an immune response against at least two strains and/or serotypes of *Streptococcus,* the method comprising:
   a) identifying at least part of a secreted protein, a surface-associated protein and/or a protein which has at least 50% sequence identity to a bacterial virulence factor;
   b) selecting at least one protein identified in step a) which is conserved over at least two *Streptococcus* strains and/or serotypes; and
   c) determining whether at least one protein selected in step b) or an immunogenic part, derivative and/or analogue thereof is capable of specifically binding an antibody and/or immune cell of an animal infected by a first *Streptococcus* strain and/or serotype, and an antibody and/or immune cell of an animal infected by a second *Streptococcus* strain and/or serotype.
2. A method according to item 1, wherein said secreted protein and/or surface-associated protein is identified by identifying in at least part of the genomic sequence of *Streptococcus* a gene comprising a motif of a secreted and/or surface-associated protein.
3. A method according to item 1 or 2, wherein said protein which has at least 50% sequence identity to a bacterial virulence factor is identified by identifying in at least part of the genomic sequence of *Streptococcus* a gene which has at least 50% sequence identity to a bacterial virulence factor gene.
4. A method according to item 1 or 2 or 3, wherein said protein which has at least 50% sequence identity to a bacterial virulence factor is identified by identifying in at least part of the genomic sequence of *Streptococcus* a gene which hybridizes to the full length nucleotide sequence of any of the nucleic acid sequences listed in Figure 4 at 65°C in a buffer having 0.5 M sodium phosphate, 1 mM EDTA, and 7% sodium dodecyl sulphate at a pH of 7.2,
   wherein the nucleic acid molecule remains hybridized after washing twice with a buffer containing 40 mM sodium phosphate (pH 7.2), 1 mM EDTA and 5% sodium dodecyl sulphate for 30 minutes at 65°C and;
   washing twice with a buffer containing 40 mM sodium phosphate (pH 7.2), 1 mM EDTA and 1% sodium dodecyl sulphate for 30 minutes at 65°C.
5. A method according to item 2, 3 or 4, further comprising selecting a gene which is conserved over at least two *Streptococcus* strains and/or serotypes.
6. A method according to item 5, further comprising obtaining a protein encoded by said gene, or an immunogenic part, derivative and/or analogue of said protein.
7. A method according to any one of items 2-6, wherein said gene is expressed in a prokaryotic expression system.
8. A method according to any one of items 1-7, comprising:
   - obtaining isolated and/or recombinant proteins of *Streptococcus;*
   - incubating said proteins with an antibody and/or immune cell of an animal infected by a first *Streptococcus* strain and/or serotype and an antibody and/or immune cell of an animal infected by a second *Streptococcus* strain and/or serotype, and
   - determining whether a protein is capable of binding an antibody and/or immune cell of an animal infected by a first *Streptococcus* strain and/or serotype and an antibody and/or immune cell of an animal infected by a second *Streptococcus* strain and/or serotype.
9. A method according to item 8, further comprising expressing said protein using a nucleic acid sequence encoding said protein.
10. A method according to any one of items 1-9, wherein said antibody and/or immune cell is derived from a convalescent serum.
11. A method according to any one of items 1-10, wherein said *Streptococcus* protein is capable of eliciting opsonophagocytosis inducing antibodies.
12. A method according to any one of items 1-11, wherein at least two *Streptococcus* proteins capable of eliciting an immune response against at least two strains and/or serotypes of *Streptococcus* are identified.
13. A method for producing at least one protein identified by a method according to any one of items 1-12.
14. A method according to any one of items 1-13, wherein said *Streptococcus* is *Streptococcus uberis.*
15. *A Streptococcus* protein which is capable of eliciting an immune response against at least two strains and/or serotypes of *Streptococcus* obtainable by a method according to any one of items 1-14.
16. Use of a protein obtainable by a method according to any one of items 1-14, or an immunogenic part, derivative and/or analogue thereof, for the preparation of an immunogenic composition capable of eliciting an immune response against at least two strains and/or serotypes of *Streptococcus.*
17. Use according to item 16, wherein said protein is selected from Table 5 and/or Table 6.
18. An immunogenic composition capable of eliciting an immune response against at least two strains and/or serotypes of *Streptococcus* comprising at least one isolated and/or recombinant protein obtainable by a method according to any one of items 1-14, or an immunogenic part, derivative and/or analogue thereof.
19. An immunogenic composition according to item 18 comprising at least two isolated and/or recombinant proteins, and/or an immunogenic part, derivative and/or analogue thereof, obtainable by a method according to any one of items 1-12.
20. An immunogenic composition capable of eliciting an immune response against at least two strains and/or serotypes of *Streptococcus* comprising at least one nucleic acid molecule encoding at least one protein obtainable by a method according to any one of items 1-12, or an immunogenic part, derivative and/or analogue of said protein.
21. An immunogenic composition according to any one of items 18-20, wherein said *Streptococcus* is *Streptococcus uberis.*
22. An immunogenic composition according to any one of items 18-20, wherein at least one, preferably at least two of said proteins is selected from Table 5 and/or Table 6.
23. A method for producing an immunogenic composition capable of eliciting an immune response against at least two strains and/or serotypes of *Streptococcus,* said method comprising providing a cell with at least one recombinant vector, said at least one vector comprising a nucleic acid sequence encoding at least one protein obtainable by a method according to any one of items 1-14 and/or at least one protein selected from Table 5 and/or Table 6, and/or an immunogenic part, derivative and/or analogue thereof.
24. A recombinant nucleic acid molecule comprising a nucleic acid sequence encoding at least two proteins obtainable by a method according to any one of items 1-14 and/or at least two proteins selected from Table 5 and/or Table 6, and/or an immunogenic part of at least one of said proteins, under the control of a functionally linked promoter.
25. A recombinant carrier comprising a nucleic acid encoding at least two proteins obtainable by a method according to any one of items 1-14 and/or selected from Table 5 and/or Table 6, and/or an immunogenic part of at least one of said proteins, or a recombinant nucleic acid molecule according to item 24.
26. A recombinant carrier according to item 25 which is a live carrier.
27. A recombinant carrier according to item 25 or 26, which is a *Streptococcus* species.
28. A recombinant carrier according to item 27, wherein said streptococcus species is *a Streptococcus uberis.*
29. A recombinant carrier according to item 27 or 28, wherein said *Streptococcus* is lacking at least part of a capsular gene expression product.
30. A recombinant carrier according to any one of items 27-29, wherein said *Streptococcus* is a non-capsular streptococcus.
31. A recombinant carrier according to any one of items 25-30, comprising a nucleic acid encoding at least one protein and/or immunogenic part thereof derived from a first *Streptococcus* strain and/or serotype, and a nucleic acid encoding at least one protein and/or immunogenic part thereof derived from a second *Streptococcus* strain and/or serotype.
32. An isolated host cell comprising a nucleic acid sequence encoding at least two proteins obtainable by a method according to any one of items 1-14 and/or selected from Table 5 and/or Table 6, and/or an immunogenic part of at least one of said proteins, or a recombinant nucleic acid molecule according to item 24 or a recombinant carrier according to any one of items 25-31.
33. An immunogenic composition capable of eliciting an immune response against *Streptococcus,* said composition comprising a recombinant carrier according to any one of items 25-31.
34. An immunogenic composition according to any of items 18-22 and/or 33 for use as a medicament.
35. Use of an immunogenic composition according to any one of items 18-22 and/or 33 for the preparation of a medicament against *Streptococcus uberis* mastitis.
36. Use of an immunogenic composition according to any one of items 18-22 and/or 33 for the preparation of a vaccine.
37. A method for decreasing and/or controlling the number of *Streptococcus* organisms in an individual and/or non-human animal comprising providing said individual and/or non-human animal with an immunogenic composition according to any one of items 18-22 and/or 33.
38. A pharmaceutical composition comprising an immunogenic composition according to any one of items 18-22 and/or 33 and a suitable carrier, diluent and/or excipient.
39. A method for measuring the immunity of an individual and/or non-human animal against *Streptococcus,* said method comprising determining in at least one sample from said individual and/or animal the presence of antibodies directed against a protein obtainable by a method according to any one of items 1-14 and/or selected from Table 5 and/or Table 6, or an immunogenic part thereof.
40. A diagnostic kit comprising at least one protein obtainable by a method according to any one of items 1-14 and/or selected from Table 5 and/or Table 6, or an immunogenic part thereof, and a means of detecting antibody binding to said protein or immunogenic part thereof.
41. An immunogenic composition capable of eliciting an immune response against *Streptococcus uberis* comprising at least two recombinant and/or isolated surface proteins derived from at least one *Streptococcus uberis* strain, and/or an immunogenic part or analogue or derivative of either or both of said proteins.
42. An immunogenic composition according to item 41, wherein said at least two proteins are selected from Table 5 and/or Table 6.
43. A method for producing an immunogenic composition according to item 41 or 42, said method comprising providing a cell with a recombinant vector, said vector comprising a nucleic acid encoding at least two proteins as listed in Table 5 and/or Table 6, and/or an immunogenic part or analogue or derivative of either or both of said proteins.
44. A method according to item 43, comprising providing at least two cells with a recombinant vector according to item 43 and/or a recombinant vector comprising a nucleic acid encoding a protein as listed in Table 5 and/or Table 6, and/or an immunogenic part or analogue or derivative of either or both of said proteins.
45. A recombinant molecule comprising a nucleic acid sequence encoding a protein as listed in Table 5 and/or Table 6, and/or an immunogenic part or analogue or derivative thereof under the control of a functionally linked promoter.
46. A recombinant molecule comprising a nucleic acid sequence encoding at least two proteins as listed in Table 5 and/or table 6, and/or an immunogenic part or analogue or derivative of either or both of said proteins under the control of a functionally linked promoter.
47. A live recombinant carrier comprising a nucleic acid sequence according to item 45 or 46.
48. An isolated host cell comprising a nucleic acid sequence according to item 45 or 46.
49. An isolated and/or recombinant proteinaceous molecule that has at least 80% sequence identity to a protein encoded by a nucleic acid according to item 45 or 46.
50. An isolated and/or recombinant proteinaceous molecule that has at least 95% sequence identity to a proteinaceous molecule encoded by a nucleic acid according to item 45 or 46.
51. An isolated and/or recombinant proteinaceous molecule comprising a stretch of at least 25 consecutive amino acids of a proteinaceous molecule encoded by a nucleic acid according to item 45 or 46.
52. A nucleic acid encoding a proteinaceous molecule according to any of items 49 to 51.
53. An immunogenic composition according to item 40 or 41, said composition comprising an isolated and/or recombinant proteinaceous molecule according to any of items 49 to 51.
54. An immunogenic composition capable of eliciting an immune response against *Streptococcus uberis,* said composition comprising a live or killed recombinant carrier according to item 47.
55. An immunogenic composition according to item 54, wherein said live or killed recombinant carrier is a *Streptococcus species.*
56. An immunogenic composition according to item 54 or 55, wherein said live or killed recombinant carrier is a *Streptococcus uberis.*
57. An immunogenic composition capable of eliciting an immune response against *Streptococcus uberis,* said composition comprising a nucleic acid according to item 45 or 55.
58. An immunogenic composition according to item 41 or 42 and/or items 53 to 57 as a medicament.
59. Use of an immunogenic composition according to item 41 or 42 and/or items 53 to 57 for the preparation of a medicament against *Streptococcus uberis* mastitis.
60. Use of an immunogenic composition according to item 41 or 42 and/or items 53 to 57 for the preparation of a vaccine.
61. Use of an immunogenic composition according to item 41 or 42 and/or items 53 to 57 for decreasing and/or controlling the number of *S. uberis* organisms in the milk and/or an udder of a cow.
62. A pharmaceutical composition comprising an immunogenic composition according to item 41 or 42 and/or 53 to 57 and a suitable carrier.
63. A method for measuring the immunity of an animal against *S.uberis,* said method comprising determining in at least one sample from said animal the presence of antibodies directed against a protein selected from Table 5 and/or Table 6, or a protein according to any one of items 49 to 51 or an immunogenic part thereof.
64. A diagnostic kit comprising at least one protein selected from Table 5 and/or Table 6, or a protein according to any one of items 49 to 51 or an immunogenic part thereof and a means of detecting antibody binding to said protein or immunogenic part thereof.
65. A method for detecting *S. uberis* immunogenic strains, said method comprising isolating nucleic acid from a *S. uberis* organism from a case of mastitis, subjecting said nucleic acid to a PCR comprising a nucleic acid according to item 45, 46 or 52, or a primer thereof.

The invention is further explained in the following examples. These examples do not limit the scope of the invention, but merely serve to clarify the invention. Many alternative embodiments can be carried out, which are within the scope of the present invention.

### Legends to the Figures

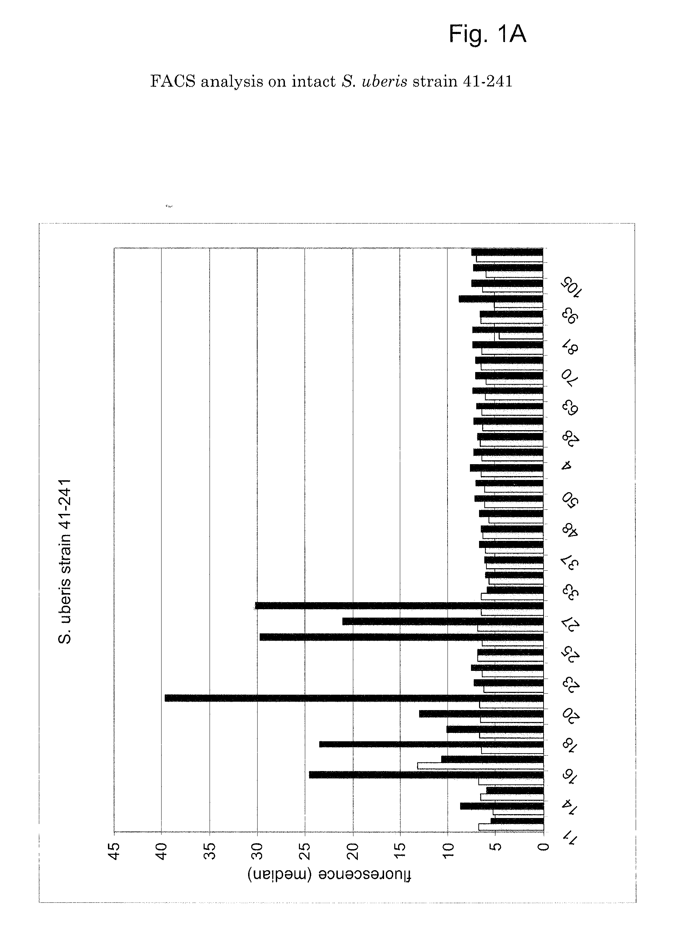

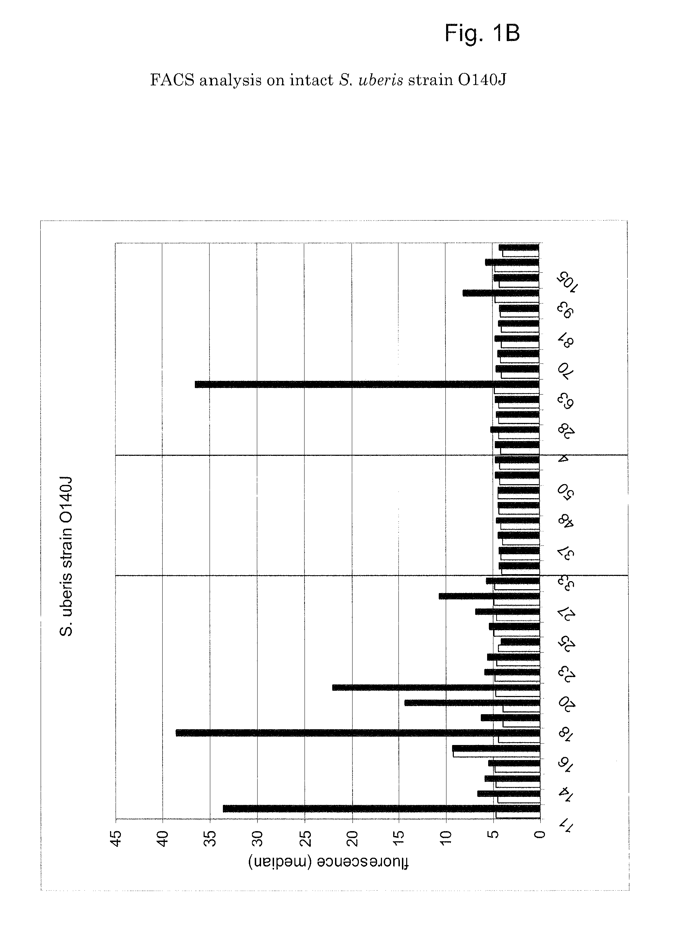

**Figure 1****. FACS analysis on intact *S.uberis* strains.** *S.uberis* strains 41-241 (A) and 0140J (B) were incubated with mice immune-sera (dark bars) or with the corresponding pre-immune sera (light bars). Bound antibodies were detected using FITC-conjugated secondary antibodies. Data are expressed as the median of fluorescence associated with bacterial cells. A fluorescence of ≥ 10 (2 x background) was considered as being positive. Numbers of the sera used refer to the gene/protein numbers as indicated in Tables 1 to 4.

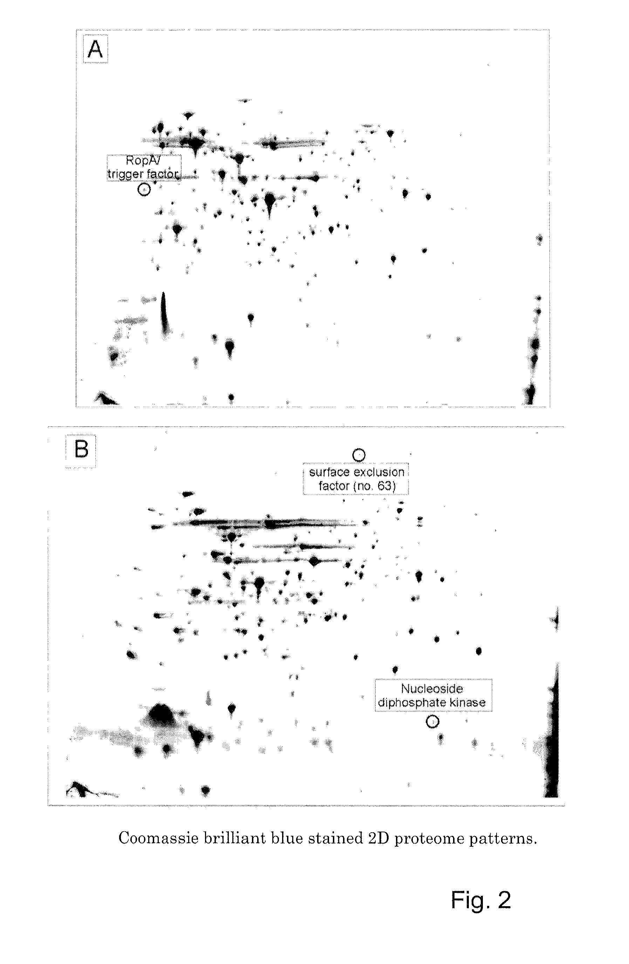

**Figure 2****. Coomassie brilliant blue stained 2D proteome patterns.** Lysates of exponentially growing *S.uberis* strains 41-241 (A) and 0140J (B) were probed with bovine sera obtained from cows after experimental infection with strain 0140J. Circled proteins were identified as being immunogenic proteins. The properties of the identified proteins as analysed by in-gel tryptic digestion, MALDI-TOF mass spectrometry are shown in Table 6.

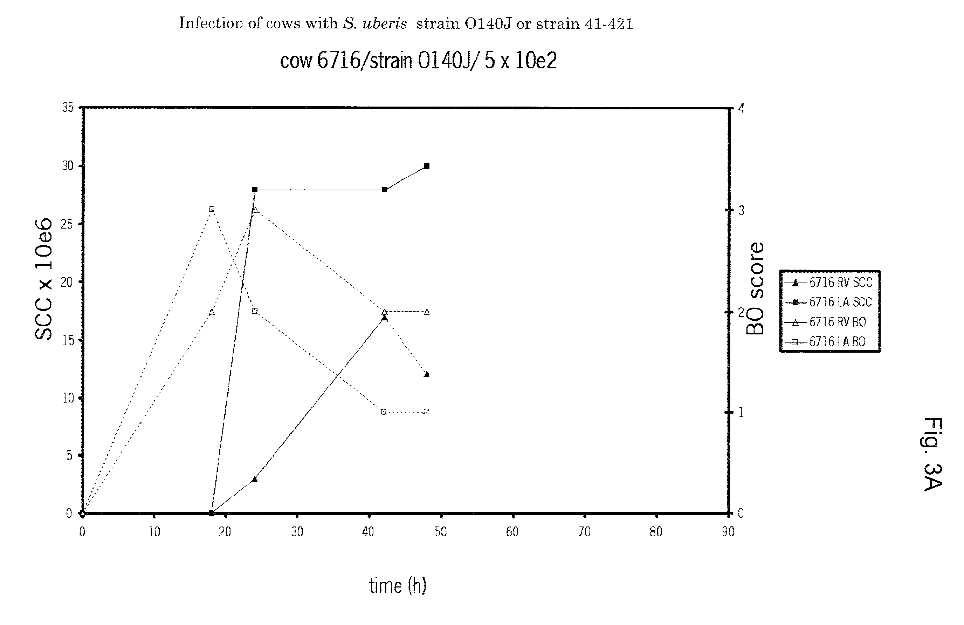

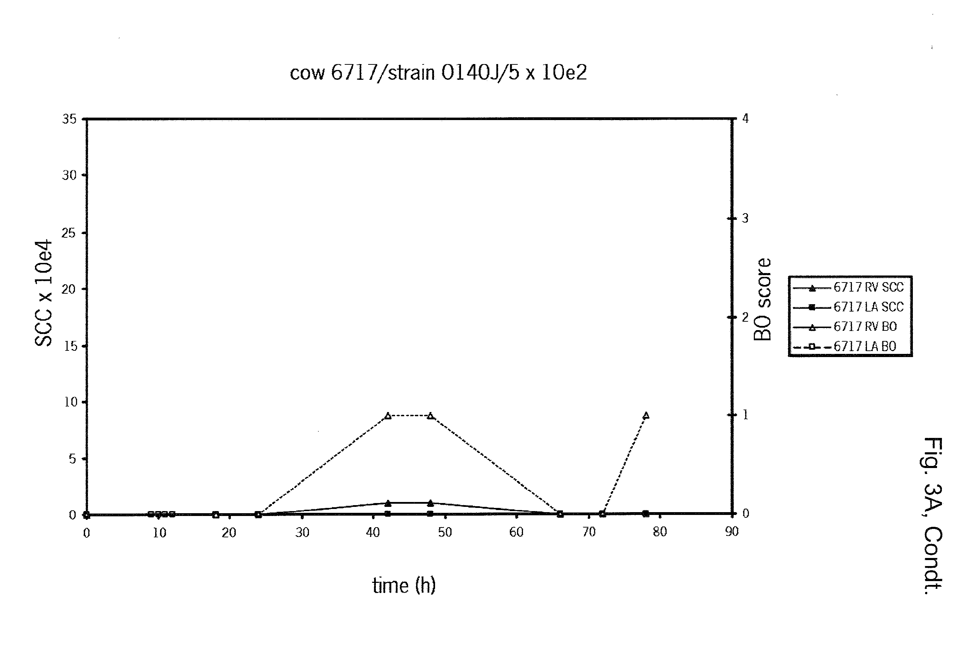

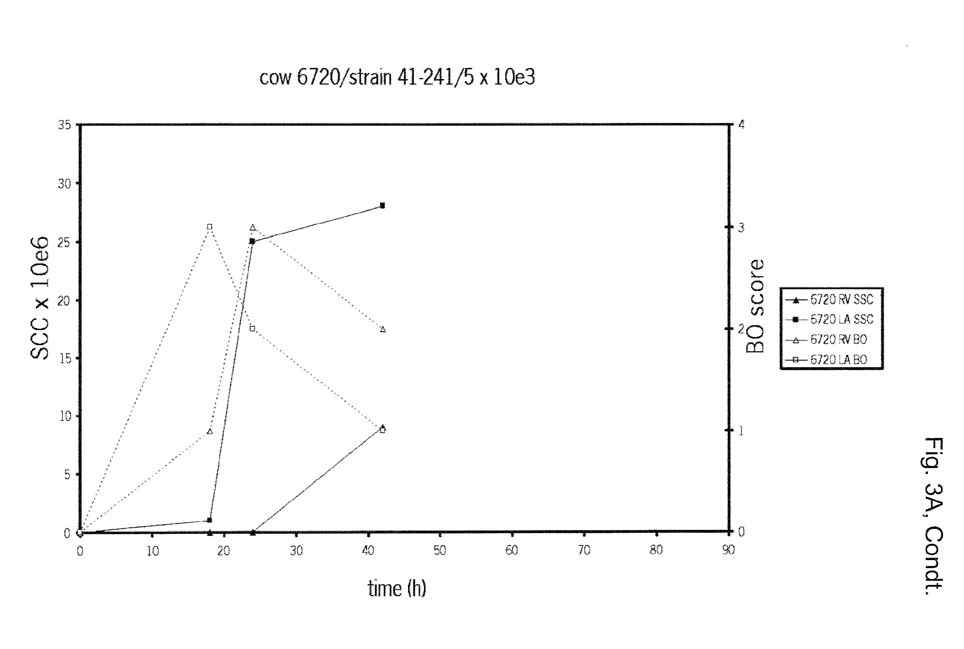

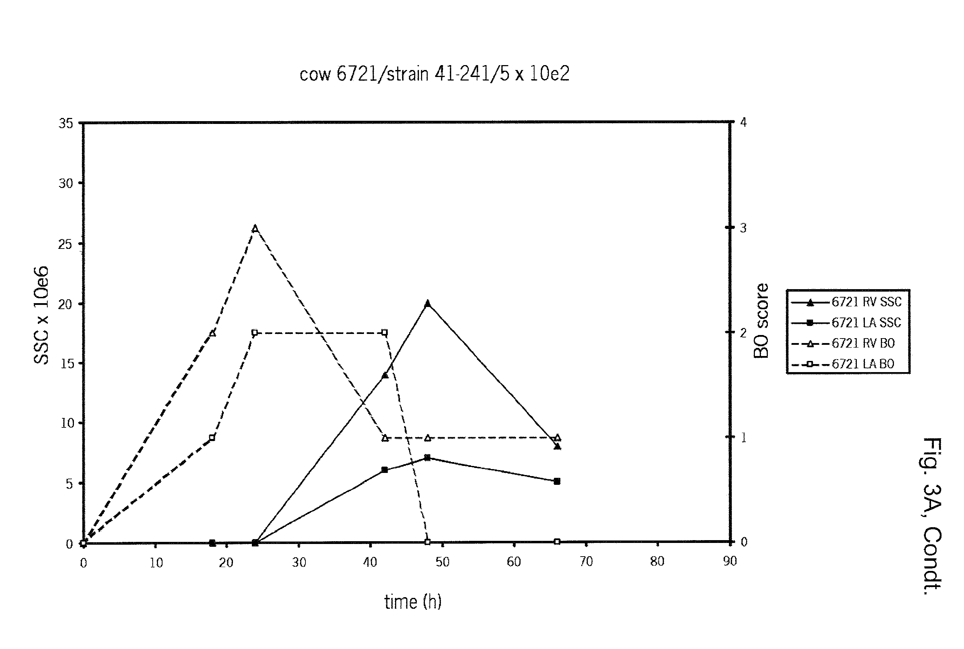

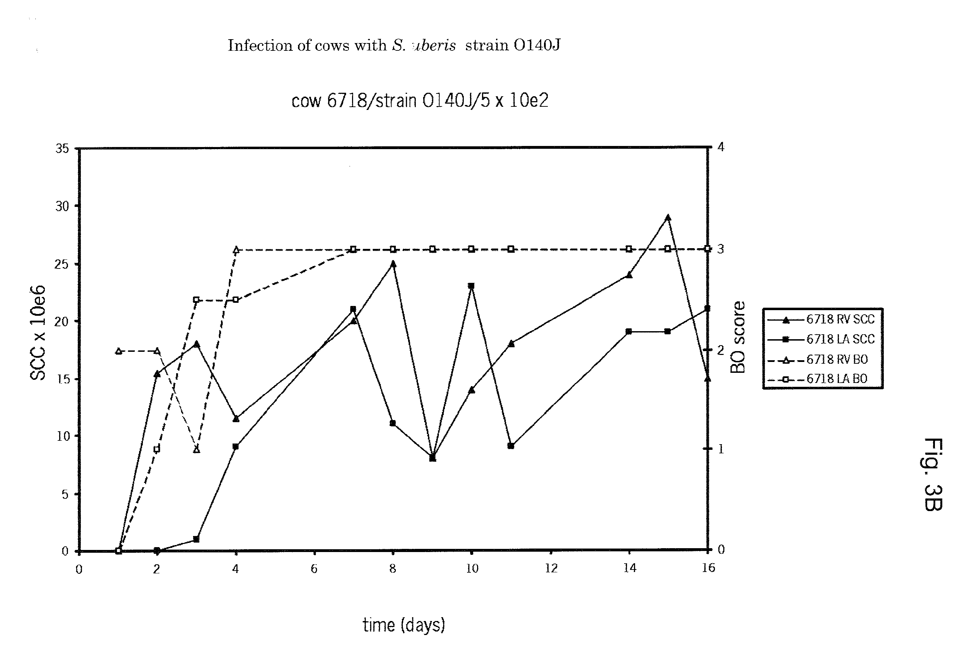

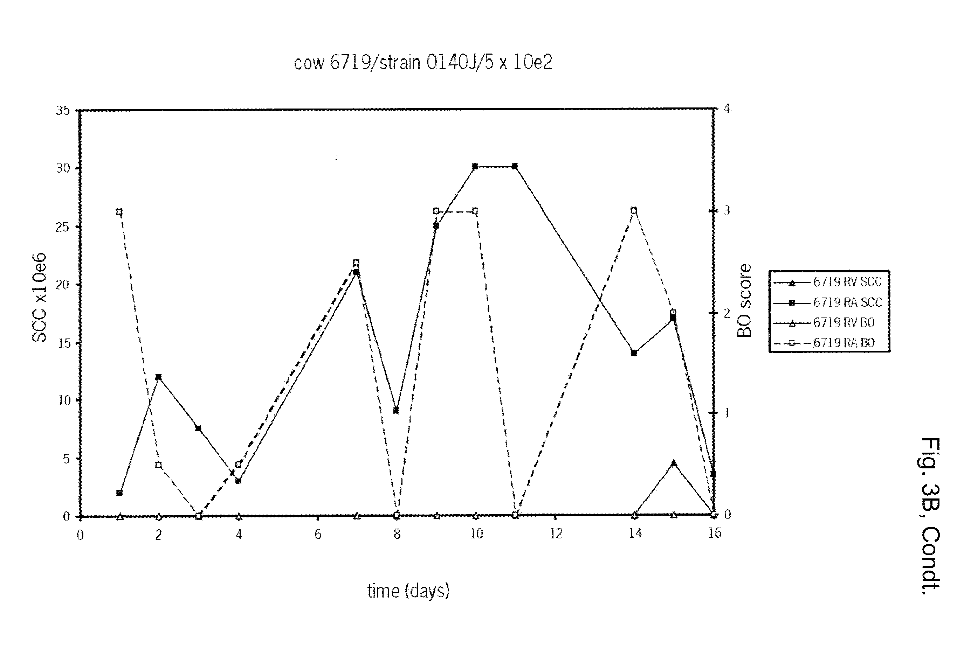

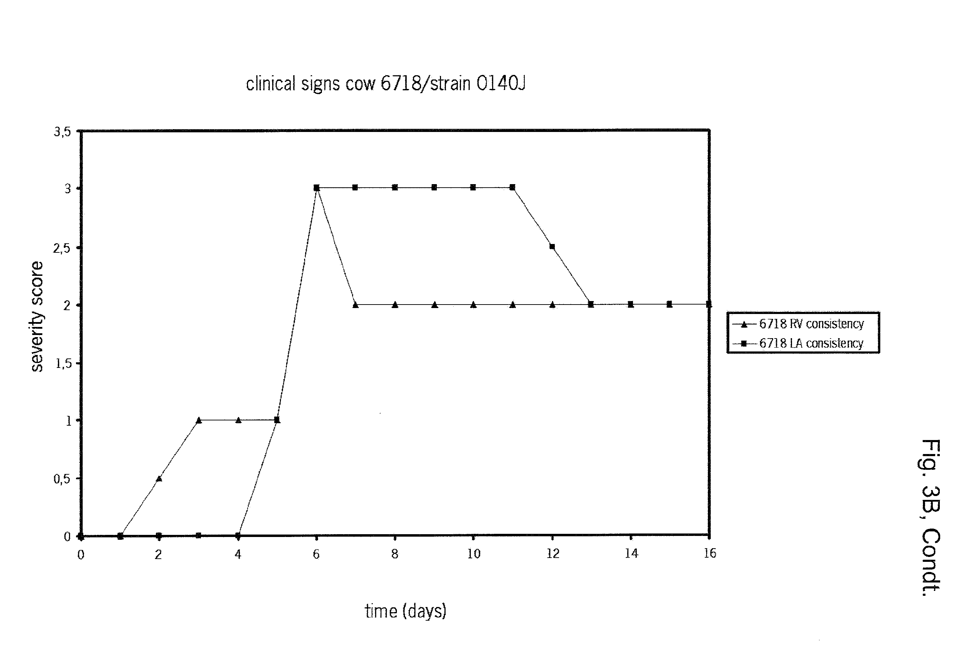

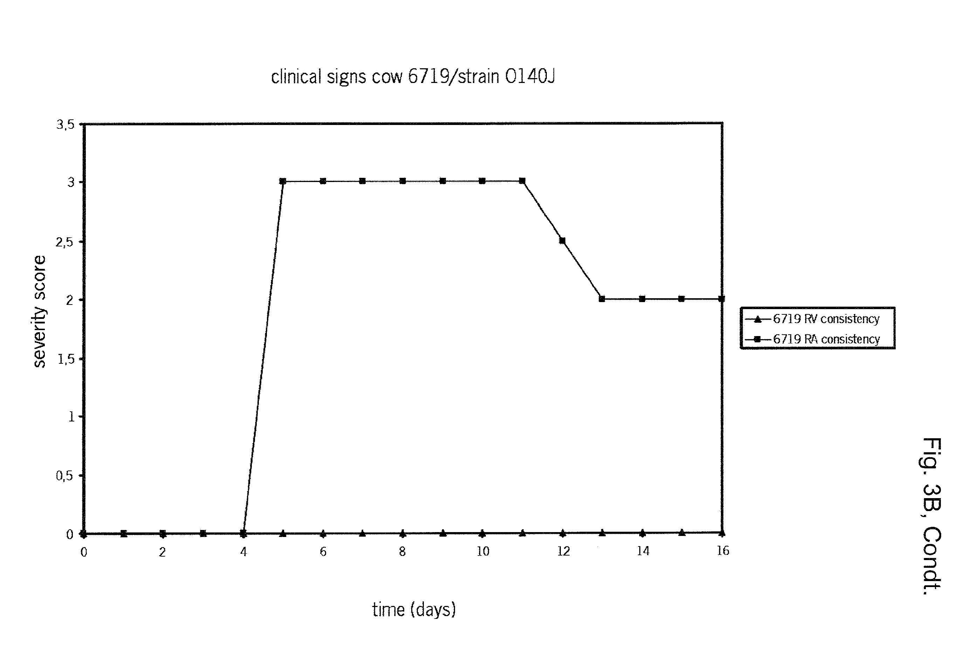

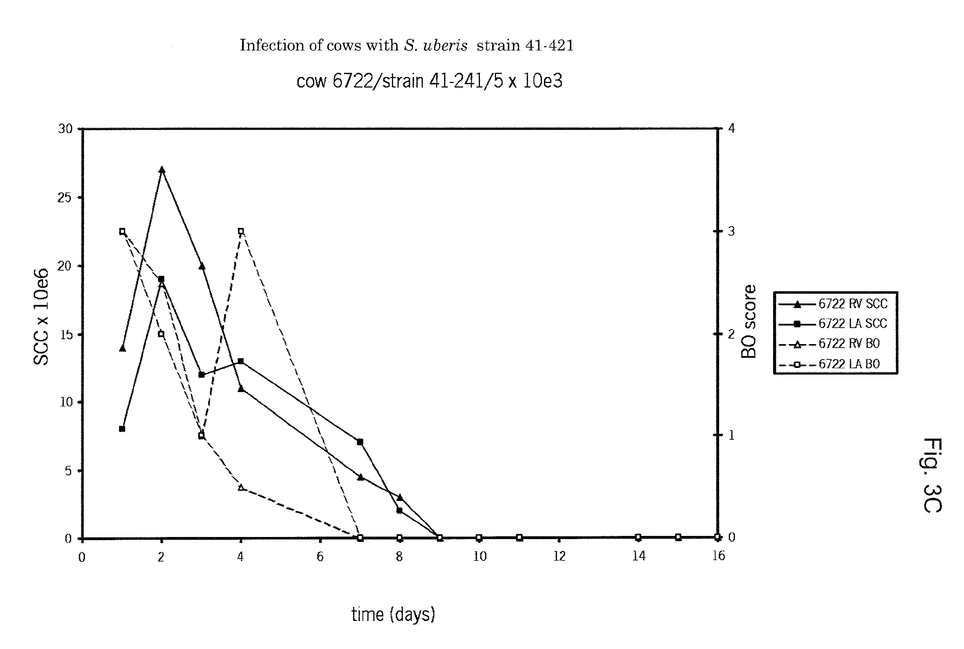

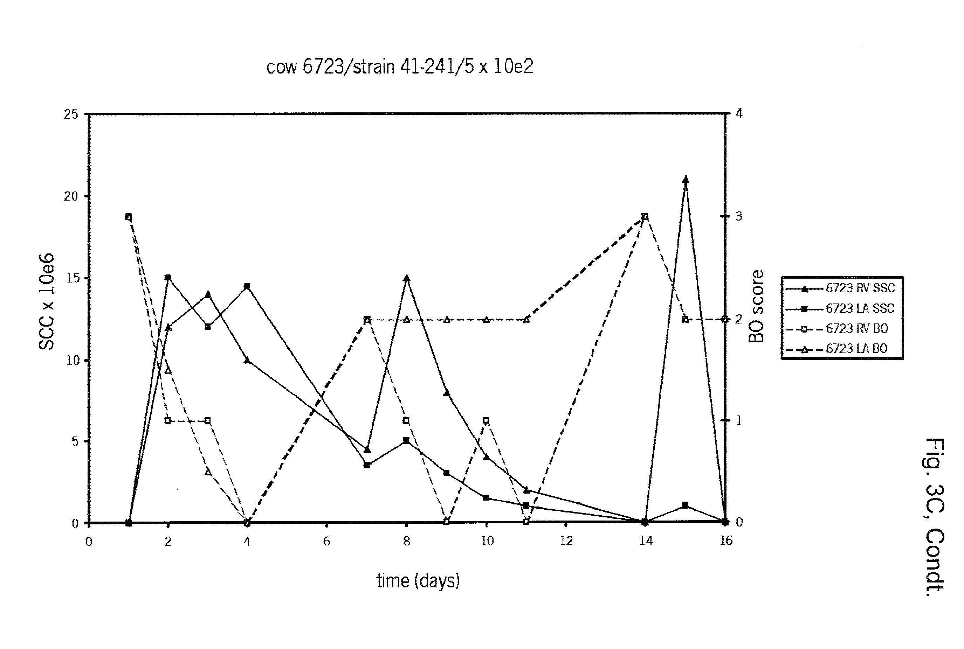

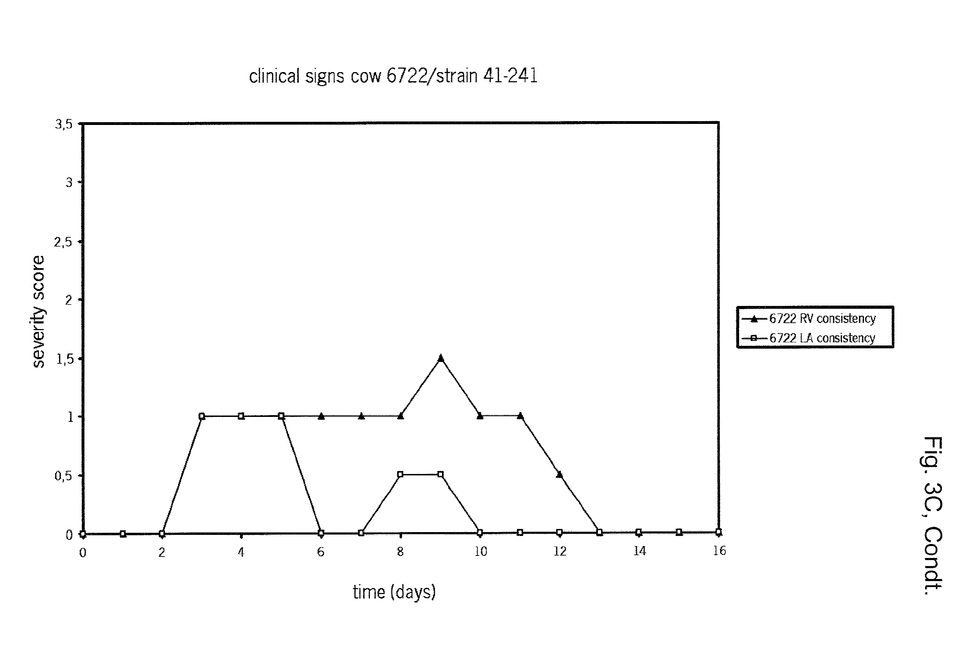

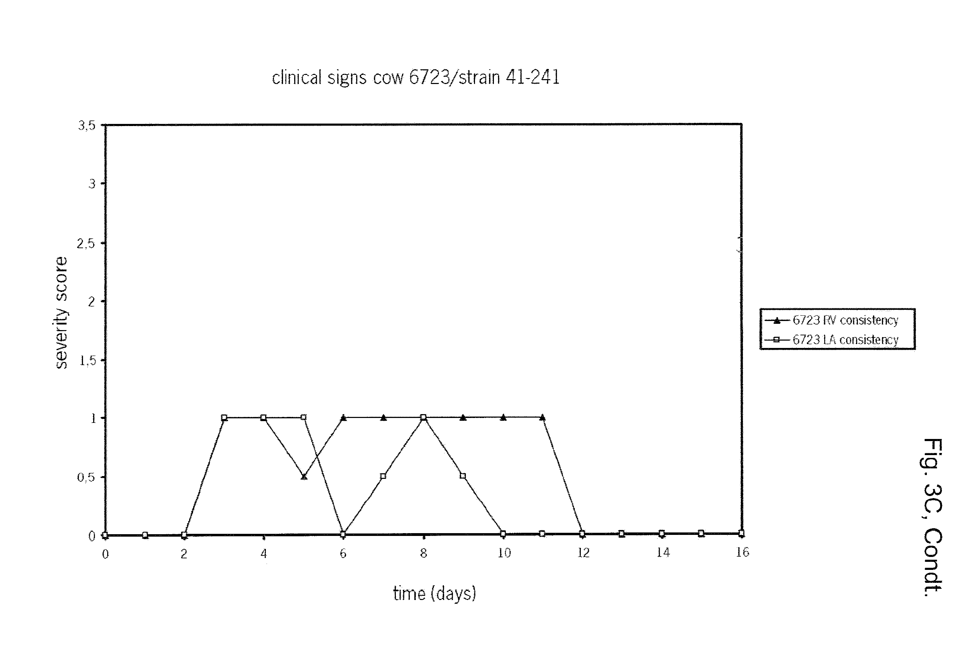

**Figure 3****. Infection of cows with *S.uberis* strain 0140J or strain 41-421.**
   **3 A**.Cows 6716 and 6717 are infected via the milk duct with *S.uberis* strain 0140 J. Cows 6720 and 6721 are infected via the milk duct with *S. uberis* 41-421. Notice that cow 6720 is infected with 5000 cfu *S. uberis* and 6721 is infected with 500 cfu *S. uberis.* SSC means somatic cell counts in the milk. BO means bacterial investigation and is presented as the number of organisms as colony forming units (cfu) isolated form the milk.
   RV means right anterior quarter, LA means left posterior quarter.
   **3 B.** Clinical signs and bacterial and cytological results of cows 6718 and 6719 after infection with *S. uberis* strain 0140J.
   **3 C.** Clinical signs and bacterial and cytological results of cows 6722 and 6723 after infection with *S.uberis* strain 41-241.
**Figure 4****.** Nucleic acid sequences and amino acid sequences of *S.uberis* proteins of table 5.

### Examples

### Example 1

### Selection of common surface antigens

**DNA sequence analysis.** The DNA sequence of the *S. uberis* strain 41-241 has been determined with a 2 x coverage. Sequencing data were assembled to obtain 572 contiguous sequences containing 1815 ORFs. At the Sanger center the *S.uberis* strain 0140J (Hill, 1988) has been sequenced. The sequence data available at the Sanger site in April 2002 were assembled as well, to obtain 61 contigs containing 1938 ORFs.

**Selection of common surface antigens.** Successful vaccine antigens are proteins accessible to antibodies at the bacterial surface and common to a number of *S. uberis* strains. Surface proteins were identified from the genome sequences of strains 41-241 and 0140J by selecting for genes containing one or more sequences that form a signature motif (see M&M) commonly found in surface proteins of gram-positive bacteria. Among all ORFs analysed, 17 ORFs contained a LPXTG sortase motif (Table 1) required for anchoring of the protein to the cell wall. Four (P12, P23, P24 and P25) of these 17 proteins were exclusively found in strain 41-241.

Thirty-one ORFs contained a lipid attachment motif required for lipoproteins (Table 2).

All these proteins were found in strain O140J as well as in strain 41-241. Moreover, 87 ORFs were selected that contained a signal sequence or a transmembrane region predicting a surface localization of the encoded protein (Table 3).

These proteins were found in strain 0140J as well as in strain 41-241.

### Example 2

### Distribution of selected genes among various clinical and subclinical isolates of S.uberis

To examine the presence of the selected genes among various *S.uberis*strains, spot hybridization experiments were performed in which chromosomal DNA of a considerable number of clinical *S. uberis* strains was probed with PCR products obtained from 99 of the selected genes. The data (Table 4) show that most of the selected genes hybridise with most *S.uberis* strains, suggesting that most of the selected genes are commonly present among the various *S.uberis* strains. In contrast, 4 out of the 99 genes tested hybridised only with a limited number of strains. All of these genes are present in strain 41-241 and encode proteins having a LPXTG sortase motif required for anchoring of the protein to the cell wall.

### Example 3

### Immunogenicity of selected surface proteins.

To evaluate a role of the proteins as vaccine candidates the proteins encoded by the 115 of the selected genes were cloned and expressed in *E*. *coli* with polyhistidine tags. The products of 106 of these genes were successfully cloned and expressed in *E*. *coli.* Subsequently, sera obtained from *S.uberis* infected cows and from rabbits immunized with formalin-killed or sonicated *S.uberis* cells were tested for the presence of specific antibodies directed against the expressed proteins by Western blot analysis. The results (Table 5) show that 19 of the expressed proteins were recognized by antibodies present in sera of *S. uberis* infected animals, indicating that these proteins are expressed *in vivo* and are immunogenic in cows. Moreover, 30 of the expressed proteins were recognized by antibodies present in sera from rabbits immunized with formalin-killed or sonicated *S.uberis* cells. Twelve of the expressed proteins were recognized both by sera obtained from *S.uberis* infected cows as well as by sera from rabbits immunized with formalin-killed or sonicated *S.uberis* cells. These data indicate that most of the proteins are antigenic.

Table 5 also shows that some proteins were recognised by antisera induced after experimental infection with both strains 41-241 and 0140J. However, other proteins reacted positive exclusively with either sera obtained after infection with strain O140J or with sera obtained after infection with strain 41-241. This probably indicates differences between the two strains either in protein expression in vivo or in accessibility of the proteins to the immune system.

### Example 4

### Purification of the selected proteins.

To further evaluate a role of the proteins as vaccine candidates, all 36 proteins recognized either by sera from infected cows and/or by sera from immunized rabbits were purified. In addition, 4 proteins that contained a sortase motif but that did not react with both sera were purified. Thirty-one of the 40 selected recombinant proteins were successfully over-expressed in soluble form and could be purified under native conditions, whereas 5 proteins were expressed as insoluble inclusion bodies. These proteins were purified using denaturing conditions and the proteins were refolded after purification. For 4 proteins we were unable to purify amounts sufficient for immunization. All 36 purified proteins were subsequently used to immunize mice. As shown on Western blots none of the proteins was reactive with serum obtained from mice before immunization. In contrast however, the proteins strongly reacted with immune serum obtained from the mice (Table 5), indicating that the proteins are highly immunogenic in mice. The specificity of the induced antibodies was confirmed by immunoblotting against lysates (or protoplast supernatants) of *S.uberis* cells. The results showed that most of the induced antibodies specifically reacted with a *S.uberis* protein of the expected molecular mass clearly indicating that these proteins represent (surface) antigens that are capable of inducing an immune response in mice.

### Example 5

### Functional characteristics of the induced antibodies

We used the mice sera in a FACS analysis to study the binding of antibodies to whole encapsulated *S.uberis* cells (grown in Todd-Hewitt). As shown in Fig. 1 none of the sera obtained from mice before immunisation was able to bind to whole *S.uberis* cells. In contrast, however, eight of the immune sera (sera induced against proteins P11, P15, P17, P20, P25, P26, P27, P63) strongly bound to whole bacterial cells, whereas two of the sera (directed against proteins P 17, P18) showed a weak binding to whole bacterial cells. This clearly indicates that the proteins recognised by these sera were expressed under the conditions used for growing *S. uberis* bacterial cells, and were accessible for binding to antibodies. In addition, Fig. 1 clearly shows that the expression and/or surface accessibility of the proteins differed between the two strains used. Expression and/or surface accessibility were conserved in three of the proteins (P17, P19 and P20). In contrast, P11 and P63 were exclusively detected by using strain 0140J, whereas P15, P18, P25 and P26 were exclusively detected by using strain 41-241. P27 was surface exposed both on strains 41-241 and 0140J, but was only weakly recognised by antiserum on strain 41-241.

Taken together, the data clearly showed that 10 of the selected antigens are expressed on the surface of the bacterium grown in vitro and are available for binding of antibody on intact encapsulated cells. Three of these proteins were conserved among the two strains used.

### Example 6

### Serological proteome approach. As an alternative approach for the identification of S.uberis vaccine candidates, serological proteome analysis was applied.

Proteins of *S.uberis* strains grown in TH broth were separated by 2D gel electrophoresis and probed with antibodies present in sera of *S. uberis* infected animals. A number of highly immunogenic *S.uberis* proteins were identified (data not shown). Three spots were successfully matched to proteins present on a Coomassie brilliant blue stained 2D gel (Fig. 2). From these proteins tryptic digestion products were analyzed by Q-TOF and the resulting peptide-mass fingerprints were compared with the *in silico* generated peptide-mass fingerprints of all proteins predicted from the genome sequence analysis of strains 41-241 and 0140J. In addition, two major tryptic peptides selected from each fingerprint were used for tandem MS. The resulting amino acid sequence of the peptides was subsequently compared to sequences predicted from the *S.uberis* genome sequences. All three proteins could be matched successfully using this procedure. The properties of the identified proteins are listed in Table 6. One of these vaccine candidates was also identified using the genomic approach (P63). This underlines the importance of this protein as a vaccine candidate.

### Example 7

### ELISA on culture supernatant with mice immune-sera

P93 en P105 were strongly recognised in culture supernatants of both strains indicating that these proteins are secreted by the bacteria.

### Example 8

### FACS analysis using S.uberis cells grown in whey to mimic in vivo conditions.

The procedure of example 5 was followed to test binding of antibodies to *S.uberis* cells that were grown in a medium resembling milk.

### Example 9

### Conservation of antigens among diverse S.uberis strains.

The conservation of the expression of the selected proteins and the accessibility to antibodies among diverse *S.uberis* isolates by FACS analysis are studied. These studies allow selection of vaccine candidates directed to various *S.uberis* strains.

### Example 10

### Vaccination and challenge in cattle.

**Experimental infection of non-vaccinated animals.** The virulence of the *S.uberis* strains 0140J and 41-241 was determined after experimental infection. An udder is divided in four parts, generally called the quarters. Each quarter comprises milk secreting cells, milk ducts, a milk cistern and a teat. In this experiment, each quarter is individually infected in the milk cistern through the milk duct in the teat. Six out of eight quarters inoculated with strain 0140J became successfully infected (Fig. 3, Table 7). Pure cultures of *S.uberis* 0140J were isolated from milk obtained from these quarters and increased levels of somatic cell count (SCC) were detected. In two quarters (of two different cows; cows 6717 and 6719; Figs 3A and 3B) no infection could be detected after challenge. Both quarters remained bacteriological negative during the course of the experiment. In one of the two quarters a slight increase in SCC was observed. In contrast, in all eight quarters challenged with strain 41-241 infection was established (Figs. 3A and 3C; Table 7). Four of these quarters (cows 6721 and 6723) were inoculated with a dose of 5 x 10² cfu of strain 41-241, whereas the other four quarters were inoculated with a dose of 5 x 10³ cfu (cows 6720 and 6722). More severe effects were observed after inoculation with the higher dose: body temperature of the cows increased more significantly and more severe clinical signs of disease (clots in milk and firm consistency of udder) were observed (Table 7; Figs 3A and 3C). However, clinical signs of mastitis were also induced using strain 41-241 at an inoculation dose of 5 x 10² cfu . Similar data had previously been observed for strain 0140J (Hill, 1988).

Compared to strain 41-241, the clinical signs of mastitis obtained with strain 0140J (inoculation dose of 5 x 10² cfu and studied for 16 days) seemed more severe (Figs.3B and 3C). Three out of four quarters became successfully infected with strain 0140J and all three showed clinical signs of mastitis for at least 16 days after infection. Two of these quarters remained bacteriological positive for 16 days after infection (Fig. 3B) and in one quarter an increased level of SCC was detected for 35 days (data not shown). All four quarters infected with strain 41-241 showed clinical signs of mastitis for 10-13 days after inoculation, but were negative for clinical signs from day 13 onwards (Fig 3C). Two of these quarters (cow 6723) remained bacteriological positive during the course of the experiment (16 days), indicating the persistence of *S.uberis* in the mammary gland (Fig.3C).

Histological examination of udder material collected 3-5 days after infection generally corresponded to the clinical observations. Both cows infected with strain 41-241 and one cow infected with strain 0140J displayed a moderate to severe mastitis throughout the entire gland with multifocal intra-alveolar accumulation of polymorpho-nuclear granulocytes, focal disruption of the epithelial layer in the alveoli and moderate interstitial infiltration of mononuclear cells (data not shown). The second cow infected with strain 0140J had a mild, multifocal catarrhal mastitis. Taken together, these data show that both strains 0140J and 41-241 are pathogenic for cows.

### Immunization of dairy cows

Polyclonal antibodies against *S.uberis* proteins were raised in cows. Cows were immunized through various immunization schedules, using either subcutaneous inoculation, and/or intramuscularly and/or intra-mammary inoculation.

The immunogenic composition was formulated with an solvent like for example phosphate buffered saline and an adjuvant, for example water-in-oil adjuvant or an adjuvant without oil.

After immunization, a blood sample was collected and serum was tested for antibodies against *S.uberis.*

### Experimental infection of vaccinated animals.

Vaccinated and non-vaccinated cows were challenge infected with 500 cfu *S.uberis* strain 0140J. Each udder quarter was individually infected via the milk duct in the teat.

After challenge, cows vaccinated with an immunogenic composition of the invention showed less clinical signs of mastitis, less alterations in the milk, lower SCC levels, shorter period of clinical mastitis, less fever. Clinical scores and histological evidence of the udders clearly show that immunization according to the present invention is effective against mastitis caused by *S.uberis.*

### Example 11

**Reactivity of antigens with convalescent sera.** The ability of a selected number of proteins (P15, P16, P20, P22, P27, P54, P28, P63, P68, P75, P93 and P105) to induce convalescent antibodies was tested by Western blot analysis using field sera obtained from 14 cows having recovered from a recent *S*.*uberis* infection. Six out of 12 antigens selected (P15, P16, P54, P28, P63, P105) were recognized by all 14 convalescent sera used. These data indicate that these antigens are expressed by all *S. uberis* strains that caused the respective infections, that these antigens are expressed during infection in the host and that these antigens are highly immunogenic. Five of the antigens (P 68, P27, P20, P93 and P22) were recognized by 8, 9, 10, 11 or 12 of the convalescent sera respectively. With one of the antigens no reaction with any of the convalescent sera could be detected (P75).

### Example 12

**Conservation of antigens among diverse *S*. *uberis* strains.** To indicate the suitability of the antigens for conferring protection against various *S*. *uberis* strains, the conservation and expression of the 12 selected antigens (P15, P16, P20, P22, P27, P54, P28, P63, P68, P75, P93 and P105) among a collection of recently isolated field strains (35 strains) was determined. Expression of antigens was demonstrated by screening Western blots with mouse immune sera against the purified antigens. Five out of the 12 antigens (P15, P16, P28, P75, and P105) were expressed in >97% of the strains tested; two of the antigens (P27, P63) were expressed in 94% of the strains and four of the antigens (P20, P22, P54, P93) were expressed in 81-92% of the strains. These data clearly showed that expression of the most of the antigens is highly conserved among the various *S*. *uberis* strains.

### MATERIALS AND METHODS

### Bacterial strains and growth conditions.

One *S.uberis* strain 41-241 was isolated in 1998 from a commercial Dutch dairy farm on which an outbreak of *S.uberis* mastitis was observed (Hill, 1988). Strain 41-241 showed the RAPD fingerprinting type B predominantly found on the particular herd during the outbreak (Zadoks et al., 2003). The strain was isolated from a cow infected with *S.uberis* for at least two months. The onset of the infection was sub clinical and was followed by multiple clinical flare-ups.

The *S.uberis* strains 0140J and EF20 were kindly provided by Dr. J. Leigh, Institute for Animal Health, Compton, England. Other *S.uberis* and *S. parauberis* strains, isolated from clinical cases of mastitis on various Dutch dairy farms were kindly provided by Dr. D. Mevius, CIDC, Lelystad, The Netherlands; by Drs. O. Sampimon, Animal Health Service, Deventer, The Netherlands, or by Dierenartsen Praktijk, Diessen, The Netherlands. All other streptococcal species were from the laboratory collection of the ASG, Lelystad, The Netherlands. Streptococcal strains were grown in Todd-Hewitt broth (code CM189, Oxoid), and plated on Columbia agar blood base (code CM331, Oxoid) containing 6% (v/v) horse blood and 0.1% aesculin (w/v) unless indicated otherwise. *E.coli* strains were grown in Luria broth (18) and plated on Luria broth containing 1.5% (w/v) agar. If required, 50 µg/ml of kanamycin was added. **Preparation of whey.** Bulk milk was obtained from a dairy farm without a *S.uberis* infection in its history (Waiboerhoeve, Lelystad, The Netherlands). The milk was centrifuged for 30 minutes at 12,800 x *g* and fat was removed. Subsequently, 40 µl/ml of rennin (Lactoferm, Brouwland, Belgium) was added and the milk was incubated for 2 hr at 37°C with regular mixing. Coagulated milk was removed by sifting and the remaining supernatant was centrifuged for 30 minutes at 12,800 x *g*. The cleared supernatant was sterilized by filtration over a 0.2 um Sartobran P filter (Sartonus, Goettingen, Germany).

**Milk samples and sera.** Milk samples and sera were obtained from clinical *S.uberis* mastitis cases from various Dutch dairy farms (Drs. O. Sampimon, Animal Health Service, Deventer, The Netherlands). None of the animals had been treated with antibiotics before the samples were collected.

In addition, milk and sera were collected from cows at various time points after experimental infection with *S.uberis* strains O 140J and 41-241.

**Rabbit antisera.** Polyclonal antibodies directed against formalin-killed whole *S. uberis* cells as well as against sonicated *S. uberis* cells were raised in rabbits. Rabbits were immunized subcutaneously using 2-4 x 10⁹ killed cells in water-in-oil adjuvant. Inoculations were repeated two, three and four weeks later. After 6 weeks, rabbits were killed and serum was collected.

To prepare the antigens, *S.uberis* strains were grown for 16 h in Todd-Hewitt broth. The cultures were diluted 10 times in 1 pre-warmed Todd-Hewitt broth and cells were grown till optical density (600 nm) reached 0.5. The cultures were centrifuged for 15 min at 10,000 x *g*, and the pellets were dissolved in 100 ml of PBS (136.89 mM NaCl, 2.68 mM KCl, 8.1 mM Na₂HPO₄, 2.79 mM KH₂PO₄ pH 7.2). Subsequently, the optical density (600 nm) was adjusted to 1.0 with PBS. To prepare formalin-fixed cells 10 ml portions of these cells were centrifuged for 20 min. at 10,000 x g and the pellets were resuspended in 2.5 ml of PBS. To this suspension 250 µl of 3% formalin was added and it was maintained for 16 h at room temperature. The suspension was checked for the absence of live bacteria by plating on Columbia Agar plates. To remove formalin the cells were washed twice with PBS. To prepare sonicated cells 10 ml portions of the cells were centrifuged for 20 min. at 10,000 x g and the pellets were resuspended in 250 µl of PBS. Cells were sonicated for 15 min using a tip sonifier at 100% output, 50% duty cycle. After sonication, cells were diluted 10 times in PBS. Both antigens were mixed 1:1 with Specol to produce water-in-oil emulsions. **Genome sequencing.** Genomic DNA was isolated from *S.uberis* strain 41-241 as described by Sambrook et al. (1989). DNA was sheared and used to create a plasmid library. Random clones were sequenced using dye-terminator chemistry and analyzed with an ABI PRISM 3700 DNA analyzer (Applied Biosystems, Warrington, GB). Sequencing data were assembled to obtain 572 contiguous sequences. An initial set of open reading frames (ORFs) was identified with GLIMMER and GENEMARK software. Transmembrane helices and subcellular locations in the genes were predicted with a computer program called TMHMM available at www.cbs.dtu.dk/services/TMHMM. To search each ORF for the presence of signal peptides the program SignalP was used (Nielsen et al., 1999). Alternative predictions of signal peptides were done using the program PSORT available on http://psort.nibb.ac.jp and a program called GCG-SPScan available at http://www.biology.wustl.edu /gcg/spscan.html. Lipoproteins were found by using the GCG-Findpatterns program with the following expressions: PS00013: ∼(D,E,R,K)6(L,I,V,M,F,W,S,T,A,G)2(L,I,V,M,F,Y,S,T,A,G,C,Q)(A,G,S)C; g-lpp:<(M,V)X{0,13}(R,K)-(D,E,R,K,Q){6,20}(L,I,V,M,F,E,S,T,A,G)(L,V,I,A,M)(I,V,M,S,T, A,F,G)(A,G)C and g-lpp_rvh: (M,V,L)X{0,13}(R,K)∼(D,E,R,K,Q){6,20}(L,I,V,M,F,E,S,T,A,G)(L,V,I,A,M)(I,V,M,S,T,A, F,G)(A,G)C. Proteins with cell wall anchor domains were identified using InterPro accession IPR001899. The BLAST program was used to search for protein sequences with sequence identity to the deduced amino acid sequences.

**Spot blotting, Southern blotting and hybridization.** Chromosomal DNA was isolated as described by Sambrook et al. (1989). For spot blotting one µg of chromosomal DNA was spotted onto Genescreen Plus membranes. The membranes were incubated in 0.4 M NaOH-1 M NaCl at room temperature for 10 min. to denature the DNA and for 10 min in 0.6 M NaCl, 0.06 M sodium citrate (pH 7.0) for neutralization. For Southern blotting DNA fragments were separated on 0.8% agarose gels and transferred to Gene-Screen Plus membranes (NEN) as described by Sambrook et al. (1989). DNA probes were labeled with [(α-³²P]dCTP (3000 Ci mmol⁻¹; Amersham) by use of a random primed labeling kit (Boehringer). The DNA on the blots was hybridized at 65°C in a buffer having 0.5 M sodium phosphate, 1 mM EDTA, and 7% sodium dodecyl sulphate at a pH of 7.2, with the appropriate DNA probes as recommended by the supplier of the Gene Screen Plus membranes. After hybridization, the membranes were washed twice with a solution of 40 mM sodium phosphate, pH 7.2, 1 mM EDTA, 5% SDS for 30 min at 65°C and twice with a solution of 40 mM sodium phosphate, pH 7.2, 1 mM EDTA, 1% SDS for 30 min at 65°C. Signals were detected on a phosphor-imager (Storm; Molecular Dynamics).

**Cloning and expression of selected proteins.** Selected ORFs were amplified by PCR with specific oligonucleotide primers for cloning into pET200/D-TOPO (Invitrogen). Proteins were cloned without putative signal sequences or predicted transmembrane regions. Constructs were transformed into *Escherichia coli* BL21 Star (DE3) (Invitrogen) for expression of recombinant proteins.

For PCR reaction (25 µl) Platinum Pfx DNA polymerase (Invitrogen) was used as described by the supplier. DNA amplification was carried out in a Perkin Elmer 9700 thermal cycler and the program consisted of an incubation for 5 min at 94°C, 35 cycles of 15 sec at 94°C, 30 sec at 57°C and 2 min at 68°C, and 5 min at 68°C. **Immunodetection of the expressed antigens.** Proteins were separated by SDS-polyacrylamide gel electrophoresis using the XCell *SureLock* mini-cell system (Invitrogen). Proteins in the gel were visualized using SYPRO-orange (Molecular Probes, Sunnyvale, Calif.) staining according to the manufacturer's recommendations. Signals were detected on a phosphor-imager (Storm; Molecular Dynamics).

Proteins were transferred to a nitrocellulose membrane by standard procedures (19). The membranes were blocked in Blotto: Tris-buffered saline (TBS) (50 mM Tris-HCl [pH 7.5], 150 mM NaCl) containing 4% skimmed milk, 5% foetal calf serum and 0.05% Tween 20, at room temperature (RT) for 16 h. To detect recombinant antigens, membranes were incubated with a monoclonal antibody against the 6 x HIS tag (Clontech, Palo Alto, CA.). Bound antibodies were detected and visualized using alkaline phosphatase-conjugated anti-mouse antibody and nitro-blue-tetrazolium/5-bromo-4-chloro-3-indolyl-phosphate as described by Sambrook et al. (1989). Immunogenicity of expressed antigens was tested by using serum samples obtained from cows clinically or sub clinically infected with *S.uberis* or using rabbit anti-*S.uberis* antisera. Bound antibodies were detected with rabbit-anti-cow or goat-anti-rabbit immunoglobulins conjugated with alkaline phosphatase (Jackson Immunoresearch) and visualized using nitro-blue-tetrazolium/5-bromo-4-chloro-3-indolyl-phosphate as described by Sambrook et al. (1989).

**Protein purification.** Proteins were affinity purified from solubilised cell pellets using Ni-nitrilotriacetic acid (Ni²⁺-NTA) column chromatography as described by the manufacturer (Qiagen). In short, cells were grown exponentially; 1 mM IPTG was added and the cells were allowed to grow another 4 hr at 37°C. Subsequently, cells were harvested and lysed. The cleared supernatants were loaded onto Ni²⁺-NTA agarose columns. The columns were washed and the protein was eluted. Different buffers were used for native and for denaturing purification. Proteins purified under denaturing conditions were renaturated by dialysis using a linear 6 M - 0 M urea gradient in 286,89 mM NaCl, 2.68 mM KCl, 8.1 mM Na₂HPO₄, 2.79 mM KH₂PO₄ pH 7.2. Purified proteins were further concentrated using Amicon Ultra-4 5000 MWCO filters (Millipore).

**Protein concentration.** Protein concentration in the samples was determined after SDS polyacrylamide gel electrophoresis. Proteins in the gel were visualized using SYPRO-orange (Molecular Probes, Sunnyvale, Calif.) staining according to the manufacturer's recommendations. Signals were detected on a phosphor-imager (Storm; Molecular Dynamics). A known bovine serum albumin concentration range was used as a standard, to calculate the amounts of protein present in the gel. The Molecular Dynamics program was used for the calculations.

**Immunogenicity of purified proteins.** OF1 mice were immunized subcutaneously using 20 µg of purified proteins in Freunds complete adjuvant. Inoculations were repeated three weeks later using 20 µg of purified proteins in Freunds incomplete adjuvant. Three weeks after the second inoculation mice were killed and serum was collected.

**FACS-analysis.** *S.uberis* cells were grown in Todd-Hewitt broth until the OD₆₀₀ reached 0.5. The cells were collected by centrifugation, washed once in FACS-buffer (PBS-13, pH7,2 [137 mM NaCl, 2,68 mM KCl, 8.1 mM Na₂HPO₄, 2.8 mM KH₂PO_{4]} - 0,5%BSA) and the cell density was adjusted to approximately OD₆₀₀ 1.0 in FACS buffer. The cells (250 µl) were collected by centrifugation and resuspended in 50 µl of FACS-buffer containing mice antisera (in a 1: 50 dilution). The sample was incubated for 45 minutes on ice. To remove unbound antibodies the cells were washed twice with 250 µl of FACS-buffer. Subsequently cells were incubated with 50 µl FACS buffer containing fluorescein isothiocyanate (FITC)-labeled rabbit-anti-mouse secondary antibody (1: 100 dilution; DAKO A/S, Glostrup, Denmark) for 30 minutes on ice. Cells were washed twice with 250 µl of FACS-buffer, resuspended in 100 µl FACS-buffer and bound antibody was detected a fluorescence activated cell sorter (FACS Calibur, Benton Dickinson, Franklin Lakes, USA).

**Whole cell ELISA.** Exponentially growing *S.uberis* cells were collected by centrifugation, resuspended in coating buffer pH 9,6 (0,05M NaHCO₃, 0,05M Na₂CO₃) and cell density was adjusted to OD₆₀₀ 1.0. High binding 96-wells plates were coated with 100 µl of this suspension per well for 16 hr at 4°C. Wells were washed 4 times with ELISA buffer (5% Tween-80, 0,02% Na-azide), sera (diluted 1:20 in PBS-13 containing 0,05% Tween-80, 2% NaCl, and 5% fetal calf serum) were added and plates were incubated for 1 hr at 37°C. To remove unbound antibodies, wells were washed four times with ELISA buffer. Subsequently, secondary antibody (100 µl of horse radish peroxidase conjugated rabbit-anti-mouse (DAKO) diluted 1:250 in PBS-13 containing 0,05% Tween-80, 2% NaCl, and 5% fetal calf serum) was added and plates were incubated for 1 hr at 37°C. Wells were again washed four times with ELISA buffer and bound antibodies were detected at room temperature using 100 µl of tetramethylbenzidine (TMB) (CeDi-Diagnostics, Lelystad, The Netherlands). Reactions were stopped after 15 min by the addition of 100 µl of 0,5M H₂SO₄ per well. Absorbance was read using an ELISA reader (Thermo Labsystems, Franklin, USA) at 450 nm.

**Sample preparation for two-dimensional gel electrophoresis.** *S.uberis* strains were grown for 16 h in 100 ml Todd-Hewitt broth. The cultures were diluted 20 times in 1 l pre-warme Todd-Hewitt broth and cells were grown till optical density (600 nm) reached 0.5. The cultures were centrifuged for 20 min at 10,000 x *g*, and the pellets were washed once with an equal volume of 250 mM sucrose/25 mM Tris, pH 8.0 and once with an equal volume of superQ. The resulting pellets were dissolved in 5 ml of superQ. 1.5-ml portions of these suspensions were sonicated for 15 min using a tip sonifier (Branson sonifier 250, 50% interval, amplitude 3). Subsequently, the suspensions were treated with DNAse I and MgCl₂ (final concentrations of 6.5 µg/ml and 10 mM, respectively) for 10 min at 37°C. Protease inhibitors pepstatin A, leupeptin, pefabloc and aprotinin were added to final concentrations of 2.5 µg/ml, 5 µg/ml, 25 µg/ml and 1 µg/ml, respectively. Urea, dithiothreitol and Triton-X100 were added to a final concentration of 9M, 70mM and 2%, respectively. The samples were centrifuged for 30 min at 10,000 x *g*, the supernatants were collected and centrifuged for an additional 30min at 100,000 x *g*. The supernatants were collected and protein concentration in the samples was determined using the *RC DC* Protein Assay (BioRad) according to the manufacturer's instructions.

**Two-dimensional gel electrophoresis.** Samples containing 50 - 100 µg of protein were solubilized in 450 ul of sample buffer (8M urea, 2% CHAPS, 0,5% IPG-buffer 3-10, 70 mM dithiothreitol and a trace of bromo-phenol-blue). Proteins were separated in the first dimension by isoelectric focusing using Immobiline 18 cm DryStrips (3-10 NL Amershan Pharmacia Biotech) on an IPGphor (Amershan Pharmacia Biotech) after rehydration of the strips according to the manufacturer's instruction. Immediately after being focused, the strips were subsequently equilibrated for 15 min in equilibration buffer (6M urea, 30% glycerol, 2% SDS, 50 mM Tris-HCL-pH 8.8, trace of bromo-phenol-blue) containing 10 mg/ml dithiothreitol and for 15 min in equilibration buffer containing 25 mg/ml of iodoacetamide. Proteins were separated in the second dimension by SDS-polyacylamide gelelectrophoresis on 12.5% pre-cast Ettan DALT gels (Amershan Pharmacia Biotech) in an Ettan DALT twelve system (Amersham Pharmacia Biotech) according to the manufacturer's instructions. **Staining.** Proteins in gels were stained with silver using the PlusOne™ Silver Staining Kit (Amershan Pharmacia Biotech) according to the manufacturer's instructions or with Coomassie Brilliant blue as described by Sambrook et al. (1989) with prolonged incubation times due to the plastic backing of the gels.

**Digestion of proteins from 2D gels.** Protein spots identified on Coomassie stained gels were manually excised. Gel pieces were frozen in 0.1% acetic acid at -80°C until use. Proteins in the gels were digested with trypsin as described by Li et al. (2003).

**Mass spectrometry of tryptically digested proteins spots from 2 D gels.** A Micromass Q-TOF mass spectometer was used to analyse the masses of the tryptically digested proteins spots as described by Li et al. (2004).

### Experimental infection experiments

**Animals.** Clinically healthy Holstein-Friesian cows, 2-4 weeks in their first lactation, were used for infection. The cows were milked twice daily at 7.00 a.m. and 4.00 p.m. All cows had somatic cell counts (SCC) below 2.0 x 10⁵ cells/ml, were negative for mastitis pathogens based on repeated microbiological evaluation of milk during the last 14 days prior to infection and had no history of mastitis..

**Preparation of the inoculum and inoculation.** *S.uberis* strains 0140J and 41-241 were used as inocula. Single colonies, grown on Columbia agar plates containing 6% horse blood (v/v) and 0.1% aesculin (w/v), were transferred into 90 ml Todd-Hewitt broth (Oxoid) and cultured overnight at 37°C. Overnight cultures were diluted 1 to 10 in the same medium and bacteria were grown to a concentration of approximately 3 x 10⁸ cfu/ml (logarithmical growth phase). Cells were then collected by centrifugation and resuspended and diluted in PBS.

Two quarters of each cow were inoculated intracisternally with either 5 x 10² (either strain 0140J or strain 41-241) or 5 x 10³ (strain 41-241) cfu per 5 ml of PBS. Control quarters were inoculated with 5 ml of PBS. Injections were done just after the afternoon milking using disposable teat canulas. Before inoculation teats were cleaned with alcohol. The inocula were massaged upwards into the gland cisterns. One group of four cows was challenged with 5 x 10² cfu of strain 0140J. Another group of four cows was challenged with strain 41-241. Two of these cows were challenged with 5 x 10² cfu and two cows with 5 x 10³ cfu. Cows were used in two consecutive experiments, one was finished after 45 to 80 hours and the second after 16 days.

**Sampling and clinical scores.** Milk samples were collected aseptically from all quarters at each milking. The samples were examined bacteriologically on blood agar plates containing 6% horse blood (v/v) and 0,1% aesculin (w/v) and the SCC was determined using standard procedures (International Dairy Federation, 1981). In addition milk samples were stored at -20°C until analysis for antibody responses. At each milking the body temperature of the cows and milk production was determined and cows were monitored for clinical signs of mastitis (consistency of the udder and clots in the milk). Once a week blood samples were collected for analysis of antibody responses in serum.

**Pathology.** For histological examination mammary gland tissue from each quarter was sampled from different sites of three different horizontal cross sections, i.e. at the gland basis, halfway between basis and cisterne and at the gland cisterne. Tissue samples were fixed in 4% buffered formalin and embedded in paraffin. For histological examinations tissue sections were cut and stained with hematoxylin/eosin stain.

### REFERENCES

Alber, T. 1989. Mutational effects on protein stability. Annu. Rev. Biochem. 58, 765-798.
Adv. Biochem. Eng. 43, 75-102 (1990)
Agri. Biol. Chem. 51, 51, 1587 (1987)
Appl. Environ. Microbiol. 50, 94 (1985)
Biotechnology 7, 596-603 (1989) **Bramley** 1991 *Mastitis: physiology or pathology.* p. 3-9. in **Burvenich, C., G.**
Vandeputte-van Messom, and A.W. Hill (ed.), New insights into the pathogenesis of mastitis. Rijksuniversiteit Gent, Belgium
Biseibutsugaku Kisokoza (Basic Microbiology), 1990, Vol. 8, Genetic Technology, Kyoritsu Shuppan
De Greeff et al (2002), Infect Immun. 70: 1319-1325
FEMS Microbiol. Lett. 26, 239 (1985)
Fersht, A.R. and L. Serrano, 1993. principles of protein stability derived from protein engineering experiments. Curr. Opinion Struct. Biol., 3, 75-83
Finch, J.M., A. Winter, A.W. Walton and J.A. Leigh. 1997. Further studies on the efficacy of a live vaccine against mastitis caused by Streptococcus uberis. Vaccine 15: 1138-43. **Fontaine, M.C., J. Perez-Casal, X.M. Song, J. Shelford, P.J. Willson, A.A.**
Potter. 2002. Immunisation of dairy cattle with recombinant Streptococcus uberis GapC or a chimeric CAMP antigen confers protection against heterologous bacterial challenge. Vaccine 20: 2278-2286.
Grandi, G. 2001. Antimicrobial vaccine design using genomics and proteomics. Trends in Microbiol. 19: 181-188.
Hill, A. W. 1988. Pathogenicity of two strains of Streptococcus uberis infused into lactating and non-lactating bovine mammary glands. Res Vet Sci. 45: 400-404.
Hillerton J.E., M.F. Shearn, R.M. Teverson, S. Langridge, and J.M. Booth 1993. Effect of pre-milking teat dipping on clinical mastitis on dairy farms in England. J. of Dairy Research 60: 31-41.
Hogan, J.S., K.L. Smith, K.H. Hoblet, D.A. Todhunter, P.S. Schoenberger, W.D. Hueston, D.E. Pritchard, G.L. Bowman, L.E. Heider, B.L. Brockett et al. 1989. Bacterial counts in bedding materials used on nine commercial dairies. J. Dairy Sci 72: 250-258.
J. Bacteriol. 137, 614 (1979)
J. Bacteriol. 145, 382-390 (1981)
Leigh, J.A. 1999. Streptococcus uberis: a permanent barrier to the control of bovine mastitis?. Vet. J. 157: 225-238.
Leigh, J. A. 2000. Vaccines against bovine mastitis due to Streptococcus uberis current status and future prospects. Adv. Exp. Med. Biol. 480: 307-311.**Li, K.W., M.P. Hornshaw, R.C. van der Schors, R. Watson, S. Tate, B. Casetta,**
C.R. Jimenez, Y. Gouwenberg, E.D. Gundelfinger, K-H. Smalla, and A.B. Smit 2004 Proteomics Analysis of Rat Brain Postsynaptic Density. J. Biol. Chem. Vol.279, 987-1002.
Matthews, B.W. 1991. Mutational analysis of protein stability. Curr. Opinion Struct. Biol., 1, 17-21.
McDougall, 1998. Efficacy of two antibiotic treatments in curing clinical and subclinical mastitis in lactating dairy cows. New Zealand Vet. J. 46, 226-232.
Mol. Cell. Biol. 5, 3376 (1985)
Neave F.K., F.H. Dodd, R.G. Kingwell and D.R. Westgarth. 1969. Control of mastitis in the dairy herd by hygiene and management. J Dairy Sci.52: 696-707.
Nielsen, H., S. Brunak, and G. von Heijne. 1999. Machine learning approaches to the prediction of signal peptides and other protein sorting signals. Protein Engineering 12: 3-9.
Oliver S.P., M.J. Lewis, B.E. Gillespie, S.J. Ivey, L.H. Coleman, R.A. Almeida, W. Fang, and K. Lamar. 1999. Evaluation of a postmilking teat disinfectant containing a phenolic combination for the prevention of mastitis in lactating dairy cows. J. of Food Protection 62: 1354-1357.
Paton, J.C. and P. Giammarinaro. 2001. Genome-based analysis of pneumococcal virulence factors: the quest for novel vaccine antigens and drug targets. Trends in Microbiol. 9: 515-518.
Pizza M, V. Scarlato, V. Masignani, M.M. Giuliani, B. Arico, M. Comanducci, G.T. Jennings, L. Baldi, E. Bartolini, B. Capecchi, C.L. Galeotti, E. Luzzi, R. Manetti, E. Marchetti, M. Mora, S. Nuti, G. Ratti, L. Santini, S. Savino, M. Scarselli, E. Storni, P. Zuo, M. Broeker, E. Hundt, B. Knapp, E. Blair, T. Mason, H. Tettelin, D.W. Hood, A.C. Jeffries, N.J. Saunders, D.M. Granoff, J.C. Venter, E.R. Moxon, G. Grandi and R. Rappuoli. 2000. Identification of vaccine candidates against serotype B Meningococcus by whole-genome sequencing. Science 287: 1816-1820.
Rappuoli, R. 2000. Reverse vaccinology. Current Opinion in Microbiol. 3: 445-450. Wizemann T.M., J.H. Heinrichs, J.E. Adamou, A.L. Erwin, C. Kunsch, G.H. Choi, S.C. Barash, C.A. Rosen, H.R. Masure, E. Tuomanen, A. Gayle, Y.A. Brewah, W. Walsh, P. Barren, R. Lathigra, M. Hanson, S. Langermann, S. Johnson and S. Koenig. 2001. Use of whole genome approach to identify vaccine molecules affording protection against Streptococcus pneumoniae infection. Infect. Immun. 69: 1593-1598.
Sambrook, J., E.F. Fritsch, and T. Maniatis. 1989. Molecular cloning: a laboratory manual, 2nd ed. Cold Spring Harbor Laboratory Press, Cold Spring Harbor, N. Y. Schalm et al. (1971) The mastitis complex-A brief summary. p. 1-3. In Bovine Mastitis. Lea & Febiger, Philadelphia.
Trends in Biotechnology 7, 283-287 (1989)
**Van der Burg, S.H., W.M. Kast, R.E.M. Toes, R. Offringa, C.J.M. Melief,** Methods for selecting and producing T cell peptide epitopes and vaccines incorporating said selected epitopes. International Patent Application WO 97/41440. Vriend, G. and V.G.H. Eijsink, 1993. Prediction and analysis of structure, stability and unfolding of bacillus neutral proteases. J. Computer-Aided Mol. Design 7, 367-396.
Wren, B.W. 2000. Microbial genome analysis: insights into virulence, host adaptation and evolution. Nature Genetics 1: 30-39.
Yeast 8, 423-488 (1992).**Zadoks R.N., H.G. Allore, H.W. Barkema, O.C. Sampimon, Y.T. Grohn and Y.H.**
Schukken. 2001. Analysis of an Outbreak of Streptococcus uberis mastitis. J. Dairy Sci.84: 590-599.**Zadoks, R.N., B.E. Gillerpie, S.P. Oliver, H.W. Barkema, O.C. Sampimon, and**
Y.H. Schukken. 2003. Clinical, epidemiological and molecular characteristics of Streptococcus uberis infections in dairy herds. Epidemiol Infect. 130: 335-349.
Zadoks, R. N., H. G. Allore, H. W. Barkema, O. C. Sampimon, G. J. Wellenberg, Y. T. Grohn and Y. H. Schukken. 2001. Cow- and quarter-level risk factors for Streptococcus uberis and Staphylococcus aureus mastitis. J. Dairy Sci. 84: 2649-2663.

**TABLE 1. Surface proteins of S.uberis containing a LPxTGE sortase motif**

| Protein | contig. nr. | aa (nr.) | Data base homology (accession no.) | % Identity | predicted sorting signal |
|---|---|---|---|---|---|
| P11 | 231c11 | 1270 | *S. mutans* Exo-beta-D-fructosidase (U78296) | 54 | LPMTSD SNNNLEELGILVILTTLGAFLGRVILKKEK |
| P12 | S00737 | 317 | *S. agalactiae* hypothetical protein unknown (Q8CM32) | 60 | LPNTGE SSIAPFTAIGAIILSVLGLLGFKKRRTY |
| P13 | 129h4 | 484 | *S. pyogenes* collagen like protein (AY069936) | 45 | LPSTGD KANPFFTAAALAVMASAGMVAVSRKRKED |
| P14 | K00518 | 565 | *S. pyogenes* collagen like protein (AF336814) | 44 | LPSTGD KANPFFTAAALAVMASAGMVAVSRKRKED |
| P15 | 130g06 | 693 | *S. pyogenes* 5'-nucleotidase (NC_004070) | 46 | LPTTSS QEDTAILLSLLGASSLAMAVALKKKENN |
| P16 | 223b05 | 268 | no homology | -- | LPSTGE DYQAYLVAAAMALIASSGMVAYGSYRKKKQK |
| P17 | 52g05 | 1074 | *Bacillus halodurans* unknown (NC_002570) | 34 | LPALAD GSHKDDSKLFWVTGLLVASGGLFAALKRREED |
| P18 | 240d11 | 499 | *S. aureus* fibrinogen-binding protein homolog (AJ005646) | 26 | LPMAGE RGSRLFTFIGLSLILGIAGYLLKHKKVKS |
| P19 | 32a09 | 278 | *S. pneumoniae* beta-N-acetyl-hexosaminidase precursor (NC_003098) | 32 | LPPTGS QESGIFSLFSALISTALGLFLLKSNKND |
| P20 | 122b06 | 506 | *S. uberis* lactoferrin binding protein (AAQ83577.1) | 42 | LPSTGD KPVNPLLVASGLSLMIGAGAFVYAGKRKKG |
| P22 | 130g06 | 1483 | *S. pneumoniae* serine proteinase precursor PrtA (AF127143) | 25 | LPETRD SSSMANWSLAFFLSAVICFFKGRRKRLNKL |
| P23 | S03520 | 456 | no hits | -- | LPTTGD KADGSIVQMVIGALMVSFVGFSALKDRKKEK |
| P24 | S00737 | 238 | *S. agalactiae* hypothetical protein unknown (Q8CH3) | 46 | LPHTGE EKGFLSIIGGTILSFVAFLFKKKITLN |
| P25 | S00737 | 876 | *S. agalactiae* hypothetical protein unknown (Q8CMF2) | 49 | LPHTGE EGLSILTVIGASILSVLGLSVLKKPKEN |
| P26 | 224g12 | 649 | *Schizosaccharomyces pombe* hypothetical protein (NC_003421) | 22 | LPTTGD DQNLLVTLMSSLLLMSLGLGLKKKEDE |
| P27 | 113f05 | 1144 | *S. pyogenes* C5a peptidase (NC_004070) | 34 | LPKTDS QKTMTFLGIAMLFGGILQVLWSYFKKRD |
| P115 | 69h12 | 818 | S. suis cyclo-nucleotide phosphodiesterase (AB066354) | 64 | LPKAGS QESKGLFFMGLSLLGLAGLITKKEERQ |

**TABLE 2. Putative lipoproteins of S.uberis**

| Protein | contig. nr. | aa (nr.) | Data base homology (accession no.) | % Identity | predicted sorting signal |
|---|---|---|---|---|---|
| P30 | 198g02 | 293 | *S.pneumoniae* phosphate binding protein (AAL00697.1) | 71 | MKIMKMNKMLTLAVLTLSSFGLAAC |
| P31 | 111h03 | 451 | *S.pneumoniae* sugar binding protein (AAK75762.1) | 24 | MSKKILKLATLAILPFVGLTAC |
| P32 | 32a09 | 309 | *S. pyogenes* laminin adhesion (AAL98544.1) | 62 | MKRKFLSFILVLTFFLPFLVGLSAC |
| P33 | 71g04 | 314 | *S*. *pyogenes* protease maturation protein (AAK34209.1) | 61 | MNTSKKIVTGFVTLASVLTLAAC |
| P34 | 113f05 | 416 | *S. pyogenes* maltose/maltodextrin-binding protein (AAL97920.1) | 85 | MKSWQKIIVSGASLTLASTLLVGC |
| P35 | 121e03 | 212 | *S. pyogenes* hypothetical protein (AAK34138.1) | 57 | MIGLLMKTQKSITLLLLSVLC |
| P36 | 121e03 | 277 | *S. uberis* amino acid binding protein (AF086736) | 97 | MNLKKILLTTLALASTLFLVAC |
| P37 | 108f04 | 351 | *S. pyogenes* putative lipoprotein (AAK34087.1) | 78 | MNKKFIGLGLASVAILSLAAC |
| P38 | 108f04 | 285 | *S. pyogenes* phosphate binding protein (AAK34100.1\|) | 80 | MKKFFLVGMLTLSMLTLTAC |
| P39 | 45f07 | 282 | *B. subtilis* amino acid binding protein (CAB12132.1) | 49 | MKKLFIYLSLAFSLLVLGAC |
| P40 | 198g02 | 310 | *S. pyogenes* metal binding protein (AAL97215.1) | 79 | MKKKLSLAIMAFLGLLMLGAC |
| P41 | 130c11 | 311 | *S. pyogenes* ferrichrome binding protein (AAL97215.1) | 70 | MKKLLVTLVLIFSTLSLIAC |
| P42 | 130c11 | 307 | *S. pyogenes* hypothetical protein (AAK33400.1) | 70 | MKIKLNRILFSGLALSILITLTGC |
| P43 | 130c11 | 281 | *S. pyogenes* hypothetical protein (AAL97072.1) | 76 | MSYKKILGLIGLTLVSSVLVAC |
| P44 | 130c11 | 274 | *S. pyogenes* hypothetical protein (AAL97071.1) | 66 | MTLKKNLGILSLTLGTLAILAAC |
| P45 | 240d11 | 434 | *S. mutans* sugar-binding protein (AAN59568) | 52 | MIKFETKKGQKLFLFGLILCFC |
| P46 | 141f06 | 272 | *S. pyogenes* hypothetical protein (AAK33324.1\|) | 67 | MKVKKNIKIAALLPMLTLLAAC |
| P47 | 125g06 | 539 | *S. pneumoniae* substrate-binding protein (AAK75869.1) | 76 | MKKRWIASSVIVLASTIVLGAC |
| P49 | 122b05 | 347 | *S. pyogenes* putative ABC transporter (AAM78733.1) | 65 | MNKKLTSLALLSAAIIPLAAC |
| P50 | 161c09 | 552 | *S. equi* hyaluronate-associated protein (AF100456) | 79 | MTVAQKSTFKRFGLGAVTLASAALLMAC |
| P51 | 198b02 | 268 | *S. pyogenes* substrate-binding protein (AAL97497.1) | 52 | MKTKKILKAAIGLMTLVSMTAC |
| P52 | 198g02 | 270 | *S. pyogenes* peptidyl-prolyl cis-trans isomerase (AAM78928.1) | 67 | MKKIISFALLTLSLFSLSAC |
| P53 | 231c11 | 173 | *Methanosarcina acetivorans* hypothetical protein (AAM03977.1) | 29 | MKKTFTSTLVLLSALMLTAC |
| P54 | 53f09 | 286 | *S. uberis* streptokinase (AJ131604) | 100 | MKKWFLILMLLGIFGC |
| P55 | 68d07 | 127 | *S. pyogenes* hypothetical protein (AE014145) | 61 | MGNYFKSLCLLLFSFLLVAC |
| P56 | 69h12 | 357 | no homology | -- | MSKIVKKIFFLTAFLIMFFLSAC |
| P57 | 87h02 | 390 | *S. pneumoniae* branched-chain amino acid binding protein (AE007382) | 46 | MKKKLLVSTIACLSLLSLAAC |
| P58 | 69h12 | 221 | *Clostridium acetobutylicum* cell wall-associated hydrolases (AE007516) | 44 | MINKKIIFTSLTVICISNC |
| P112 | 231c11 | 320 | *S. agalactiae* putative lipoprotein (NP_689064) | 45 | MKKGMRFSLILLALMLLTAC |
| P113 | 52g05 | 322 | L. lactis ribose binding protein (NP_689064.1) | 59 | MKCIKKLGFLALFLSMLLLLGAC |
| P117 | 198g02 | 291 | *S. pneumoniae* phosphate binding protein (AE007497) | 72 | MKMNKMLTLAVLTLSSFGLAAC |

**TABLE 3. Selected putative surface proteins/virulence factors of S.uberis**

| Protein | contig. nr. | aa (nr.) | Data base homology (accession no.) | % Identity | predicted cleavable signal sequence |
|---|---|---|---|---|---|
| P2 | 130c11 | 183 | *S.pyogenes* cytoplasmic membrane protein (AAK33386.1) | 80 | - |
| P3 | 130c11 | 297 | *S.pyogenes* heat shock protein | 66 | MLYQQIAQNKRKTIFLILAFFFLLTAIGA (AE014141) |
| P4 | 198g02 | 452 | *S. pyogenes* sensor histidine kinase (AE014144) | 82 | - |
| P5 | 67h05 | 362 | *S. pyogenes* choline binding protein (AE006476) | 52 | MKFIKILLSQIVSLFLLLTISLHALETVNA |
| P6 | 113f05 | 416 | *S. pneumoniae* DNA entry nuclease (NP_346391.1) | 60 | MSNKYPSGKKISAILIALLITGLTALSQG |
| P7 | 231c11 | 300 | *S.pyogenes* ABC transporter (AAL96916.1) | 47 | - |
| P9 | 231c11 | 146 | *S. pyogenes* competence protein (AE014138) | 40 | MKISCCHSKAFTLAESLLCLAVTTFTILLLSSSLAGV |
| P21 | 80b12 | 246 | *S. pyogenes* acyltransferase (AAM79682.1) | 77 | - |
| P28 | 130g06 | 246 | *S. pyogenes* peptidoglycan hydrolase (AE006536\|) | 60 | MRFLKGKKVFLAVIGLAVMMTLVIMFQPQAKN |
| P29 | 224g12 | 550 | *S. pyogenes* hypothetical protein | 46 | MKKIFQRKWFKRTSIVLGILLVALIALG (AAK34725.1) |
| P59 | 111h03 | 174 | *Corynebacterium jeikeium* YpkK | 43 | MKKKFLKIMTCIIAICSIFPYLSSMASTVYA (AF486522_8) |
| P60 | 113b07 | 248 | *S. pyogenes* D,D-carboxypeptidase | 70 | MMKNNKLLSFLFQLLVILLFIFCLFYYIKA (AAM80142.1) |
| P61 | 130c11 | 195 | *S. pyogenes* N-acetyl-muramidase (AAL98352.1) | 45 | MRNRLTFSYFIGIFLTFLFLLITPLIVNSQA |
| P62 | 130c11 | 421 | *S. pyogenes* protein DltD | 67 | MLRKLLTIVGPVFLALLLVLVTIFS (AAM79598.1) |
| P63 | 114a06 | 878 | *S. pyogenes* surface exclusion protein (AAK33344.1) | 36 | MEFENTKSNQIKTTLALTSTLALLGTGVGMGHTVNA |
| P64 | 115e06 | 428 | *S. pyogenes* hypothetical protein (AAK33154.1\|) | 57 | MKKLLACMLMVFFLSPISVISTEKSIS |
| P65 | 115e06 | 400 | *S. pyogenes* serine protease (AAK34840.1\|) | 71 | MPVSKFKHFFKYIMIVGLGFIGGALAFFVMNLLPHPSST |
| P66 | 115e06 | 656 | *S.pyogenes* hypothetical protein (AAM80444.1) | 71 | MKKFRFETIHLVMMGLILFGLLALCVRIMQSKMLIILA |
| P67 | 115e06 | 302 | *S. pyogenes* hypothetical protein (AAK34806.1) | 22 | MKTWKKTILITSLCLLISGAALAGFGFIRGGWS |
| P68 | 115e06 | 216 | *S. pyogenes* hypothetical protein (AAK34820.1) | 41 | MIRKENFKKRYISFGILGFAVALLALVFAF |
| P69 | 121e03 | 185 | *S. pyogenes* signal peptidase I (AAM79518.1) | 54 | MVKRDFIRNIILALLAIVIFILLRIFVFS |
| P70 | 129h04 | 318 | *S. pyogenes* hypothetical protein (AAM79277.1) | 73 | MKSFFNSRIWLGLVSVFFAIVLFLTA |
| P71 | 130c11 | 535 | *S. pyogenes* hypothetical protein (AAK34403.1\|) | 77 | - |
| P72 | 130c11 | 739 | *S. pyogenes* penicillin binding protein 1A (AAL98205.1) | 80 | - |
| P73 | 130g06 | 307 | *S. pyogenes* hypothetical protein (AAL97680.1\|) | 68 | MRRQKKQQKKIIPLFLILLFSTLLLFTGFLFKKELRA |
| P74 | 130g06 | 317 | *S. pneumoniae* hypothetical protein (AAK99735.1) | 61 | MFKKKLMLTGLILFSGMTVSTASA |
| P75 | 133f06 | 241 | *S. pyogenes* hypothetical protein (AAM80084.1\|) | 29 | MKPSNTEKLFLILSLLTLILAGSFYLFFARNHIGNA |
| P76 | 141f06 | 212 | no significant hits | -- | MKKIQKIFIALSTMILLLSNIFSTIIYA |
| P77 | 141f06 | 304 | *S. uberis* UDP-glucose pyrophosphorylase (AJ400707) | 100 | MTKVRKAIIPAAGLGTRFLPATKALA |
| P78 | 141g05 | 386 | *S. pyogenes* hypothetical protein (AAK34063.1) | 48 | - |
| P79 | 149b09 | 304 | *S. pyogenes* hypothetical protein (AAL97494.1) | 63 | - |
| P80 | 153a02 | 1058 | *S. pneumoniae* carbamoyl-phosphate synthase (Q97QE4) | 75 | - |
| P81 | 153f08 | 518 | *S. pyogenes* adhesion protein (AAL97448.1) | 69 | MKKKTLVMMGLAGLVAGGQLYQAKAVLA |
| P82 | 198g02 | 441 | *S. pneumoniae* histidine kinase (AAL00696) | 57 | - |
| P83 | 198g02 | 288 | *S. thermophilus* Peb1 (AF327739) | 61 | MKKFKPRKKSDIKRRIAMNQFKKWTFFCLMTLLTLIFMPKASA |
| P84 | 198g02 | 126 | no significant hits | -- | MLLRKARHSLKRRHMMLEVLLIVSTFFLFIIFISLLIGIKRRS |
| P85 | 224g12 | 774 | *S. pyogenes* penicillin binding protein 2A (AAL98575.1) | 77 | - |
| P86 | 224g12 | 498 | *S. pyogenes* dipeptidase (AAM80370.1) | 80 | MNTKKFTLATVTVMTALACYSSA |
| P87 | 224g12 | 386 | no significant hits | -- | MFKTKKEIFSIRKTALGVGSVLLGVILTTQVASA |
| P88 | 231c11 | 119 | *S. pyogenes* hypothetical protein (AE009962) | 37 | - |
| P89 | 231c11 | 772 | *S. pyogenes* penicillin binding protein 1B (AAK33215.1) | 77 | - |
| P90 | 238b05 | 331 | *S. pyogenes* hypothetical protein (AAL97637.1) | 63 | - |
| P91 | 238b05 | 550 | *S. pyogenes* fibronectin-binding protein-like protein A (AAK33911.1) | 80 | - |
| P92 | 240d11 | 200 | no significant hits | - | MANYKKITSLSLLTLLSLATFSATQYSKVYA |
| P93 | 31e02 | 758 | *Bacillus halodurans* N-acetylmuramoyl-L-alanine amidase (BAB07384.1) | 32 | MKSKKSYVLLLAPFVLASFWQSKMVSA |
| P94 | 38f04 | 143 | *S. pyogenes* hypothetical protein (AAK34637.1) | 27 | MKKRKNKWRFFMIKMRKSQLSVSLALFALLTFAASPIYA |
| P95 | 40c10 | 747 | *S. pyogenes* penicillin binding protein 2X (AAK34426.1) | 74 | - |
| P96 | 45f07 | 200 | *S. suis* Cps9F (AAF18949) | 77 | MYQVVKRLLAILISGLAIIILSPVLLAVAIA |
| P97 | 45f07 | 424 | *S. agalactiae* CpsA (AF349539) | 61 | MASLLLILLKKAKLLTMIGLILANIGLAVTLFA |
| P98 | 52g05 | 347 | *S. pyogenes* hypothetical protein (AAK34330.1) | 68 | MKVIKTYKWWVLSILSMVLILFALFFPLPYYIEMPGGA |
| P99 | 52g05 | 506 | *B. anthracis* amidase (NP_655785) | 40 | - |
| P100 | 53f09 | 169 | *L. lactis* unknown protein (AAK04688.1) | 50 | - |
| P101 | 67h05 | 425 | *S. mutans* immunodominant glycoprotein (AAK94501.1) | 41 | MKKRILSAVLVSGVTLGTATTVNA |
| P102 | 67h05 | 137 | *S. pyogenes* hypothetical protein (AAL96870.1) | 57 | - |
| P103 | 68d07 | 280 | *S. pyogenes* endolysin (AAL97346.1) | 73 | MRRRIKPIVVLVFFLLFALLLIIGKTHS |
| P104 | 71g04 | 429 | *S. pyogenes* putative peptidoglycan GlcNAc deacetylase (AAM79651.1) | 47 | MKKFYVIVGTLLSIFILSVSLFVYS |
| P105 | 71g04 | 727 | *S. pyogenes* internalin A (AAL97968.1) | 52 | MKKKTYLFVAGITVTCGTAL |
| P106 | 77f12 | 393 | *S. pyogenes* D D carboxypeptidase (AAM78820.1) | 62 | MIKKILLFLSIFALTISTIPVIA |
| P107 | 77f12 | 400 | *S. pyogenes* D-alanyl-D-alanine carboxypeptidas, (AAM78821.1) | 38 | MKKTILSTIIVGLFLWTLSTLVLA |
| P108 | 77f12 | 415 | *S. pyogenes* D-alanyl-D-alanine carboxypeptidase (AAM78821.1) | 60 | MKKMLLLCFIFLILFPINFVNA |
| P109 | 80b12 | 202 | *S. pyogenes* superoxide dismutase [Mn] (AAM79678.1) | 89 | - |
| P110 | 80b12 | 350 | *S. pyogenes* hypothetical protein (AAL98000.1) | 71 | MRLQMMTFLRKINSTKVLIFLCVSLFLGLWTVSA |
| P111 | 198g02 | 128 | *S. pneumoniae* hypothetical protein (AAL00353.1) | 36 | MKRKISLFLFLASIFATTNSVFA |
| P116 | 84d07 | 427 | *S. pyogenes* RopA (AAM80241.1) | 82 | - |
| P118 | 108f04 | 115 | *S. pyogenes* hypothetical protein (AAL97738.1) | 34 | MKAKRDGLIIGLVTGVVAGTLSYLSLSHS |
| P119 | 113f05 | 301 | *S. pyogenes* tRNA isopentenylpyrophosphate transferase (AAL97618.1) | 68 | MTKKEKIIVIVGPTAVGKTALGIQVAQA |
| P120 | 115e06 | 216 | *Bacillus halodurans* hydroxybutyryl-CoA dehydrogenase (BAB05714.1) | 52 | MTNIKTIGVVGAGAMGGGIANLFA |
| P121 | 115e06 | 287 | *B. halodurans* 3-hydroxybutyryl-CoA dehydrogenase (BAB05714.1) | 43 | MTNIKTIGVVGAGAMGGGIANLFA |
| P122 | 130c11 | 589 | *S. pyogenes* aminodeoxychorismate lyase (AAL97146.1) | 52 | - |
| P123 | 130g06 | 331 | *S. pyogenes* thioredoxin reductase (AAM79182.1) | 80 | - |
| P124 | 130g06 | 203 | *S. pyogenes* peptidoglycan hydrolase (AAK33784.1) | 68 | - |
| P125 | 130g06 | 246 | *S. pyogenes* peptidoglycan hydrolase (AAK33785.1) | 61 | MRFLKGKKVFLAVIGLAVMMTLVIMFQPQAKNKSVSAE |
| P126 | 130g06 | 357 | *S. pyogenes* spermidine/putrescine ABC transporter (AAK33983.1) | 80 | MRRLYSFIAGVLGIILILASSTFILQKKTGSA |
| P127 | 130g06 | 200 | *S. pyogenes* hypothetical protein (AAL97729.1) | 74 | - |
| P128 | 133f06 | 609 | *S. pyogenes* alpha-glycerophosphate oxidase (AAK34439.1) | 80 | - |
| P129 | 198g02 | 337 | *Listeria morcocytogerces* autolysin (AF035424) | 38 | - |
| P130 | 19e05 | 289 | *S. pyogenes* hypothetical protein (AAM79683.1) | 78 | - |
| P131 | 224g12 | 545 | *S. pyogenes* hypothetical protein (AAK34725.1) | 46 | MKKIFQRKWFKRTSIVLGILLVALIALGS |
| P132 | 240d11 | 308 | *L. lactis* unknown protein (AAK06265.1) | 29 | MKSNDPLALLAKKRRRKTFLMTIVFSLLATLLLFALCFKLLS |
| P133 | 240d11 | 322 | *Clostridium acetobutylicum* Ribose ABC transporter (AAK79421.1) | 39 | - |
| P134 | 24d11 | 485 | *Staphylococcus aureus* hypothetical protein (BAB95577.1) | 29 | MLSYRVVKRRLGMVKKQVAIIGMGVSGLAVLLALS |
| P135 | 241b08 | 195 | *S. pyogenes* hypothetical protein (AAK34290.1) | 58 | MRKKRTINWWKWSFLILLALNLAFVCVIA |
| P136 | 45f07 | 347 | *Chlorobium tepidum* glycosyl transferase (AAM71444.1) | 31' | - |
| P137 | 52g02 | 393 | *Streptococcus pyogenes* cell division protein (AAK34317.1) | 43 | - |
| P138 | 68d07 | 280 | *S. pyogenes* endolysin (AAL97346.) | 73 | MRRRIKPIVVLVFFLLFALLLIIGKTHSD |
| P139 | 7f09 | 197 | *S. pyogenes* signal peptidase I (AAK34563.1\|) | 69 | MKHFFKEWGLFTLVILIFGISRLFFWQPVKVDG |

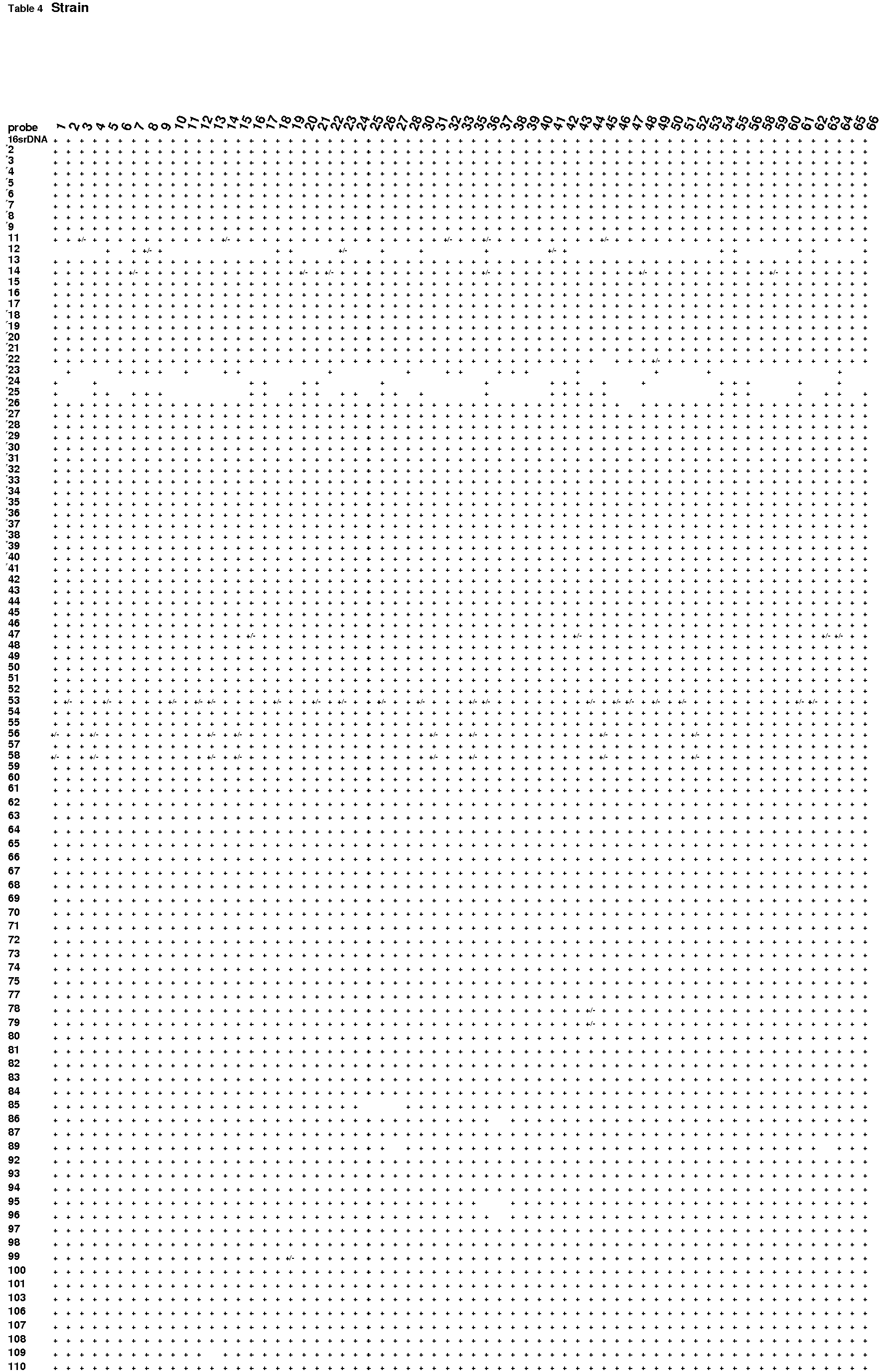

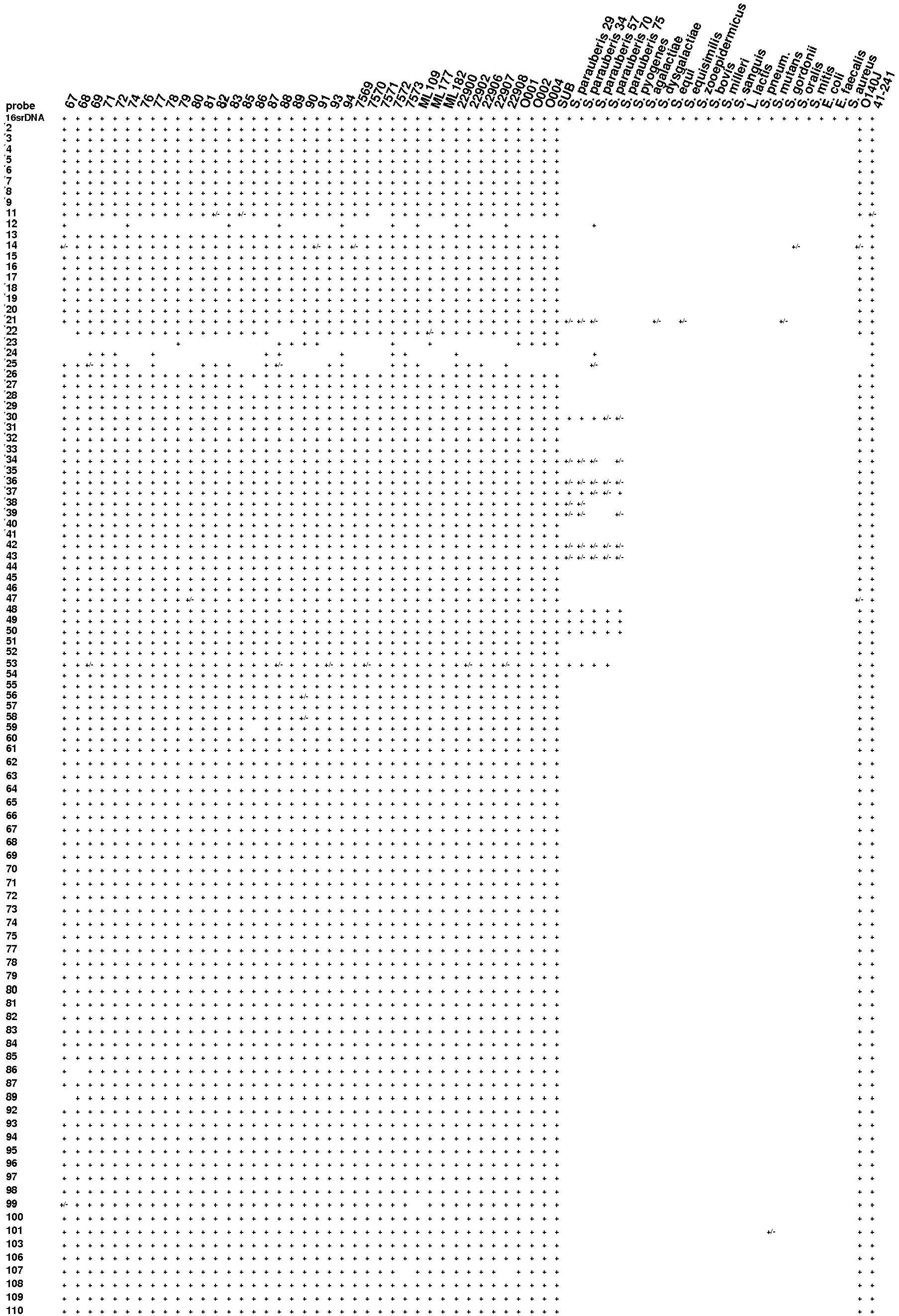

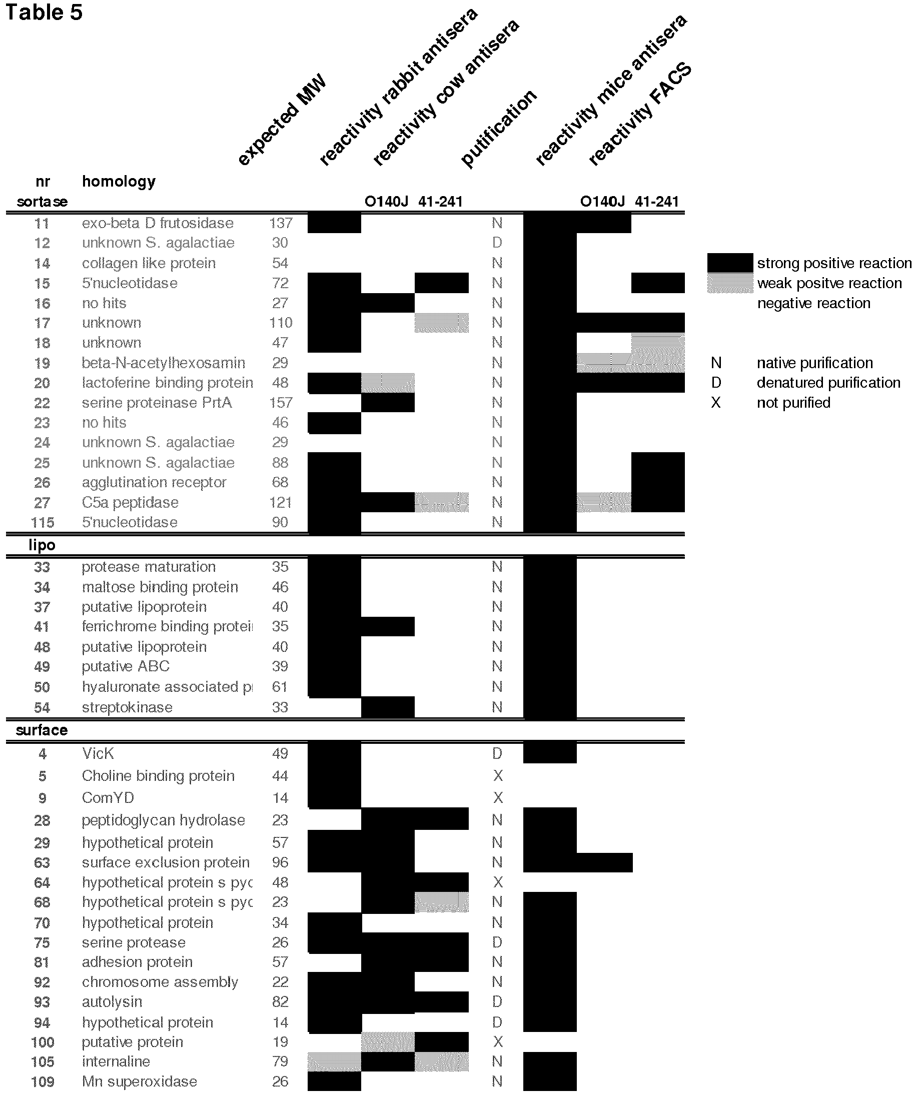

**TABLE 6. Immunogenic proteins of S.uberis identified on 2D gels and characterized by mass spectrometry.**

| spotnr. | protein name | mass (kDa) | pI |
|---|---|---|---|
| 1 | surface exclusion protein | 94,8 | 5,8 |
| 2 | trigger factor (ropA) | 47,3 | 4,3 |
| 3 | nucleoside diphosphate kinase | 16,2 | 5,7 |

**TABLE 7. Summary of the results of experimental intra-mammary infections by S.uberis strains 0140J and 41-241**

| | - | | | | | | | | | |
|---|---|---|---|---|---|---|---|---|---|---|
| cow no. strain udder | | experiment inoculation | | max. SCC | SCC | max. temp | no quarters | BO | clots in milk | consistency |
| | | finished after | dose (cfu) | | prior to inoculation | | successfully infected | max. score¹⁾ | max. score²⁾ | max. score³⁾ |
| | - | | | | | | | | | |
| 6716 | O140J | 50 hours | 5 x 10² | 2,9 x 10⁷ | 2,7 x 10⁴ | 40,2 | 2 | 3 | 0 | 0 |
| 6717 | O140J | 80 hours | 5 x 10² | 9,7 x 10⁵ | 1,2 x 10⁵ | 38,6 | 1 | 1 | 0 | 0 |
| 6720 | 41-241 | 45 hours | 5 x 10³ | 2,8 x 10⁷ | 4,8 x 10⁴ | 40,8 | 2 | 3 | 2 | 0 |
| 6721 | 41-241 | 67 hours | 5 x 10² | 7,2 x 10⁶ | 0.9 x 10⁵ | 38,7 | 2 | 3 | 1 | 0 |
| | | | | | | | | | | |
| 6718 | O140J | 16 days | 5 x 10² | 2,9 x 10⁷ | 0.9 x 10⁵ | 40,9 | 2 | 3 | 4 | 3 |
| 6719 | O140J | 16 days | 5 x 10² | 2,4 x 10⁷ | 0.9 x 10⁵ | 40,0 | 1 | 3 | 3 | 3 |
| 6722 | 41-241 | 16 days | 5 x 10³ | 2,7 x 10⁷ | 4,4 x 10⁴ | 41.0 | 2 | 3 | 3 | 2 |
| 6723 | 41-241 | 16 days | 5 x 10² | 2,1 x 10⁷ | 3,6 x 10⁴ | 39,5 | 2 | 3 | 2 | 1 |

| | | | | | | | | | | |
|---|---|---|---|---|---|---|---|---|---|---|
| ¹) BO score: BO negative: 0; < 10³ cfu/ml milk: 1; > 10³< 10⁵ cfu/ml milk: 2: > 10⁵ cfu/ml milk: 3 ²) normal milk: 0; few clots: 1; numerous clots: 2; milk with pus: 3; milk with blood: 4. ³) consistency score: normal consistency: 0; mild changes: 1; moderate changes: 2; severe changes: 3. | | | | | | | | | | |

## Claims

1. A method for identifying a *Streptococcus* protein which is capable of eliciting an immune response against at least two strains and/or serotypes of *Streptococcus,* the method comprising:
a) identifying at least part of a secreted protein, a surface-associated protein and/or a protein which has at least 50% sequence identity to a bacterial virulence factor;
b) selecting at least one protein identified in step a) which is conserved over at least two *Streptococcus* strains and/or serotypes; and
c) determining whether at least one protein selected in step b) or an immunogenic part, derivative and/or analogue thereof is capable of specifically binding an antibody and/or immune cell of an animal infected by a first *Streptococcus* strain and/or serotype, and an antibody and/or immune cell of an animal infected by a second *Streptococcus* strain and/or serotype.

2. A method according to claim 1, comprising:
- obtaining isolated and/or recombinant proteins of *Streptococcus*;
- incubating said proteins with an antibody and/or immune cell of an animal infected by a first *Streptococcus* strain and/or serotype and an antibody and/or immune cell of an animal infected by a second *Streptococcus* strain and/or serotype, and
- determining whether a protein is capable of binding an antibody and/or immune cell of an animal infected by a first *Streptococcus* strain and/or serotype and an antibody and/or immune cell of an animal infected by a second *Streptococcus* strain and/or serotype.

3. A method according to claim 1 or 2, wherein at least two *Streptococcus* proteins capable of eliciting an immune response against at least two strains and/or serotypes of *Streptococcus* are identified.

4. A method for producing at least one protein identified by a method according to any one of claims 1-3.

5. A *Streptococcus* protein which is capable of eliciting an immune response against at least two strains and/or serotypes of *Streptococcus* obtainable by a method according to any one of claims 1-4.

6. A recombinant nucleic acid molecule comprising a nucleic acid sequence encoding at least two proteins obtainable by a method according to any one of claims 1-4 and/or an immunogenic part of at least one of said proteins, under the control of a functionally linked promoter.

7. A recombinant carrier comprising a nucleic acid encoding at least two proteins obtainable by a method according to any one of claims 1-4 and/or an immunogenic part of at least one of said proteins.

8. An immunogenic composition capable of eliciting an immune response against at least two strains and/or serotypes of *Streptococcus* comprising at least one isolated and/or recombinant protein according to claim 5, a recombinant nucleic acid molecule according to claim 6, a recombinant carrier according to claim 7 and/or an immunogenic part, derivative and/or analogue thereof.

9. A method for producing an immunogenic composition capable of eliciting an immune response against at least two strains and/or serotypes of *Streptococcus,* said method comprising providing a cell with at least one recombinant vector, said at least one vector comprising a nucleic acid sequence encoding at least one protein obtainable by a method according to any one of claims 1-4 and/or an immunogenic part, derivative and/or analogue thereof.

10. A method for measuring the immunity of an individual or non-human animal against *Streptococcus,* said method comprising determining in at least one sample from said individual or animal the presence of antibodies directed against a protein according claim 5 and/or an immunogenic part thereof.

11. A diagnostic kit comprising at least one protein according to claim 5 or an immunogenic part thereof and a means of detecting antibody binding to said protein or immunogenic part thereof.

12. Use of an immunogenic composition according to claim 8 for the preparation of a vaccine and/or a medicament against a *Streptococcus* related disease.

13. An immunogenic composition according to claim 8 for use as a vaccine or medicament.

14. An immunogenic composition according to claim 8 for use in a method for treating *a Streptococcus* related disease.

15. A method for identifying a surface-associated protein and/or a protein which has at least 50% sequence identity to a bacterial virulence factor comprising screening a first *Streptococcus* genomic sequence for its capability of hybridizing to a nucleotide sequence encoding a secreted and/or surface-associated of a second *Streptococcus.*

16. A method for determining whether a *Streptococcus* protein has at least 50% sequence identity to a bacterial virulence factor comprising:
- comparing the amino acid sequence of said *Streptococcus* protein with the amino acid sequence of said bacterial virulence factor; or
- screening a *Streptococcus* genomic sequence for a nucleotide sequence which has at least 50% sequence identity to a bacterial gene encoding a virulence factor.
